# EUROPEAN PATENT APPLICATION

(11) **EP 4 339 289 A1**
(43) Date of publication of application: **20.03.2024**
(21) Application number: 22807519.8
(22) Date of filing: 12.05.2022
(51) Int. Cl.: C12N 15/13, A61K 39/395, A61P 29/00, A61P 37/06, C07K 16/28

(54) **ANTI-HUMAN PD-1 AGONIST ANTIBODY FOR TREATING OR PREVENTING INFLAMMATORY DISEASES, AND PHARMACEUTICAL COMPOSITION CONTAINING SAME**

(30) Priority: 13.05.2021 JP 2021081913; 21.05.2021 JP 2021086534
(71) Applicant: Foundation for Biomedical Research and Innovation at Kobe, Kobe-shi, Hyogo 650-0047 (JP); National Institutes of Biomedical Innovation, Health and Nutrition, Ibaraki-shi, Osaka 567-0085 (JP); Meiji Seika Pharma Co., Ltd., Tokyo 104-8002 (JP)
(72) Inventor: HONJO,Tasuku, Kobe-shi, Hyogo 650-0047 (JP); OHTA,Akio, Kobe-shi, Hyogo 650-0047 (JP); TAJIMA,Masaki, Kobe-shi, Hyogo 650-0047 (JP); KAMADA, Haruhiko, Ibaraki-shi, Osaka 567-0085 (JP); NAGATA, Satoshi, Ibaraki-shi, Osaka 567-0085 (JP); SUZUKI, Kensuke, Tokyo 104-8002 (JP); FUKUSHIMA, Takayoshi, Odawara-shi, Kanagawa 250-0852 (JP); TOKUMARU, Yosuke, Tokyo 104-8002 (JP)
(74) Representative: Hoffmann Eitle
(86) International application number: PCT/JP2022/020011
(87) International publication number: WO 2022/239820

(57) **Abstract**

Conditions necessary for antibodies to human PD-1 to have agonist activity are searched and an optimized agonist antibody is provided based on the conditions and used as a therapeutic for human inflammatory diseases. An agonist antibody to human PD-1 or a functional fragment thereof, wherein the antibody or a functional fragment thereof binds to domain #7 of human PD-1 as shown in SEQ ID NO: 9 is provided.

## Description

### TECHNICAL FIELD

The present invention relates to a PD-1 agonist and a pharmaceutical composition comprising a PD-1 agonist for treating or preventing inflammatory diseases.

### BACKGROUND ART

The immune response has an aspect that its inappropriate regulation could lead to diseases. An insufficient immune response to pathogens such as bacteria or viruses that have invaded the organism results in infectious diseases. Conversely, if adverse immune response to self-tissues occurs, autoimmune diseases develop. To prevent from falling into such pathological conditions and to utilize the system effectively, the immune system is provided with both mechanisms activating the function of immune cells to promote immune response and mechanisms suppressing immune cells to reduce immune response.
Disruption of these endogenous regulatory mechanisms is considered to contribute to diseases resulting from insufficient or excessive immune response.

The best example demonstrating the importance of endogenous immunoregulation can be found in cancer immunotherapy that has rapidly become a reality in recent years. The molecules such as CTLA-4 and PD-1 appearing in this novel therapy, which is clearly distinct from conventional "immunotherapy", are a type of endogenous immunosuppressive mechanisms also called immune checkpoints, and have a particularly remarkable impact among the same type of immunosuppressive mechanisms. The pathology of cancer can be regarded as a condition where cancer cells that should have been eliminated have proliferated because of insufficient immune response thereto. The cause of this insufficient immune response is related to the fact that the inside of cancer tissues is filled with various types of immunosuppressive mechanisms. This means that cancer tissues evade attacks from immune cells by actively using the immunosuppressive mechanisms intrinsic to the organism. By blocking CTLA-4 or PD-1, representative immunosuppressive mechanisms, the inhibition of anti-tumor immunity is reversed and the resulting immune activation brings about actual therapeutic effects. The fact shows that the above-mentioned endogenous immunosuppressive mechanisms have highly significant effects and can be important targets for the treatment of diseases by correcting the imbalance in immune response.

The critical importance of endogenous immunosuppressive mechanisms is also shown by the fact that the absence of any one of these mechanisms can cause much exaggerated inflammatory responses. For example, PD-1-deficient mice have been shown to spontaneously develop various inflammatory (Non-Patent Document No. 1: Okazaki et al. Nat. Immunol., 2013). Inflammatory adverse response resulting from excessive immune response is observed at a certain proportion of cancer patients undergoing immunotherapy with PD-1 inhibitors (Non-Patent Document No. 2: Young et al. Cancer Immunol. Res., 2018). These facts indicate that the immunosuppressive mechanisms that are actively utilized in cancer tissues are essentially physiological feedback mechanisms to protect healthy tissues in the body from excessive inflammatory responses. Importantly, the clinical efficacy of pharmaceutical drugs such as anti-PD-1 antibody in cancer therapy suggests that it is possible to modulate the intensity of immune response by regulating the function of PD-1.

### PRIOR ART LITERATURE

### Non-Patent Documents

Non-Patent Document No. 1: Okazaki T, Chikuma S, Iwai Y, Fagarasan S, Honjo T. A rheostat for immune responses: the unique properties of PD-1 and their advantages for clinical application. Nat. Immunol. 14:1212-1218 (2013).
Non-Patent Document No 2: Young A, Quandt Z, Bluestone JA. The Balancing Act between Cancer Immunity and Autoimmunity in Response to Immunotherapy. Cancer Immunol. Res. 6:1445-1452 (2018).

### DISCLOSURE OF THE INVENTION

### PROBLEM FOR SOLUTION BY THE INVENTION

As shown in the above-described background, drugs that inhibit immunosuppressive mechanisms were used in cancer therapy where the enhancement of immune response was desirable. However, to suppress overwhelming immune response in inflammatory diseases, the treatment demands an opposite approach that actively stimulates the immunosuppressive mechanisms. PD-1 is expressed on immune cells such as activated T cells and interact with ligand molecules such as PD-L1 and PD-L2 on the surface of the other cell. Upon antigen recognition, PD-1 stimulation with these ligand molecules interferes with the activating signal of the immune cells. Since PD-1 itself can initiate the immunosuppressive signaling, artificial stimulation of PD-1 might lead to the treatment of inflammatory diseases. While anti-PD-1 antibodies used in cancer therapy (eventually) enhance immune function by blocking PD-1 interaction with its ligand molecule, PD-1 agonists capable of inducing the function of PD-1 by binding to PD-1 are highly promising as drugs for positive immunosuppression.

The present invention aims at searching for conditions necessary for anti-human PD-1 antibody to have agonistic activity, establishing agonist antibody optimized based on such necessary conditions, and applying the agonist antibody as a therapeutic agent for human inflammatory diseases.

### MEANS TO SOLVE THE PROBLEM

There are a number of inflammatory diseases caused by excessive immune response, and a novel and more effective treatment is demanded in many cases. Targeting an immune regulatory molecule PD-1, the present inventors have found a large number of anti-human PD-1 antibodies, which induce the immunosuppressive activity of PD-1 and are potentially capable of preventing or treating inflammatory diseases in human with the agonistic activity. The present inventors have examined binding domains of these antibodies bind to human PD-1 and found that the biological activity of these antibodies varies depending on the binding domain. Furthermore, the present inventors have found that binding to Fc receptors is necessary for anti-PD-1 antibodies to exert their agonistic activity and that an enhanced affinity to Fc receptors is necessary for immunosuppression in humans. In addition, the present inventors have found that the addition of ADCC-inducing capability to anti-PD-1 agonist antibodies enables depletion of PD-1-expressing activated immune cells, further improving desirable immunosuppressive activity.

The present invention has been achieved based on these findings, and a summary thereof is described as below.
(1) An agonist antibody to human PD-1 or a functional fragment thereof, wherein the antibody or a functional fragment thereof binds to domain #7 as shown in SEQ ID NO: 9, of human PD-1.
(2) The antibody or a functional fragment thereof of (1), wherein the antibody or a functional fragment thereof has any one of (A) to (D) below:
   (A) an antibody heavy chain variable region sequence comprising CDR1, CDR2 and CDR3 in the amino acid sequence as shown in SEQ ID NO: 17 and an antibody light chain variable region sequence comprising CDR1, CDR2 and CDR3 in the amino acid sequence as shown in SEQ ID NO: 34;
   (B) an antibody heavy chain variable region sequence comprising CDR1, CDR2 and CDR3 in the amino acid sequence as shown in SEQ ID NO: 22 and an antibody light chain variable region sequence comprising CDR1, CDR2 and CDR3 in the amino acid sequence as shown in SEQ ID NO: 38;
   (C) an antibody heavy chain variable region sequence comprising CDR1, CDR2 and CDR3 in the amino acid sequence as shown in SEQ ID NO: 28 and an antibody light chain variable region sequence comprising CDR1, CDR2 and CDR3 in the amino acid sequence as shown in SEQ ID NO: 42; or
   (D) an antibody heavy chain variable region sequence comprising CDR1, CDR2 and CDR3 in the amino acid sequence as shown in SEQ ID NO: 33 and an antibody light chain variable region sequence comprising CDR1, CDR2 and CDR3 in the amino acid sequence as shown in SEQ ID NO: 46.
(3) The antibody or a functional fragment thereof of (1), wherein the antibody or a functional fragment thereof has either the following (A) to (D) below:
   (A) an antibody heavy chain variable region comprising complementarity determining region (CDR) 1 of SEQ ID NO: 345, CDR2 of SEQ ID NO: 346 and CDR3 of SEQ ID NO: 347, as well as an antibody light chain variable region comprising CDR1 of SEQ ID NO: 348, CDR2 of SEQ ID NO: 349 and CDR3 of SEQ ID NO: 350;
   (B) an antibody heavy chain variable region comprising CDR1 of SEQ ID NO: 351, CDR2 of SEQ ID NO: 352 and CDR3 of SEQ ID NO: 353, as well as an antibody light chain variable region comprising CDR1 of SEQ ID NO: 354, CDR2 of SEQ ID NO: 355 and CDR3 of SEQ ID NO: 356;
   (C) an antibody heavy chain variable region comprising CDR1 of SEQ ID NO: 357, CDR2 of SEQ ID NO: 358 and CDR3 of SEQ ID NO: 359, as well as an antibody light chain variable region comprising CDR1 of SEQ ID NO: 360, CDR2 of SEQ ID NO: 361 and CDR3 of SEQ ID NO: 362; or
   (D) an antibody heavy chain variable region comprising CDR1 of SEQ ID NO: 363, CDR2 of SEQ ID NO: 364 and CDR3 of SEQ ID NO: 365, as well as an antibody light chain variable region comprising CDR1 of SEQ ID NO: 366, CDR2 of SEQ ID NO: 367 and CDR3 of SEQ ID NO: 368.
(4) The antibody or a functional fragment thereof of any one of (1) to (3), wherein the antibody variable region comprises the framework of human antibody or the framework of humanized antibody.
(5) The antibody or a functional fragment thereof of (1), wherein the antibody or a functional fragment thereof has either the following (A) to (D) below:
   (A) an antibody heavy chain variable region having the amino acid sequence as shown in SEQ ID NO: 20 and a light chain variable region having the amino acid sequence as shown in SEQ ID NO: 37;
   (B) an antibody heavy chain variable region having the amino acid sequence as shown in SEQ ID NO: 21 and a light chain variable region having the amino acid sequence as shown in SEQ ID NO: 37;
   (C) an antibody heavy chain variable region having the amino acid sequence as shown in SEQ ID NO: 26 and a light chain variable region having the amino acid sequence as shown in SEQ ID NO: 40; or
   (D) an antibody heavy chain variable region having the amino acid sequence as shown in SEQ ID NO: 30 and a light chain variable region having the amino acid sequence as shown in SEQ ID NO: 44.
(6) The antibody or a functional fragment thereof of any one of (1) to (5), wherein the antibody heavy chain constant region and the antibody light chain constant region comprise an amino acid sequence derived from human antibody.
(7) The antibody or a functional fragment thereof of any one of (1) to (5), wherein the antibody heavy chain constant region is the antibody heavy chain constant region of human IgG.
(8) The antibody or a functional fragment thereof of (7), wherein the antibody heavy chain constant region of said human IgG is the antibody heavy chain constant region of human IgG1.
(9) The antibody or a functional fragment thereof of any one of (1) to (8), wherein the antibody light chain constant region is the antibody light chain constant region of human Igκ.
(10) The antibody or a functional fragment thereof of any one of (1) to (9), wherein the antibody or a functional fragment thereof has an improved affinity to any one or more of the following receptors: human FcyRIIA, human FcyRIIB or human FcyRIIA.
(11) The antibody or a functional fragment thereof of any one of (1) to (9), wherein the antibody or a functional fragment thereof has an improved affinity to human FcγRIIB.
(12) The antibody or a functional fragment thereof of any one of (1) to (9) or (11), wherein the antibody or a functional fragment thereof has an improved affinity to human FcγRIIIA.
(13) The antibody or a functional fragment thereof of (11) or (12), wherein the improvement of affinity to human FcyRIIB is, compared to a reference antibody having the Fc region of human IgG1-K322A, by 1.5 folds or more, preferably by 2.0 folds or more, and still more preferably by 2.5 folds or more, in a test measuring Fc receptor binding affinity using the surface plasmon resonance technique.
(14) The antibody or a functional fragment thereof of any one of (11) to (13), wherein the improvement of affinity to human FcyRIIB is, compared to a reference antibody having the Fc region of human IgG1-K322A, by 2 folds or more, preferably by 5 folds or more, and still more preferably by 20 folds or more, in a test of measuring the affinity to a human Fc receptor-expressing cell line with a flow cytometer.
(15) The antibody or a functional fragment thereof of (12) or (13), wherein the improvement of affinity to human FcγRIIIA is, compared to a reference antibody having the Fc region of human IgG1-K322A, by 1.5 folds or more, preferably by 2.0 folds or more, and still more preferably by 2.5 folds or more, in a test measuring Fc receptor binding affinity using the surface plasmon resonance technique.
(16) The antibody or a functional fragment thereof of any one of (12) to (15), wherein the improvement of affinity to human FcγRIIIA is, compared to a reference antibody having the Fc region of human IgG1-K322A, by 1.5 folds or more, preferably by 2.0 folds or more, still more preferably by 4.0 folds or more, and most preferably by 5.0 folds or more, in a test of measuring the affinity to a human Fc receptor-expressing cell line with a flow cytometer.
(17) The antibody or a functional fragment thereof of any one of (10) to (16), wherein the antibody or a functional fragment has a modified Fc region of human IgG, and the affinity of the antibody or a functional fragment thereof to said receptor has been improved by modification of the Fc region of human IgG.
(18) The antibody or a functional fragment thereof of any one of (10) to (16), wherein the antibody or a functional fragment has a modified Fc region of human IgG1 or human IgG4, and the affinity of the antibody or a functional fragment thereof to said receptor has been improved by modification of the Fc region of human IgG1 or human IgG4.
(19) The antibody or a functional fragment thereof of any one of (10) to (18), wherein any of the amino acids at positions 233, 234, 236, 237, 238, 239, 267, 268, 271, 296, 323, 326, 328, 330 and 332 (according to EU numbering) in the amino acid sequence of the Fc region of human IgG1 is modified.
(20) The antibody or a functional fragment thereof of (19), wherein the modification of amino acids is any one of the combinations G236D/H268D, S239D/H268D, S239D/H268D/L328Y/I332E, G236D/H268D/K322A, S239D/H268D/K322A, S239D/S267G/H268D/K322A, G236D/H268D/E293A/K322A, S239D/H268D/K322A/L328Y/I332E, S239D/H268D/E293A/K322A, S239D/S267G/H268D/K322A/L328Y, S239D/S267G/H268D/K322A/I332E, S239D/H268D/K322A/L328Y, S239D/H268D/K322A/I332E, S239D/S267G/H268D/K322A/L328Y/I332E, E233D/G237D/P238D/H268D/P271G/A330R and S267E/L328F.
(21) The antibody or a functional fragment thereof of any one of (10) to (18), wherein any of the amino acids at positions 236, 239, 268, 328 and 332 (according to EU numbering) in the amino acid sequence of the Fc region of human IgG4 is modified.
(22) The antibody or a functional fragment thereof of (21), wherein the modification of amino acids is any one of the combinations S228P/G236D/Q268D, S228P/S239D/Q268D, and S228P/S239D/Q268D/L328Y/I332E.
(23) The antibody or a functional fragment thereof of (10), wherein the antibody comprises either the following (A) to (D) below:
   (A) an antibody heavy chain sequence having the amino acid sequence as shown in SEQ ID NO: 245 and an antibody light chain sequence having the amino acid sequence as shown in SEQ ID NO:247;
   (B) an antibody heavy chain sequence having the amino acid sequence as shown in SEQ ID NO: 277 and an antibody light chain sequence having the amino acid sequence as shown in SEQ ID NO: 279;
   (C) an antibody heavy chain sequence having the amino acid sequence as shown in SEQ ID NO: 285 and an antibody light chain sequence having the amino acid sequence as shown in SEQ ID NO: 287; or
   (D) an antibody heavy chain sequence having the amino acid sequence as shown in SEQ ID NO: 289 and an antibody light chain sequence having the amino acid sequence as shown in SEQ ID NO: 291.
(24) The antibody or a functional fragment thereof of any one of (12) to (23), wherein the antibody or a functional fragment thereof is defucosylated.
(25) An isolated nucleic acid molecule encoding the antibody or a functional fragment thereof of any one of (1) to (24).
(26) A vector comprising the nucleic acid molecule of (25).
(27) A host cell comprising the nucleic acid molecule of (25).
(28) A host cell comprising the vector of (26).
(29) A method of producing an antibody or a functional fragment thereof, comprising a step of culturing the host cell of (27) or (28).
(30) A pharmaceutical composition comprising the antibody or a functional fragment thereof of any one of (1) to (24) and a pharmaceutically acceptable carrier.

### EFFECT OF THE INVENTION

The present invention makes it possible to treat or prevent inflammatory diseases.

The present specification encompasses the contents disclosed in the specification and/or the drawings of Japanese Patent Application Nos. 2021-81913 and 2021-86534 based on which the present patent application claims priority.

### BRIEF DESCRIPTION OF DRAWINGS

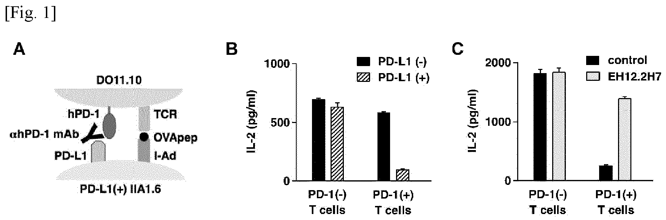

[Fig. 1] Evaluation system for suppressive activity against cytokine production from T cell by PD-1 stimulation. (A) Schematic diagram of evaluation system using DO11.10 T cell hybridomas rendered to express human PD-1 (hPD-1) and IIA1.6 B cell lymphoma cells. DO11.10 T cell hybridomas are activated in response to OVA₃₂₃₋₃₃₉ peptide presented by MHC class II molecule (I-A^{d}) of IIA1.6 cells, but the activation is suppressed when stimulation to PD-1 occurs. (B) When interaction with PD-L1 on IIA1.6 cells occurs, IL-2 production from the human PD-1 expressing DO11.10 T cell hybridomas is suppressed. In the case where PD-1 or PD-L1 is not expressed, suppression of IL-2 is not observed. (C) When EH12.2H7, a blocking antibody to human PD-1, is added to the evaluation system, suppressive effect by PD-L1 is cancelled. With this evaluation system, detection of PD-1-dependent immune regulatory activity is possible.

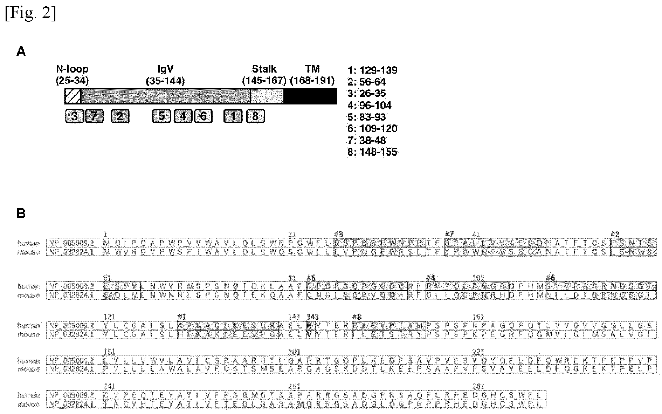

[Fig. 2] Sequences of human and mouse PD-1, and schematic diagram showing domains of PD-1 for examining the binding characteristics of a group of novel anti-human PD-1 antibodies. (A) Domains indicated by numbers 1 to 8 are representative domains constituting the surface of human PD-1 molecule. PD-1 sequences were prepared by replacing one of these domains with the corresponding amino acid sequence of mouse PD-1. When anti-human PD-1 antibody showed a decrease in binding capacity, the relevant domain was defined as a domain to which the antibody binds. Further, antibody binding was also examined on point mutants of human PD-1 in which only one amino acid residue was mutated and on mouse PD-1 only a part of which was replaced with the corresponding amino acid sequence of human PD-1. (B) Sequence comparison between human PD-1 and mouse PD-1. The framed parts represent domains No. 1 to No. 8 as shown in Fig. 2A.

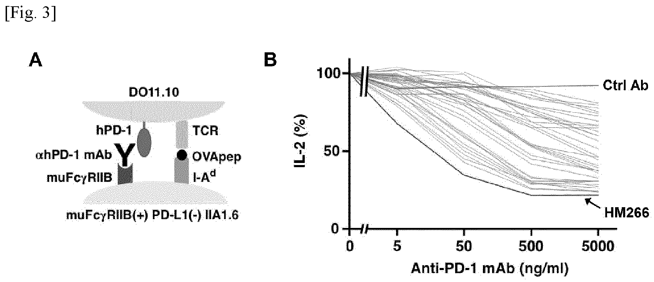

[Fig. 3] Evaluation of agonistic activity of anti-human PD-1 antibody. (A) Schematic diagram of an evaluation system combining DO11.10 T cell hybridomas rendered to express human PD-1, and IIA1.6 B cell lymphoma cells rendered to express not PD-L1 but mouse FcγRIIB. Screening of anti-human PD-1 antibodies was performed using IL-2 production suppressive activity as an indicator.
   (B) As a result of evaluation, about 30 clones having an immunosuppressive activity were obtained, including those with high activity and those with low activity. Among these anti-human PD-1 antibodies, only the clones having an immunosuppressive activity are shown in the graph.

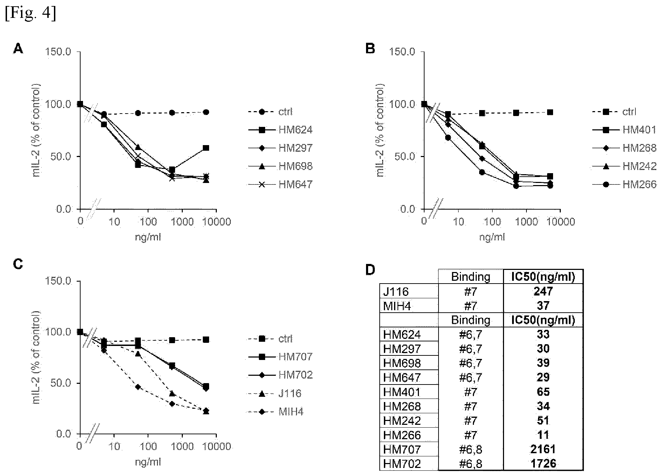

[Fig. 4] Comparison between the activities of anti-human PD-1 agonist antibodies. Among the anti-human PD-1 antibodies found to have an immunosuppressive activity, those having relatively high activity were selected and IC50 was determined. Representative antibodies from each of the following groups are shown: (A) # 6,7 group (antibody group that binds to domains #6 and #7), (B) #7 group (antibody group that binds only to domain #7) and (C) #6,8 group (antibody group that binds to domains 6 and 8). (D) IC50 values of the antibodies shown in (A) to (C). Not only the novel antibodies prepared by immunizing mice with human PD-1, but also commercially available antibodies (J116, MIH4) which were found to have agonistic activity are all mouse IgG. Based on the suppression curve of IL-2 production in a concentration-dependent manner by these antibodies, the concentration at which 50% suppression was reached was determined, taking the state of no IL-2 production as 0%.
[Fig. 5AB] Correlation between the activity of anti-human PD-1 antibody and binding site. (A) Antibodies having agonistic activity exhibit a characteristic of binding to domains #6 and #8, domains #6 and #7 or domain #7. This characteristic is completely different from that of antibodies exhibiting blocking activity. In the #6,7 group, all antibodies exhibit agonistic activity regardless of the difference in binding to domain #1. (B) A model showing the extracellular domain of human PD-1 molecule. In the model, human PD-1 molecule is connected to the transmembrane domain at the upper end. Domains #6, #7 and #8, which are the binding sites of anti-human PD-1 agonist antibodies, exist at the membrane-proximal part of human PD-1. On the other hand, sites associated with the binding of anti-PD-1 blocking antibodies are localized at the membrane-distal position. It is estimated that amino acid residue Arg143 is located in a part sandwiched between domain #6 and domain #7.
[Fig. 5CD] Correlation between the activity of anti-human PD-1 antibody and binding site. (C) Analysis example of binding characteristics. To determine the binding capacities of anti-human PD-1 antibodies, their binding to PD-1 molecules (wild-type or point-mutant of human PD-1 and substituted mouse PD-1) which were forcibly expressed in cells was evaluated by flow cytometry using a fluorescein-labeled anti-IgG antibody. The vertical axis represents the fluorescence intensity of GFP co-expressed with PD-1, which is proportional to the expression level of PD-1. The horizontal axis represents the amount of bound anti-human PD-1 antibody. The results are shown for the cases of using cells that expressed the following PD-1 proteins: (from the top) wild-type human PD-1, R143A point mutant of human PD-1, and domain #7 substituted mouse PD-1 (domain #7 of mouse PD-1 was replaced with the amino acid sequence of domain #7 of human PD-1; designated "Mouse PD-1 (hu38-48)" in the Figure). HM268, one of the newly obtained anti-human PD-1 antibodies, bound to both wild-type human PD-1 and R143A point mutant by almost equal degrees, while the binding of a known antibody 949 to R143A point mutant showed an extremely decrease. (D) Anti-human PD-1 antibodies were classified based on the difference in their binding domains on human PD-1. As a result, a group of novel anti-human PD-1 antibodies were obtained whose binding domains are different from those of a group of known antibodies including PD-1 blocking antibody such as Nivolumab. This novel antibody group is classified in those antibodies which do not bind to PD-1 if domain #7 is replaced and those antibodies which do not bind to PD-1 if either domain #7 or domain #6 is replaced. These two types are distinguished from each other as antibody group in which domain #7 is important for binding (#7 only/#7 group) and antibody group in which both domain #6 and domain #7 are important for binding (#6 and 7/#6,7 group). In #7 only group, known antibodies such as 949 and J116 are included. However, while the antibodies of the present invention retained binding capacity to human PD-1 Arg143 point mutants like R143A and R143V as well as mouse PD-1 with domain #7 being substituted by human domain #7, known antibodies showed a remarkable decrease in binding capacity to these point mutants and substituted PD-1. Further, #6 and 7 group encompassed those antibodies which do not bind to PD-1 when domain #1 is replaced (HM292, HM297, HM330 and HM624); those antibodies whose binding decreases when domain #1 is replaced (HM698, HM634 and HM643); or those which retain binding to PD-1 even when domain #1 is replaced (HM647 and HM654). These sub-groups of antibodies are characterized by a common nature that they bind to domain #6 and domain #7, and are distinguished from known antibodies. These results show that the novel anti-human PD-1 antibodies of the present invention have a binding pattern different from those of known antibodies.

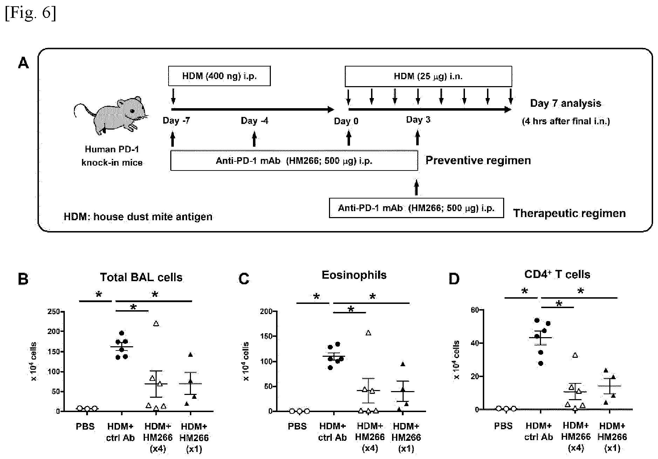

[Fig. 6] Induction of allergic asthma in mouse and anti-inflammatory action of anti-human PD-1 agonist antibody. (A) A diagram showing a method of induction of allergic asthma and a schedule of antibody administration. Allergic asthma was induced by sensitizing human PD-1 knock-in mice with house dust mite antigen (HDM) and nasally administrating the same antigen from one week later. Seven days after the start of daily HDM nasal administration, infiltration of inflammatory cells into the alveoli was examined by collecting bronchoalveolar lavage fluid. (B-D) Induction of allergic asthma increased the total number of infiltrated inflammatory cells (B), most of which were eosinophils (C) and CD4⁺ T cells (D). However, preventive administration of anti-human PD-1 agonist antibody to this inflammation model inhibited the infiltration of eosinophils and CD4⁺T cells into the alveoli. Further, even in the case where HM266 was administered only once (HM266 (x1)) 3 days after the induction of inflammation in the lung tissue in order to observe the therapeutic effect, the infiltration of inflammatory cells into the alveoli was suppressed.

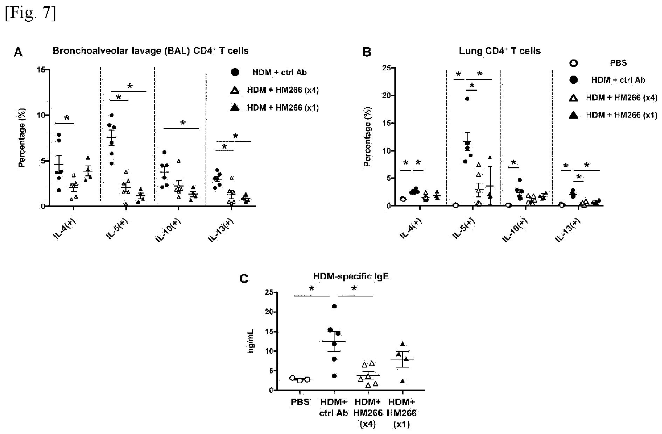

[Fig. 7] Anti-inflammatory action of anti-human PD-1 agonist antibody against allergic asthma and suppression of Th2 type immune response. Allergic asthma was induced according to the same schedule as in Fig. 6, and bronchoalveolar lavage fluid and lung tissue were collected. Cytokine production in CD4⁺ cells infiltrating into the alveoli (A) or lung tissue (B) was examined by intracellular staining. As a result, a decrease in IL-4, IL-5, IL-10 and IL-13 producing cells was observed in the group receiving HM266 four times (HM266 (x4)). Further, blood concentration of IgE specific to house dust mite antigen (C) was also significantly decreased by administration of HM266. A similar tendency was also observed when HM266 was administered only once (HM266 (x1)) 3 days after the start of inflammation induction in the lung, suggesting that anti-human PD-1 agonist antibody is useful for treatment of allergic diseases.

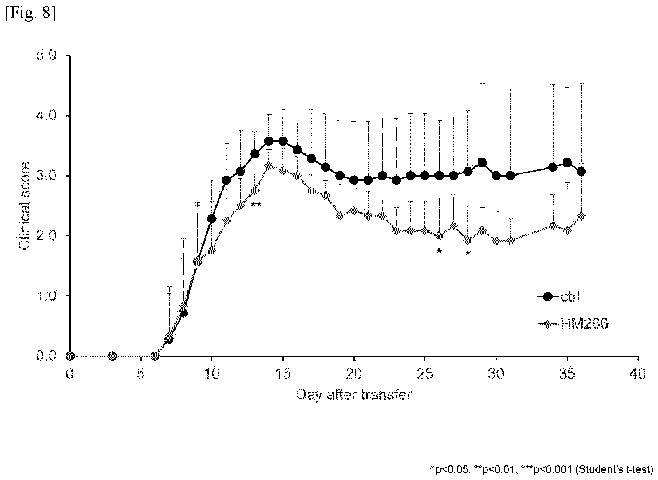

[Fig. 8] Induction of experimental encephalomyelitis (EAE) in mouse and anti-inflammatory action of anti-human PD-1 agonist antibody. Spleens and lymph nodes were collected from wild-type mice 13 days after immunization with myelin oligodendrocyte glycoprotein (MOG)-derived peptide 35-55 (MOG₃₅₋₅₅ peptide) to thereby obtain CD4⁺ T cell fraction. These CD4⁺ T cells were cultured in the presence of MOG₃₅₋₅₅ peptide and IL-23, and at the same time, human PD-1 was overexpressed using a retroviral vector. After culturing for 5 days, cells were harvested and transferred into new wild-type mice to induce EAE. About 1 week after cell transfer, mice developed EAE and the EAE score increased. However, preventive administration of HM266 to this model showed a significant improvement in symptoms. These results suggested that anti-human PD-1 agonist antibody is useful for treatment of autoimmune diseases.

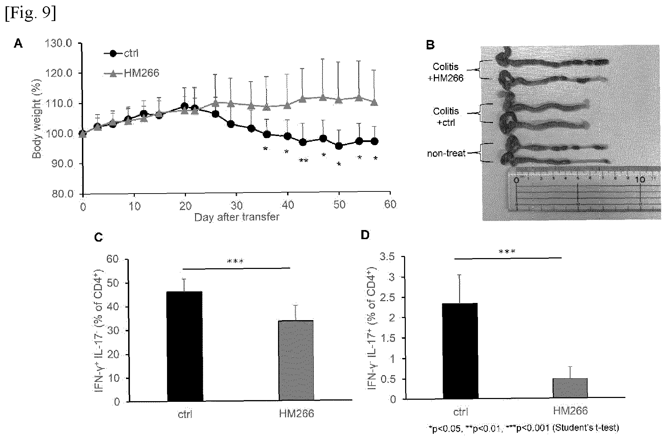

[Fig. 9] Induction of colitis in mouse and anti-inflammatory action of anti-human PD-1 agonist antibody. T cell-dependent colitis was induced by transferring human PD-1 knock-in mouse-derived CD25⁻CD4⁺ T cells into RAG2 knock-out mice. (A) Although a remarkable weight loss was observed from 4 weeks after cell transfer, administration of HM266 significantly improved the weight loss. (B-D) Analysis of the colon collected 8 weeks after cell transfer showed a decrease in colon length and a significant reduction of IFN-γ or IL-17 positive ratio in CD4⁺ T cells infiltrating the lamina propria, as compared to a control. These results suggested the applicability of anti-human PD-1 agonist antibody to a treatment of inflammatory bowel diseases.
[Fig. 10AB] Induction of acute Graft-Versus-Host Disease (GVHD) in mouse and anti-inflammatory action of anti-human PD-1 agonist antibody. Splenocytes derived from C57BL/6 human PD-1 knock-in mice (donor) were transferred into BDF1 mice (recipient) to thereby induce acute GVHD. Induction of GVHD led to weight loss, increase of donor cells in peripheral blood, and induction of PD-1 on donor T cells. When HM266, HM242, HM297 and HM268 were respectively administered to this inflammation model in a preventive manner, weight loss and donor cell increase were remarkably suppressed.

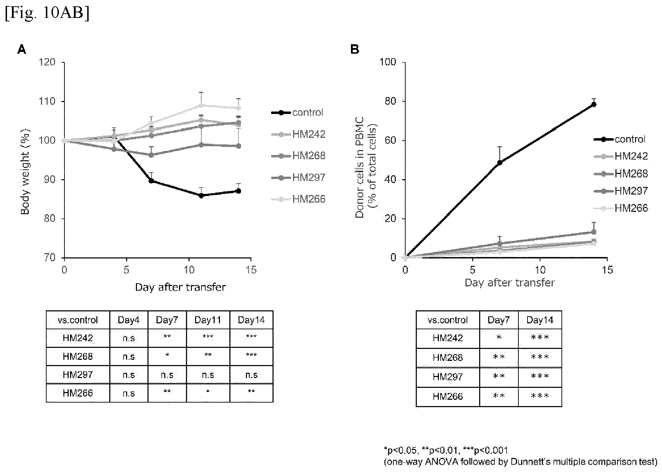

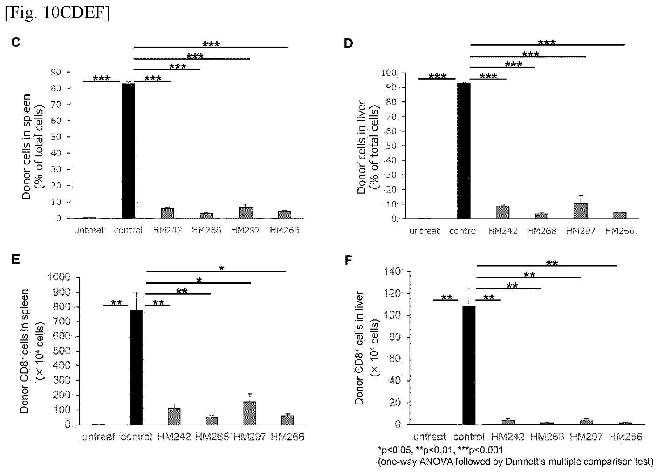

[Fig. 10CDEF] Induction of acute GVHD in mouse and anti-inflammatory action of anti-human PD-1 agonist antibody. Splenocytes derived from C57BL/6 human PD-1 knock-in mice (donor) were transferred into BDF1 mice (recipient) to thereby induce acute GVHD. When HM266, HM242, HM297 and HM268 were respectively administered to this inflammation model in a preventive manner, donor cell ratios (C-D) and donor-derived CD8⁺ cell ratios (E-F) in the spleen and the liver 2 weeks after induction were significantly suppressed. These results suggested the applicability of anti-human PD-1 agonist antibody to a treatment of inflammatory diseases.

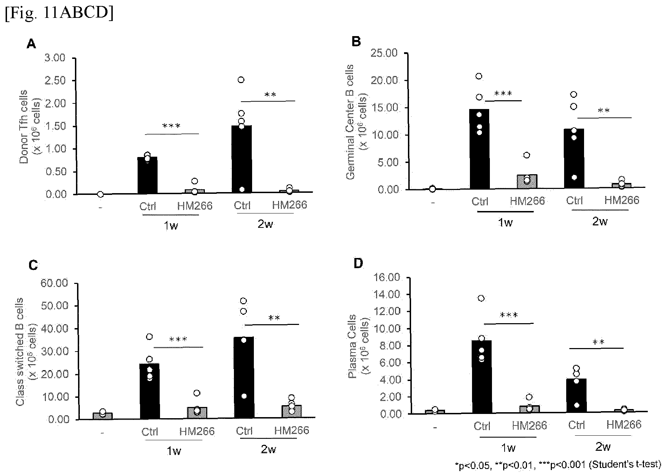

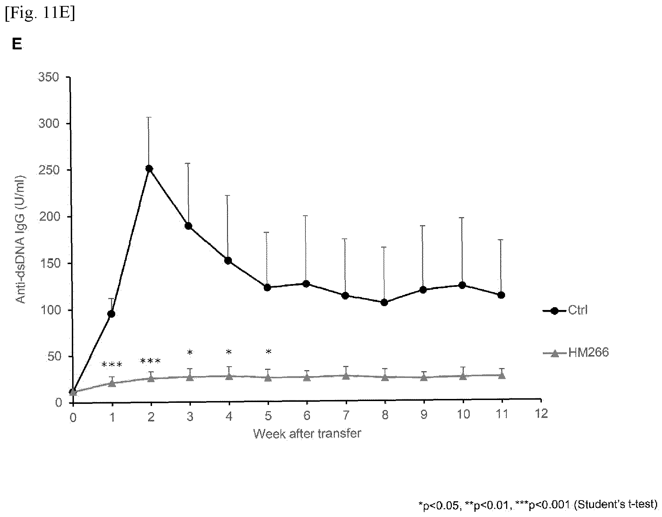

[Fig. 11ABCD] Induction of chronic GVHD/lupus-like symptoms in mouse and anti-inflammatory action of anti-human PD-1 agonist antibody. Splenocytes derived from C57BL/6 human PD-1 knock-in mice (donor) were freed of CD8⁺ cells and thereafter transferred into BDF1 mice (recipient) to thereby induce chronic GVHD/lupus-like symptoms. After the cell transfer, donor-derived Tfh cells (A), recipient-derived germinal center B cells (B), class switched B cells (C) and plasma cells (D) in the recipient's spleen were confirmed to increase remarkably. Prophylactic administration of HM266 to this inflammation model suppressed these increases. Mark "-" represents recipient mice into which no donor cells were transferred.
[Fig. 11E] Induction of chronic GVHD/lupus-like symptoms in mouse and anti-inflammatory action of anti-human PD-1 agonist antibody. Splenocytes derived from C57BL/6 human PD-1 knock-in mice (donor) were freed of CD8⁺ cells and thereafter transferred into BDF1 mice (recipient) to thereby induce chronic GVHD/lupus-like symptoms. After the cell transfer, anti-dsDNA antibody was confirmed to increase remarkably in blood. When HM266 was administered to this inflammation model in a preventive manner, the increase in blood anti-dsDNA antibody was suppressed. These results suggested the applicability of anti-human PD-1 agonist antibody to a treatment of inflammatory diseases that involve a production of autoantibody.

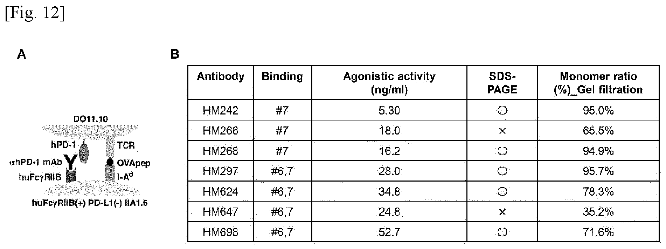

[Fig. 12] Chimerization of anti-human PD-1 agonist antibody with human Fc region and its biological activity/property profile test. From the anti-human PD-1 agonist antibodies obtained, those with high activity were selected. Then, a chimeric antibody having mouse-derived Fab region and the Fc region of human IgG1-K322A (human Fc chimeric antibody) was prepared. (A) Since the human Fc region was used, a human FcyRIIB-expressing cell line was used to evaluate the agonistic activity. (B) Evaluation of the agonistic activity and property of the human Fc chimeric antibody. In the column described "SDS-PAGE", mark "o" represents antibodies in which no abnormal bands were detected, and mark "X" represents antibodies in which abnormal bands such as dimers of heavy chain alone were detected. Differences were seen between the clones tested; especially HM242, HM268 and HM297 showed a high agonistic activity and a good property profile.

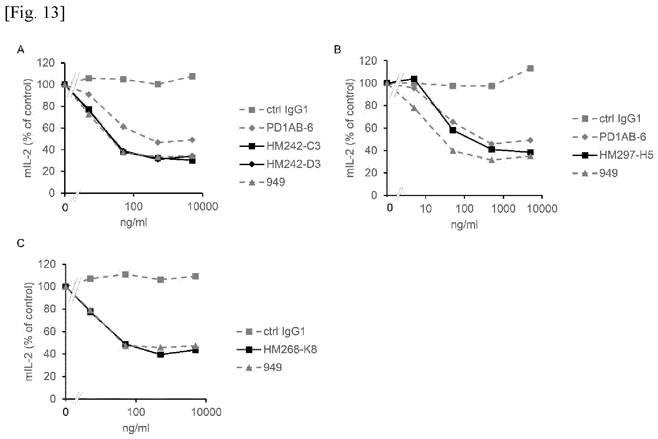

[Fig. 13] Humanization of anti-human PD-1 agonist antibody and its biological activity/property profile test. Agonistic activities of humanized (A) HM242, (B) HM297 and (C) HM268 are shown. Their property profiles are shown in Tables 1 to 3. Further, in Tables 1 to 3, "% Identical with human sequence" (= humanization ratio) means homology (%) in comparison between the sequence of antibody excluding CDRs and the sequence of antibody derived from human germline. While some antibodies showed a decrease in agonistic activity and stability as a result of humanization, a plurality of antibodies (HM242-C3, HM242-D3, HM297-HS and HM268-K8) were obtained which retained a high agonistic activity and showed a good property profile.

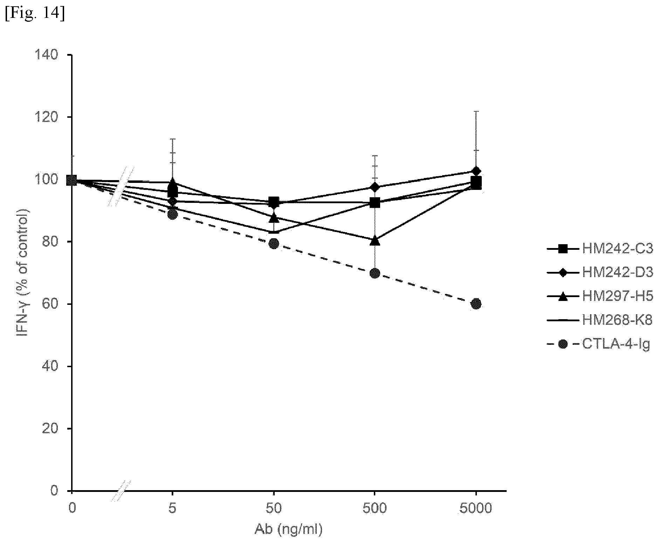

[Fig. 14] Action of humanized anti-human PD-1 agonist antibody in an evaluation system using human primary cultured cells. The present inventors examined whether the humanized anti-PD-1 agonist antibody having humanized Fab region and the Fc region of human IgG1-K322A would exhibit a T cell-suppressive action in an evaluation system solely composed of human primary cultured cells. Induction of PD-1 expression in CD4⁺ T cells was confirmed by a mixed lymphocyte reaction (MLR) using CD4⁺ T cells derived from human peripheral blood mononuclear cells (PBMC) and CD19⁺ B cells derived from a different donor (data not shown). When those T cells were to be stimulated again with B cells, the above-mentioned humanized anti-human PD-1 agonist antibody or CTLA-4-Ig (positive control) was added. After 12 hours, T cell-suppressive activity was evaluated using IFN-γ production in the supernatant as an indicator. As a result, inhibition of IFN-γ production was seen in CTLA-4-Ig but no remarkable inhibition was seen in the humanized anti-human PD-1 agonist antibody.

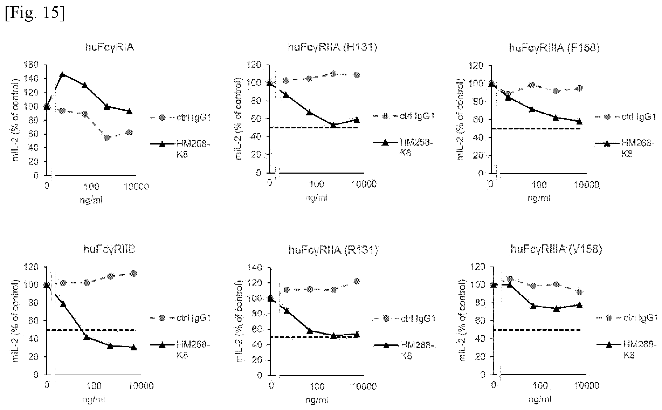

[Fig. 15] Exertion of the agonistic activity of anti-human PD-1 agonist antibody depends on Fc receptors, especially on FcγRIIB. The IL-2 production suppressive activity of the humanized anti-human PD-1 agonist antibody was examined with the same evaluation system as shown in Figs. 3 and 12. The IIA1.6 cells used here express human FcyRIA, FcyRIIA (H131), FcyRIIA (R131), FcγRIIB, FcγRIIIA (F158) or FcyRIIIA (V158) individually at similar high levels. When human FcγRIIB, FcyRIIA (H131), FcyRIIA (R131), FcγRIIIA (F158) or FcγRIIIA (V158)-expressing cells were used, IL-2 production was suppressed by humanized anti-human PD-1 agonist antibody. In particular, the highest IL-2 production suppressive activity was exhibited when FcyRIIB-expressing cells were used. When FcyRIA-expressing cells were used, IL-2 production increased rather than decreased by the addition of the antibody. These results suggest that humanized anti-human PD-1 agonist antibody exerts the highest agonist activity in the presence of FcyRIIB in particular.

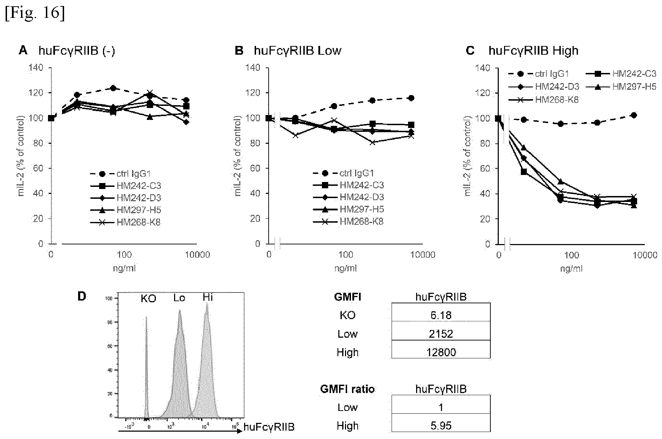

[Fig. 16] Exertion of the agonistic activity of anti-human PD-1 antibody depends on the expression level of Fc receptors. In the evaluation system shown in Fig. 3, (A) when IIA1.6 cells not expressing Fc receptors were used, humanized anti-human PD-1 antibody did not show agonistic activity. (B,C) On the other hand, when IIA1.6 cells rendered to express FcyRIIB were used, humanized anti-human PD-1 antibody showed agonistic activity and the activity depended on the expression level of FcγRIIB. (D) In FcyRIIB high expression cells and FcyRIIB low expression cells, expression levels of FcyRIIB were measured by FACS. The results revealed that there was approximately a 6-fold difference in geometric mean fluorescence intensity (GMFI).

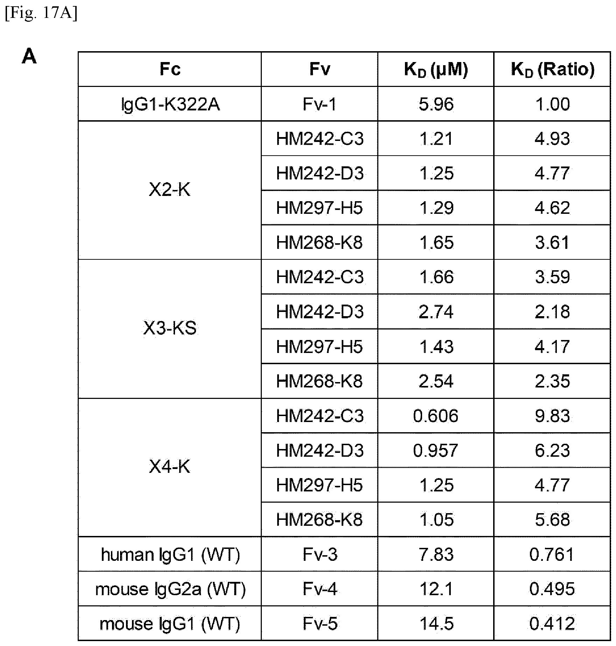

[Fig. 17A] Improvement of affinity to human FcyRIIB by modification of Fc region. (A) Affinity of Fc region-modified antibody to human FcyRIIB (K_{D} value). The affinity of human IgG1 Fc region-modified antibodies X2-K, X3-KS and X4-K to human FcyRIIB was improved by 2.18 to 9.83 folds compared to the affinity of IgG1-K322A.

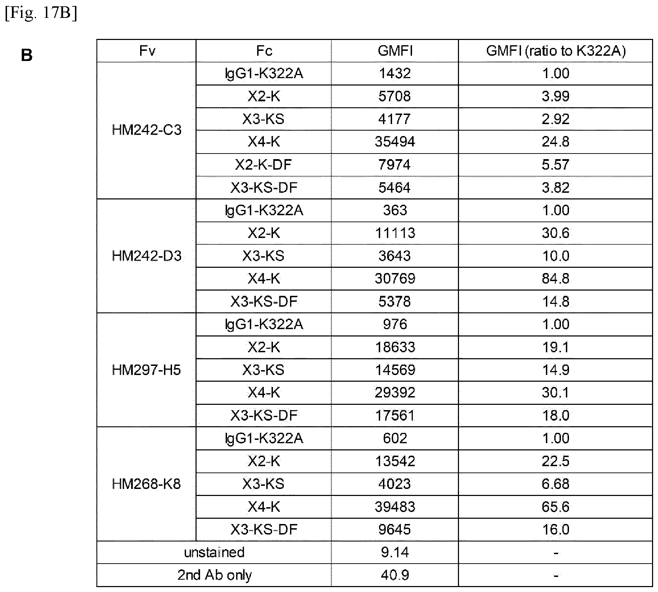

[Fig. 17B] Improvement of affinity to human FcyRIIB by modification of Fc region. (B) Affinity of Fc region-modified antibody to human FcyRIIB (GMFI). The affinity of human IgG1 Fc region-modified antibodies X2-K, X3-KS and X4-K to human FcyRIIB was improved by 2.92 to 84.8 folds compared to IgG1-K322A having the same Fv region. "DF" means defucosylated.

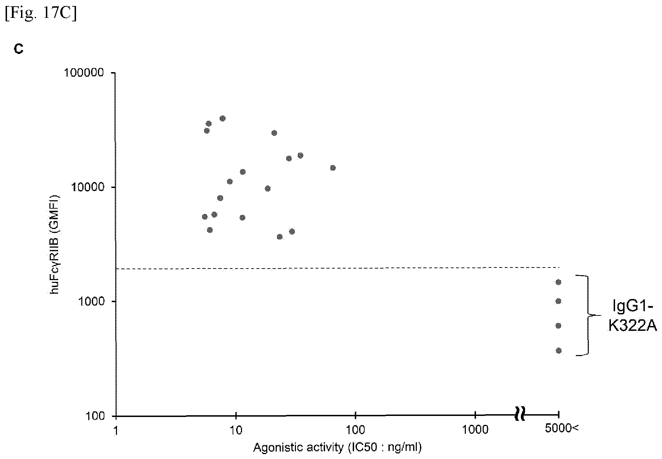

[Fig. 17C] Improvement of affinity to human FcyRIIB by modification of Fc region. (C) Correlation between affinity of Fc region-modified antibody to human FcyRIIB (GMFI) and its agonistic activity (IC50: ng/ml). Individual plots represent the antibodies shown in Fig. 17B. When Fv region was the same, the tendency was such that the higher the affinity of Fc region to human FcγRIIB, the higher the agonistic activity.

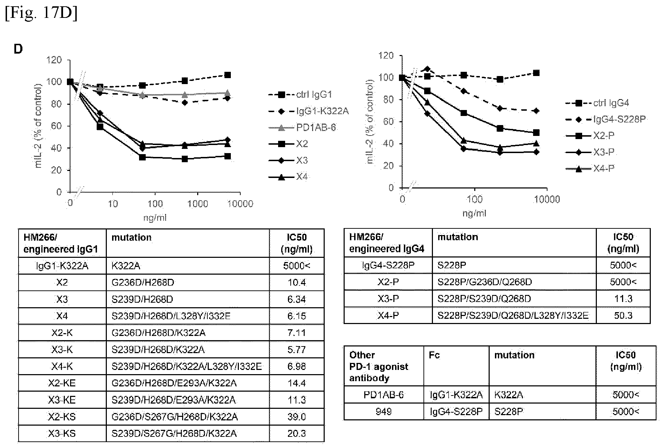

[Fig. 17D] Improvement of affinity to human FcyRIIB by modification of Fc region. (D) Fc region-modified chimeric antibodies based on HM266 were prepared and their agonistic activities were evaluated. When IIA1.6 cells with low expression of human FcyRIIB were used, IgG1-K322A type and IgG4-S228P type antibodies, and known anti-human PD-1 agonist antibodies PD1AB-6 and 949 only showed a weak agonistic activity, whereas each of the Fc region-modified antibodies with improved affinity to human FcyRIIB showed a high agonistic activity.

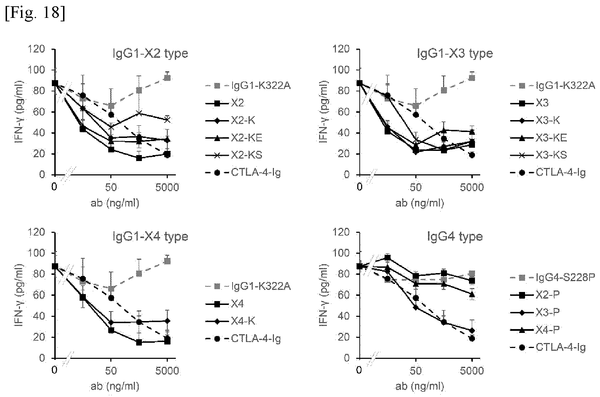

[Fig. 18] Immunosuppressive action of anti-human PD-1 agonist-human Fc region chimeric antibody in an evaluation system using human cells. Chimeric antibodies having the Fab region of HM266 and the Fc region of various Fc region-modified antibodies, chimeric antibodies with IgG1-K322A, and CTLA-4-Ig were evaluated using the MLR system of CD4⁺ T cells and CD19⁺ B cells. As a result, those chimeric antibodies whose affinity to human FcyRIIB had been improved by having the Fc region of Fc region-modified antibodies had a greatly improved T cell-suppressive activity compared to original IgG1-K322A from which they were derived, and they exerted an anti-inflammatory action superior to that of CTLA-4-Ig.

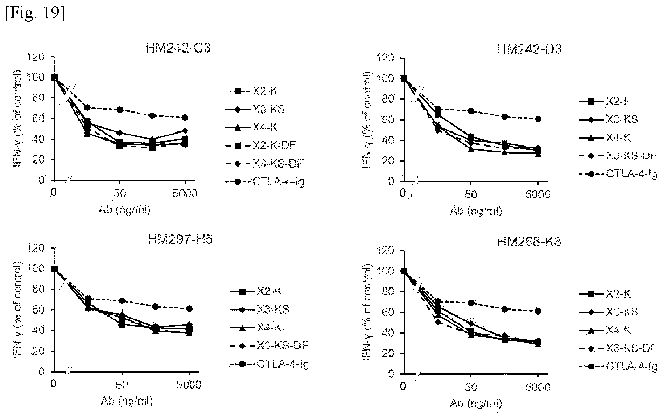

[Fig. 19] Anti-inflammatory action of humanized anti-human PD-1 agonist antibody in an evaluation system using human cells. Humanized antibodies having a combination of the selected humanized Fab region (see Fig. 13) and the Fc region of human Fc region-modified antibodies were evaluated in the MLR system of CD4⁺ T cells and CD19⁺ B cells. It should be noted that all the antibodies shown here are Fc region-modified human IgG1. As a result, all of the Fc region-modified humanized antibodies with improved affinity to FcyRIIB exerted a more potent T cell-suppressive action than CTLA-4-Ig, suggesting that these humanized antibodies may be able to exhibit an anti-inflammatory action mediated by inhibition of human T cell function.

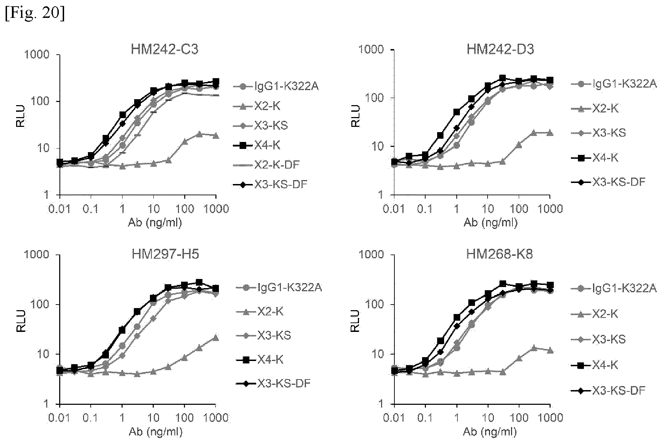

[Fig. 20] Antibody-dependent cellular cytotoxicity (ADCC) activity of humanized anti-human PD-1 agonist antibody. Antibodies with X2-K as Fc region had low ADCC activity; antibodies with X3-KS as Fc region had ADCC activity comparable to that of IgG1-K322A; and antibodies with X4-K as Fc region or defucosylated X3-KS (X3-KS-DF) had high ADCC activity.

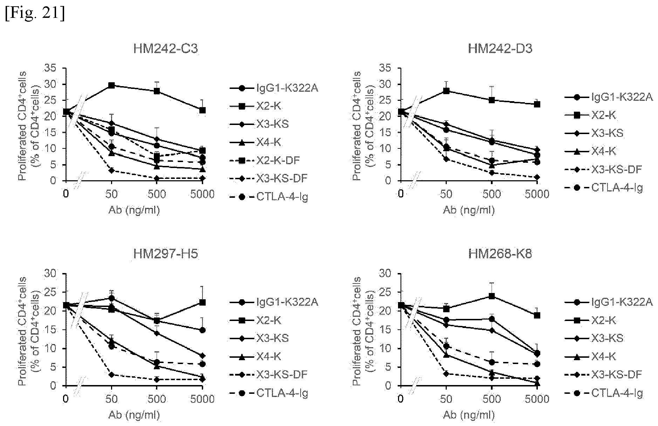

[Fig. 21] Suppressive action of humanized anti-human PD-1 agonist antibody against antigen-specific T cell activation. CD4⁺ T cells activated and proliferated upon stimulation of human PBMC with tetanus toxoid were monitored by the decay of carboxyfluorescein succinimidyl ester (CFSE) fluorescence in CD4⁺ T cells. The data shows the percentage of CD4⁺ T cells whose CFSE fluorescence intensity was weakened as a result of cell proliferation. The antibodies showing high ADCC activity in Fig. 20 showed a high proliferation suppressive action comparable to or higher than that of CTLA-4-Ig. These results suggested that antibodies having high ADCC activity in addition to agonistic activity have a suppressive action against a wider range of inflammatory diseases.

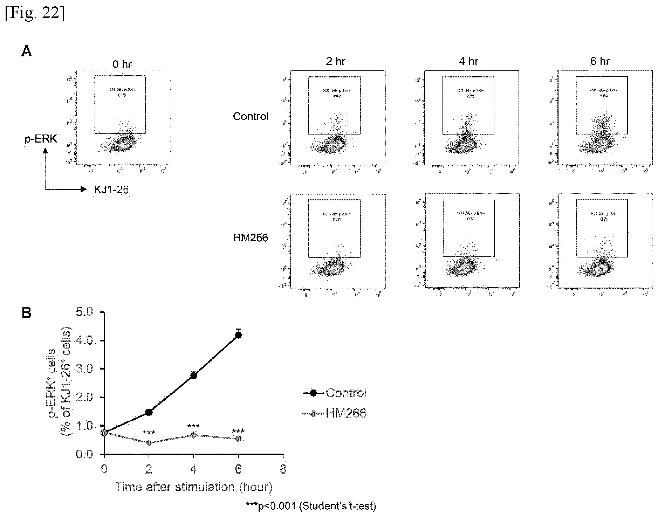

[Fig. 22] ERK phosphorylation suppressive action of anti-human PD-1 agonist antibody. Evaluation was performed in the same manner as shown in Fig. 3 using a system in which DO11.10 T cell hybridomas rendered to express human PD-1 were combined with IIA1.6 B cell lymphoma cells rendered to express not PD-L1 but mouse FcγRIIB. HM266 suppressed time-dependent increase in phosphorylated ERK positive ratio in DO11.10. This result suggested that anti-human PD-1 agonist antibody suppresses the downstream signal transduction of T cell receptors.

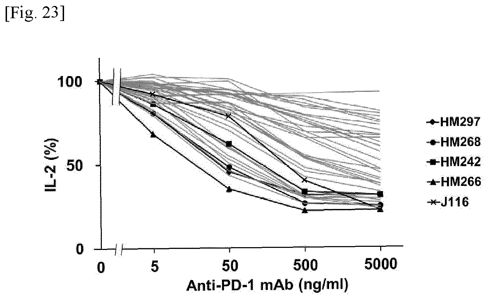

[Fig. 23] The same graph as Fig. 3B but highlighting HM266, HM242, HM297, HM268 and J116. HM266, HM242, HM297 and HM268 showed higher agonistic activity than 1116.

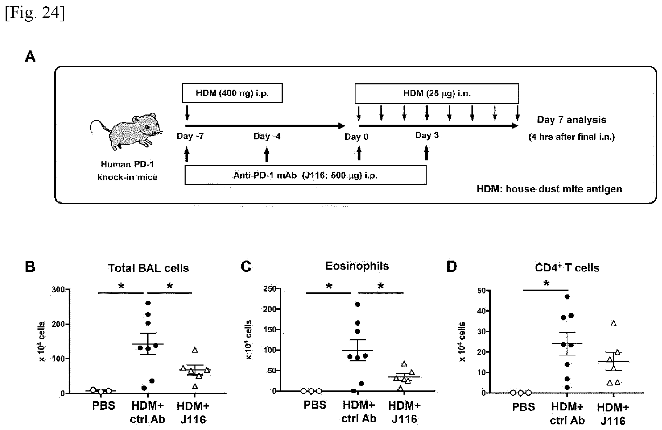

[Fig. 24] Induction of allergic asthma in mouse and anti-inflammatory action of anti-human PD-1 agonist antibody. (A) A diagram showing a method of induction of allergic asthma and a schedule of antibody administration. Allergic asthma was induced according to the same schedule as shown in Fig. 6. (B-D) By administration of J116 which showed a weaker activity than HM266 in the evaluation system for *in vitro* PD-1 agonist activity in Fig. 3, the total number of inflammatory cells infiltrating the tissue was suppressed (B) and, in particular, infiltration of eosinophils was suppressed significantly (C) whereas no significant suppressive effect was observed with respect to CD4⁺ T cells (D).

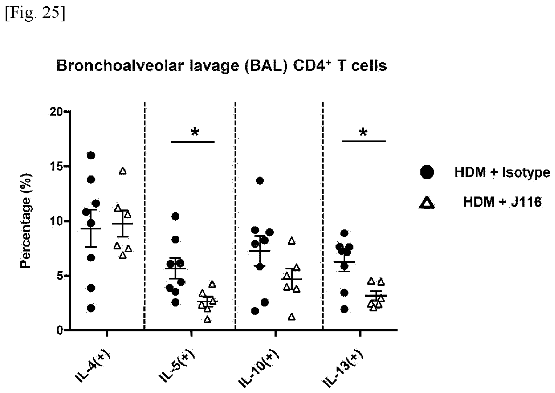

[Fig. 25] Anti-inflammatory action of anti-human PD-1 agonist antibody against allergic asthma and inhibition of Th2 type immune response. Allergic asthma was induced according to the same schedule as shown in Fig. 6, and bronchoalveolar lavage fluid was collected. Cytokine production in CD4⁺ cells infiltrating into the alveoli was examined by intracellular staining. As a result, a decrease in IL-5 and IL-13 producing cells was observed in J116 administration group, but a decrease in IL-4 producing cells and IL-10 producing cells as observed upon administration of HM266 was not observed. These results suggested the usefulness of anti-human PD-1 agonist antibody for treatment of allergic diseases, and also showed a tendency of correlation between *in vitro* agonistic activity and the effectiveness of *in vivo* model.

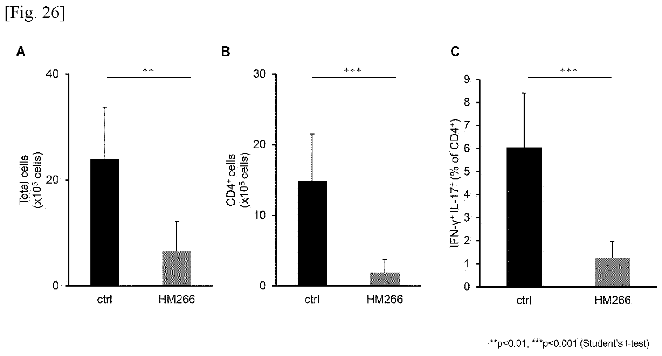

[Fig. 26] Induction of colitis in mouse and anti-inflammatory action of anti-human PD-1 agonist antibody. T cell-dependent colitis was induced by transferring human PD-1 knock-in mouse-derived CD25⁻CD4⁺ T cells into RAG2 knock-out mice. (A-C) Analysis of the colon collected 8 weeks after cell transfer showed that compared to a control, the total number of cells in the lamina propria (A), CD4⁺ T cells infiltrating the lamina propria (B) and the ratio of IFN-γ positive and IL-17 positive cells in infiltrating CD4⁺ T cells (C) decreased significantly. These results suggested the applicability of anti-human PD-1 agonist antibody to treatment of inflammatory bowel diseases.

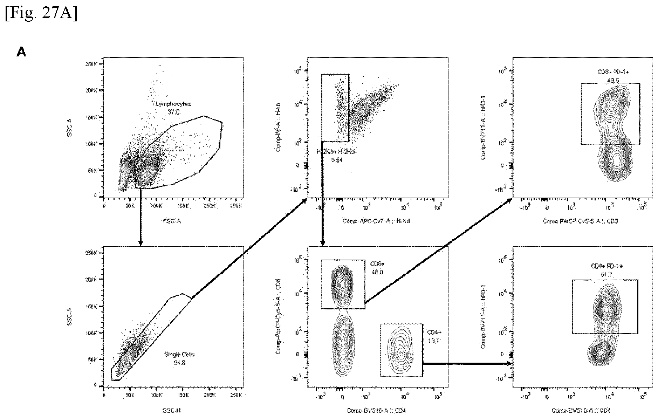

[Fig. 27A] Induction of acute GVHD in mouse and anti-inflammatory action of anti-human PD-1 agonist antibody. Splenocytes derived from C57BL/6 human PD-1 knock-in mice (donor) were transferred into BDF1 mice (recipient) to thereby induce acute GVHD. (A) A figure showing the expression of human PD-1 on CD4⁺ or CD8⁺ T cells in donor-derived cells (H-2K^{b+}H-2K^{d-}). In GVHD-induced mice, 61.7% of donor-derived CD4⁺ T cells and 49.5% of donor-derived CD8⁺ T cells were positive for human PD-1.

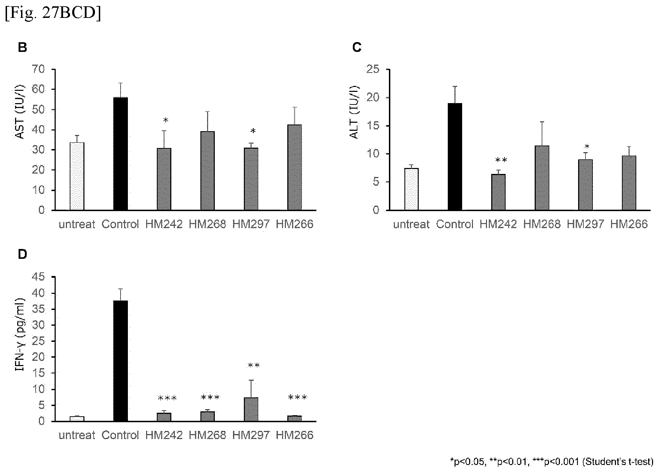

[Fig. 27BCD] Induction of acute GVHD in mouse and anti-inflammatory action of anti-human PD-1 agonist antibody. (B-D) AST (B), ALT (C) and IFN-γ (D) in mouse plasma 2 weeks after induction. Induction of GVHD increased the plasma levels of ALT, AST, and IFN-γ, but preventive administration of HM266, HM242, HM297 and HM268 suppressed these increases remarkably.

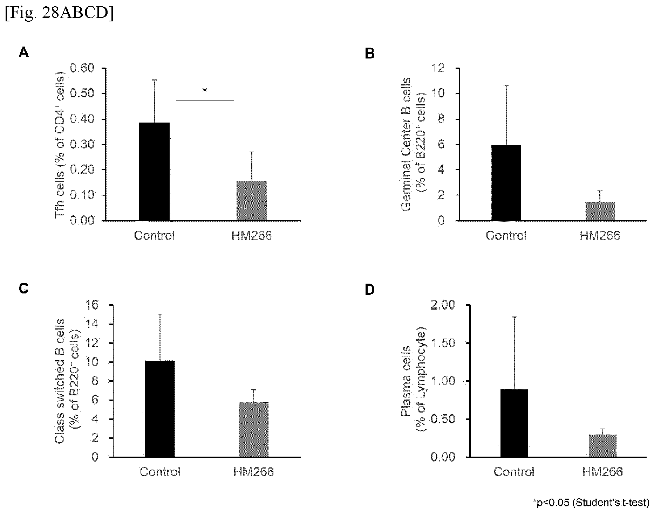

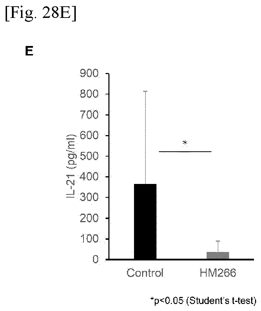

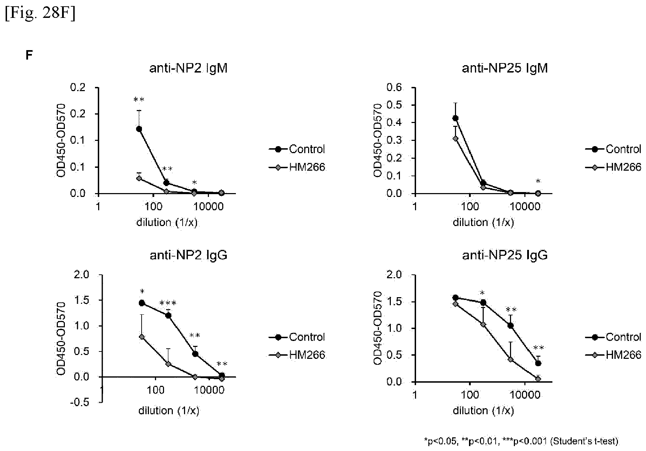

[Fig. 28ABCD] Induction of T cell-dependent antibody response in mouse and antibody production suppressive action of anti-human PD-1 agonist antibody. T cell-dependent antibody response was induced in C57BL/6 human PD-1 knock-in mice by intraperitoneal administration of 4-hydroxy-3-nitrophenylacetyl ovalbumin (NP-OVA) antigen together with alum adjuvant. Administration of HM266 suppressed the ratios of Tfh cells (A), germinal center B cells (B), class switched B cells (C) and plasma cells (D) in the mouse spleen 10 days after antigen administration.
[Fig. 28E] Further, an increase in blood IL-21 concentration was also observed as a result of antigen administration, but administration of HM266 suppressed this increase (E).
[Fig. 28F] Furthermore, analysis of the antibody titer in plasma conducted 7 days after antigen administration revealed that low-affinity IgG antibody titer (anti-NP25 IgG) and high-affinity IgG/IgM antibody titer (anti-NP2 IgG or IgM) had been suppressed (F). These results suggest a possibility that administration of anti-human PD-1 agonist antibody may suppress antibody class switching and affinity maturation.

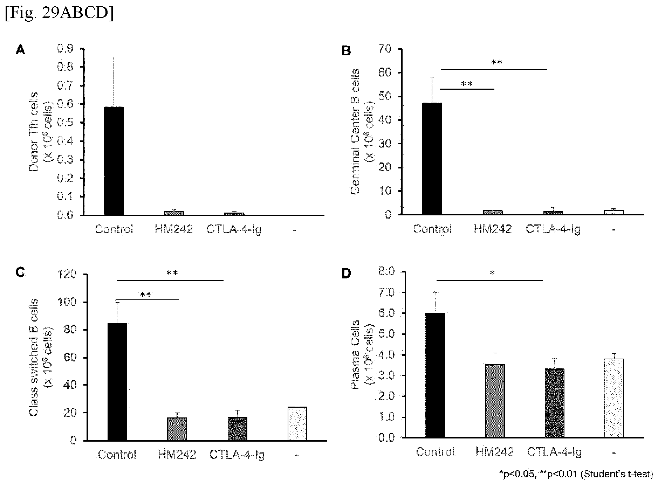

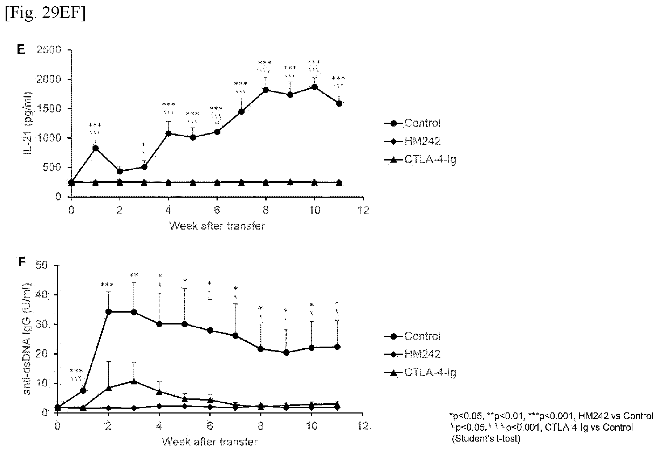

[Fig. 29ABCD] Induction of chronic GVHD/lupus-like symptoms in mouse and anti-inflammatory action of anti-human PD-1 agonist antibody. Splenocytes derived from C57BL/6 human PD-1 knock-in mice (donor) were freed of CD8⁺ cells and then transferred into BDF1 mice (recipient) to thereby induce chronic GVHD/lupus-like symptoms. After cell transfer, donor-derived Tfh cells (A), recipient-derived germinal center B cells (B), class switched B cells (C) and plasma cells (D) were confirmed to increase remarkably in the recipient's spleen. When HM242 or CTLA-4-Ig was preventively administered to this inflammation model, these increases were suppressed. Mark "-" represents recipient mice into which no donor cells were transferred.
[Fig. 29EF] Induction of chronic GVHD/lupus-like symptoms in mouse and anti-inflammatory action of anti-human PD-1 agonist antibody. Splenocytes derived from C57BL/6 human PD-1 knock-in mice (donor) were freed of CD8⁺ cells and then transferred into BDF1 mice (recipient) to thereby induce chronic GVHD/lupus-like symptoms. After cell transfer, IL-21 concentration (E) and anti-dsDNA antibody titer (F) were confirmed to increase remarkably in the blood. Prophylactic administration of HM242 or CTLA-4-Ig to this inflammation model suppressed the increase in blood anti-dsDNA antibody. These results suggested the applicability of anti-human PD-1 agonist antibody to treatment of inflammatory diseases that involve autoantibody production.

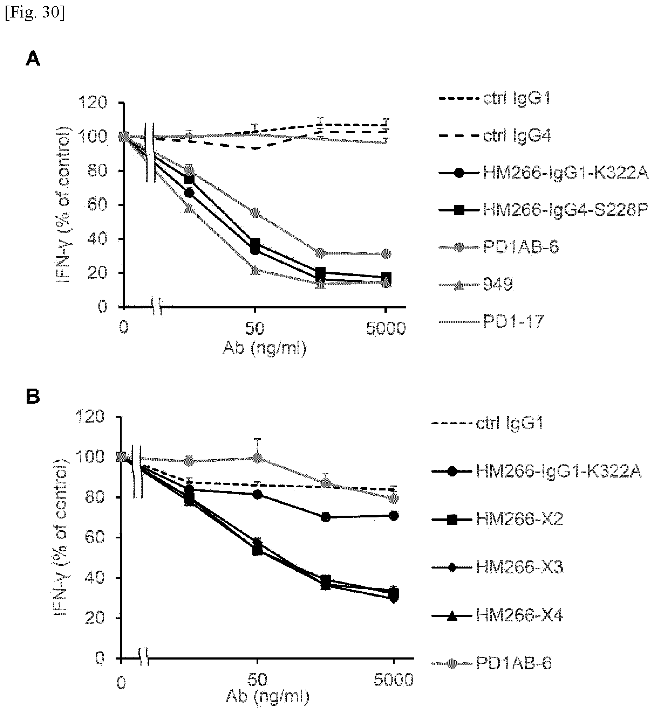

[Fig. 30] Immunosuppressive action of anti-human PD-1 agonist-human Fc region chimeric antibody in an evaluation system using human cells. Chimeric antibodies having the Fab region of HM266 and the Fc region of various Fc region-modified antibodies, and chimeric antibodies with the Fc region of IgG1-K322A or IgG4-S228P were evaluated in a co-culture system of CD4⁺ T cells and a human monocytic cell line THP-1 using IFN-γ production as an indicator. When evaluation was performed using THP-1 into which human FcyRIIB gene had been artificially introduced to be expressed highly, chimeric antibodies having the Fab region of HM266 and the Fc region of IgG1-K322A or IgG4-S228P exhibited an immunosuppressive activity comparable to or greater than that of known antibodies (A). On the other hand, in an evaluation system using THP-1 which did not undergo gene transfer but which expressed endogenous FcγRIIB, chimeric antibodies with IgG1-K322A or IgG4-S228P and known antibodies did not exhibit an immunosuppressive activity but each of the Fc region-modified antibodies with improved affinity to human FcyRIIB exhibited high immunosuppressive activity (B). These results revealed that those chimeric antibodies whose affinity to human FcyRIIB was improved by having the Fc region of Fc region-modified antibodies were greatly improved in T cell suppressive activity compared to original IgG1-K322A from which they were derived.

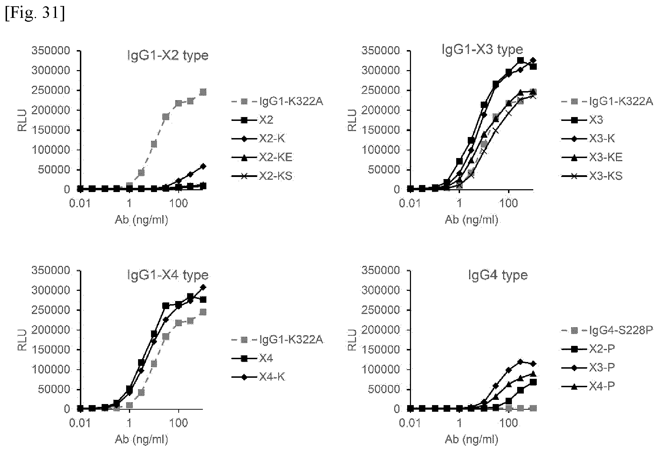

[Fig. 31] ADCC activity of anti-human PD-1 agonist-human Fc region chimeric antibody. Antibodies with X2 type as Fc region had low ADCC activity; and antibodies with X3 or X4 type as Fc region had high ADCC activity at least comparable to that of antibodies with IgG1-K322A type as Fc region. Fc region-modified antibodies based on the Fc region of IgG4-S228P had an improved ADCC activity compared to IgG4-S228P, but it was not as high as the ADCC activities of IgG1-K322A type as well as X3 and X4 types.

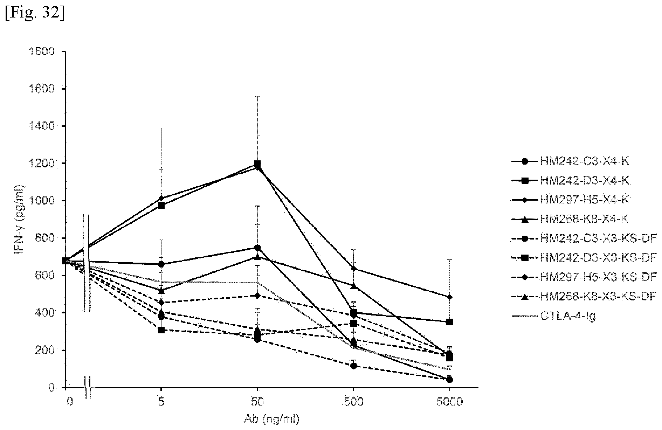

[Fig. 32] Suppressive action of humanized anti-human PD-1 agonist antibody against antigen-specific T cell activation. The immunosuppressive activity was evaluated using, as an indicator, IFN-γ produced when human PBMC was stimulated with tetanus toxoid. The antibodies showing a high CD4⁺ cell proliferation suppressive effect at least comparable to that of CTLA-4-Ig in Fig. 21 also suppressed the production of IFN-γ, but the antibodies with X3-KS-DF as Fc region inhibited the production of IFN-γ even in low concentration ranges.

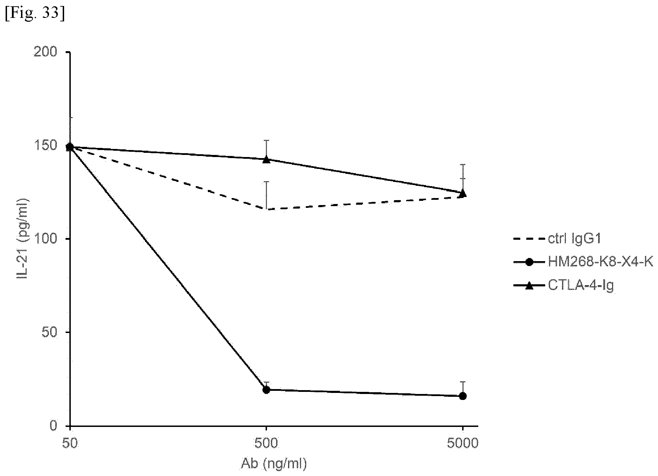

[Fig. 33] Suppressive action of humanized anti-human PD-1 agonist antibody against activation of human Tfh cells. Human CD4⁺ T cells that had been differentiated into Tfh-like cells by culturing in the presence of individual cytokines were co-cultured with THP-1 cells, stimulated by CytoStim and evaluated the activation using IL-21 as an indicator. As a result, humanized anti-human PD-1 agonist antibody suppressed the IL-21 production by Tfh-like cells.

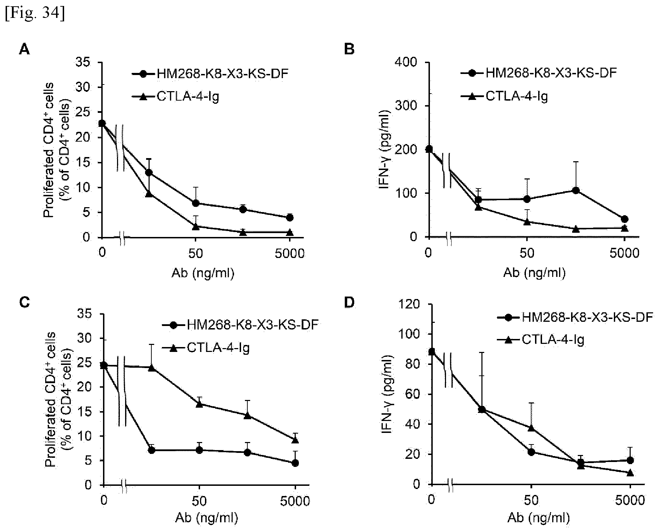

[Fig. 34] Immunosuppressive action of humanized anti-human PD-1 agonist antibody against autoimmune disease patient-derived cells. PBMC derived from patients with rheumatoid arthritis (RA) (A, B) and systemic lupus erythematosus (SLE) (C, D) were cultured in the presence of autoantigen and evaluated the activation using the proliferation of CD4⁺ T cells (A, C) and IFN-γ production (B, D) as indicators. As a result, humanized anti-human PD-1 agonist antibody showed suppressive activity against the activation of patient-derived cells in response to self-antigen stimulation equal to or more than CTLA-4-Ig.

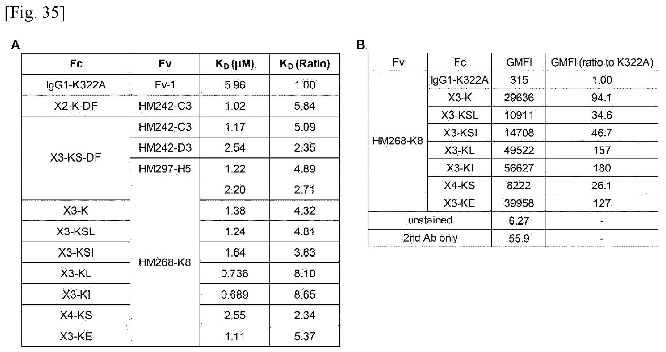

[Fig. 35] Improvement of affinity to human FcyRIIB by modification of Fc region. (A) Affinity of Fc region-modified antibodies to human FcyRIIB (K_{D} value). The affinity of human IgG1 Fc region-modified antibodies to human FcyRIIB was improved by 2.34 to 8.65 folds compared to that of IgG1-K322A. (B) Affinity of Fc region-modified antibodies to human FcyRIIB (GMFI). The affinity of human IgG1 Fc region-modified antibodies to human FcyRIIB was improved by 26.1 to 180 folds compared that of IgG1-K322A.

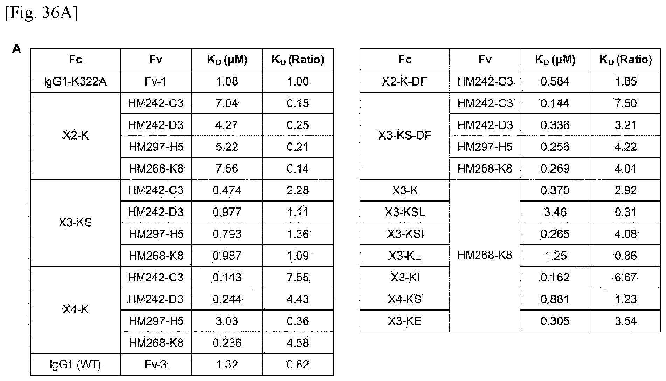

[Fig. 36A] Improvement of affinity for human FcγRIIIA by modification of Fc region. (A) Affinity (K_{D} value) of Fc region-modified antibodies to human FcγRIIIA (V158). The affinity to human FcγRIIIA (V158) of human IgG1 antibodies in which the Fc region was modified with X4-K, X3-KS-DF, X3-K, X3-KSI, X3-KI or X3-KE was improved by 2.92 to 7.55 folds compared to that of antibody with IgG1-K322A as Fc region.

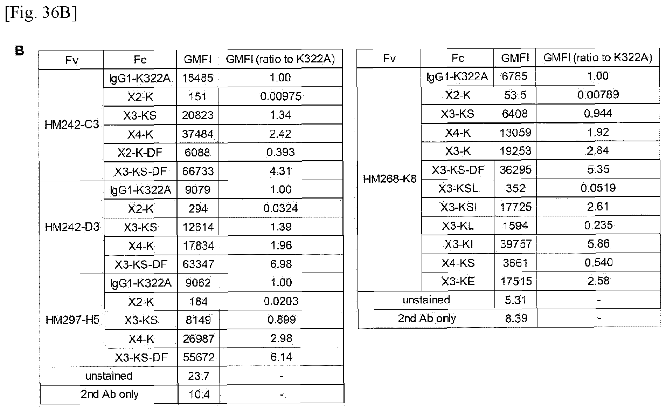

[Fig. 36B] Improvement of affinity for human FcγRIIIA by modification of Fc region. (B) Affinity of Fc region-modified antibodies to human FcγRIIIA (V158) (GMFI). The affinity to human FcγRIIIA (V158) of human IgG1 antibodies in which the Fc region was modified with X4-K, X3-KS-DF, X3-K, X3-KSI, X3-KI and X3-KE was improved by 1.92 to 6.98 folds compared to IgG1-K322A having the same Fv region.

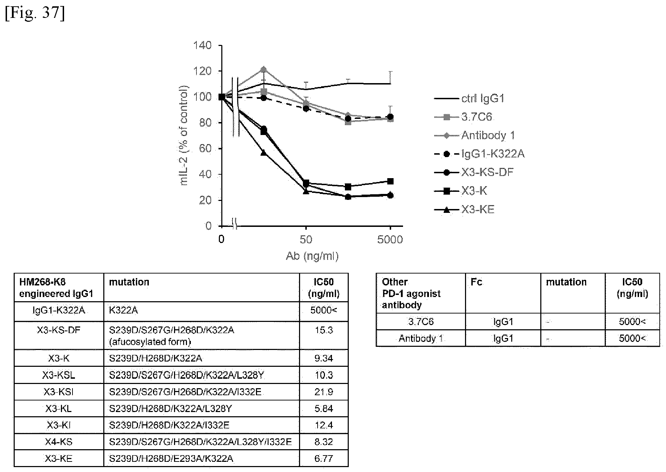

[Fig. 37] Improvement of agonistic activity by modification of Fc region. As a result of evaluation using IIA1.6 cells with low expression of human FcγRIIB, the known anti-human PD-1 agonist antibodies 3.7C6 and Antibody 1 showed only a weak agonistic activity, but each of the Fc region-modified antibodies with an improved affinity to human FcyRIIB showed high agonistic activity.

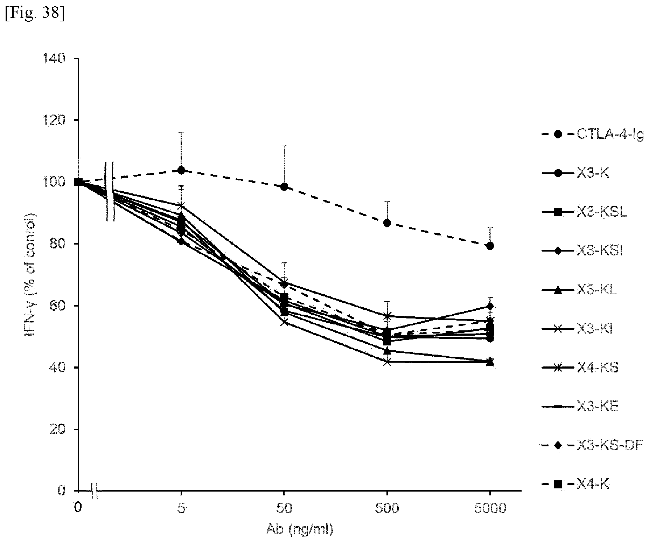

[Fig. 38] Anti-inflammatory action of humanized anti-human PD-1 agonist antibody in an evaluation system using human cells. Humanized antibodies having a combination of the humanized Fab region of HM268-K8 and the Fc region of human Fc region-modified antibodies were prepared, and evaluated by the MLR system of CD4⁺ T cells and CD19⁺ B cells in the same manner as in Fig. 19. As a result, all the Fc region-modified humanized antibodies with an improved affinity to FcyRIIB exerted a more potent T cell-suppressive action than CTLA-4-Ig, suggesting that these humanized antibodies are able to show an anti-inflammatory action through inhibition of human T cell function.

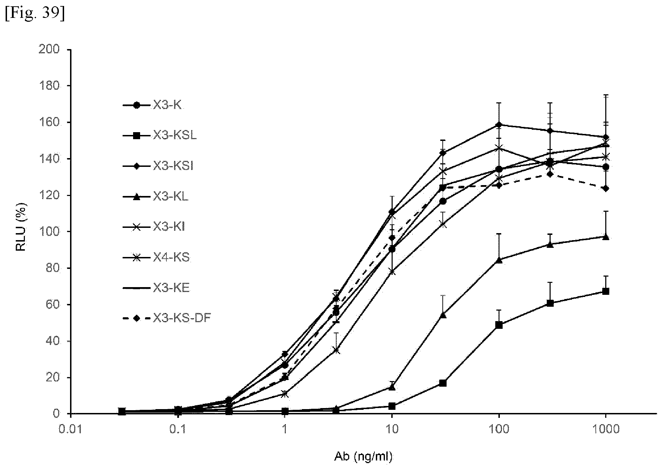

[Fig. 39] ADCC activity of humanized anti-PD-1 agonist antibody. Antibodies with X3-KSL or X3-KL as Fc region had low ADCC activity, whereas antibodies with X3-KSI, X3-KI or X3-KS-DF as Fc region had high ADCC activity.

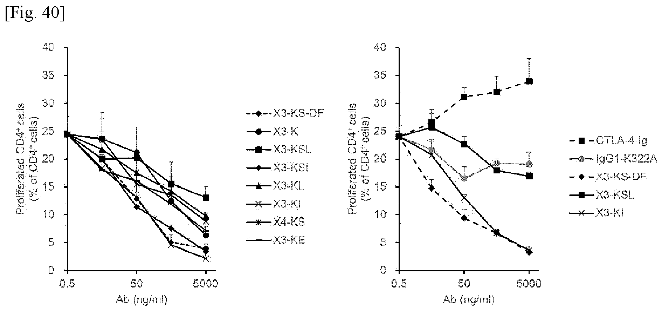

[Fig. 40] Suppressive action of humanized anti-human PD-1 agonist antibody against antigen-specific T cell activation. In the same manner as in Fig. 21, CD4⁺ T cells activated and proliferated upon stimulation of human PBMC with tetanus toxoid were monitored by the decay of carboxyfluorescein succinimidyl ester (CFSE) fluorescence in CD4⁺ T cells. The data shows the percentage of CD4⁺ T cells whose CFSE fluorescence intensity was weakened as a result of cell proliferation. The antibodies showing high ADCC activity in Fig. 39 showed an especially high proliferation suppressive effect. In the case of using cells derived from a different donor than the cells used in Fig. 21, CTLA-4-Ig did not show any proliferation suppressive effect. These results suggested that antibodies having high ADCC activity in addition to a high agonistic activity have a suppressive effect on a wider range of inflammatory diseases and can consistently exert a suppressive effect on different donors.

### BEST MODES FOR CARRYING OUT THE INVENTION

Hereinbelow, embodiments of the present invention will be described in detail.

The present invention provides an agonist antibody to human PD-1 (anti-human PD-1 agonist antibody) or a functional fragment thereof, wherein the antibody or a functional fragment thereof
(a) binds to domain #7 of human PD-1 as shown in SEQ ID NO: 1, and
(b) has an improved affinity to human Fc receptors.

As used herein, the "anti-human PD-1 agonist antibody" may be antibodies which bind to human PD-1 and trigger an intracellular signal transduction system similar to that triggered by human PD-L1 in the human body. Such antibodies may be selected in a co-culture system of T cells rendered to express human PD-1 and antigen-presenting cells rendered to express human FcγRIIB, using as an indicator the activity to suppress IL-2 which is produced from T cells in an antigen stimulation dependent manner (see Example described later).

The antibody of the present invention or a functional fragment thereof may bind to only domain #7 of human PD-1 (SEQ ID NO: 9; domain spanning from amino acid No. 38 to No. 48 in the amino acid sequence as shown in SEQ ID NO: 1) or may also bind to other domains of human PD-1 such as domain #6 (SEQ ID NO: 8; domain spanning from amino acid No. 109 to No. 120 in the amino acid sequence as shown in SEQ ID NO: 1) or domain #1 (SEQ ID NO: 3; domain spanning from amino acid No. 129 to No. 139 in the amino acid sequence as shown in SEQ ID NO: 1). Preferably, the antibody or a functional fragment thereof binds to only domain #7 of human PD-1. When the binding capacity of an anti-human PD-1 antibody is decreased by replacing domain #7 of human PD-1 as shown in SEQ ID NO: 1 with the amino acid sequence of the corresponding domain in the amino acid sequence of mouse PD-1 (SEQ ID NO: 2), the anti-human PD-1 antibody can be defined as binding to domain #7 of human PD-1 as shown in SEQ ID NO: 1. Here, the binding to domain #7 is judged independently from the binding to other domains of human PD-1. The binding to domains of human PD-1 other than #7 is defined in the same manner. The amino acid sequence of human PD-1 is registered at the NCBI database under accession number: NP_005009.2, and the amino acid sequence of mouse PD-1 at the NCBI database under accession number: NP_032824.1. Preferably, the antibody of the present invention or a functional fragment thereof also binds to substituted mouse PD-1 (Mouse PD-1 (hu38-48)) in which the amino acid sequence of domain #7 of mouse PD-1 is replaced with the amino acid sequence of domain #7 of human PD-1 and/or mutant human PD-1 (R143A point mutant) in which arginine at position 143 of human PD-1 is mutated to alanine.

The binding capacity (binding affinity) of the antibody of the present invention or a functional fragment thereof to human PD-1 may be 10⁻⁷ M or less, preferably 10⁻⁸ M or less, in terms of equilibrium dissociation constant (K_{D}). Equilibrium dissociation constant is measured by the surface plasmon resonance (SPR) method. The binding capacity of the antibody of the present invention or a functional fragment thereof to human PD-1 can also be measured by flow cytometry using the concentration dependency of binding to PD-1 expressing cells.

Examples of the sequences of the heavy chain CDR1 to CDR3 and the light chain CDR1 to CDR3 of the antibodies of the present invention are shown in Table I below. CDRs may be identified by algorithms such as Kabat (J Exp Med. 1970; 132: 211-50), Chothia (J Mol Biol. 1987; 196: 901-17), IMGT (Dev Comp Immunol. 2003; 27: 55-77) and Paratome (PLoS Comput Biol. 2012; 8: e1002388), preferably by Kabat's method. In Table I, HM242, HM266, HM268 and HM297 are mouse anti-human PD-1 antibodies exhibiting PD-1 agonist activity that were prepared by immunizing mice with human PD-1. The antibody of the present invention may advantageously have a heavy chain variable region comprising heavy chain CDR1, CDR2 and CDR3 and a light chain variable region comprising light chain CDR1, CDR2 and CDR3, as shown in Table I below. In the antibody of the present invention, the heavy chain CDR1, CDR2 and CDR3 and the light chain CDR1, CDR2 and CDR3 may comprise sequences having at least 85%, 85-90%, 90-95%, 95-97% or 97% or more identity with the sequences as shown in Table I, in terms of CDR as a whole. Identity between two sequences can be determined using BLAST.

**[Table I] Sequences of the heavy chain CDR1, CDR2 and CDR3 and the light chain CDR1, CDR2 and CDR3 in HM242, HM266, HM268 and HM297**

| | | **Heavy Chain** | | | **Light Chain** | | |
|---|---|---|---|---|---|---|---|
| | | **CDR1** | **CDR2** | **CDR3** | **CDR1** | **CDR2** | **CDR3** |
| **HM242** | **Kabat** | SYYIQ | WIYPGDGTTKYNEKFKG | YYGSYFDY | RASQDISNYLN | YTSKLHS | QQSSTLPFT |
| | **Chothia** | GYTFTSY | YPGDGT | YYGSYFDY | RASQDISNYLN | YTSKLHS | QQSSTLPFT |
| | **IMGT** | GYTFTSYY | IYPGDGTT | ASYYGSYFDY | QDISNY | YTS | QQSSTLPFT |
| | **Paratome** | YTFTSYYIQ | WIGWIYPGDGTTKY | SYYGSYFDY | QDISNY | LLIYYTSKLHS | QQSSTLPF |
| **HM266** | **Kabat** | SYYIQ | WIYPGDGSSKYNEKFKG | YYGSSFDY | RASQDISNYLN | YSSRLHS | QQGSTLPFT |
| | **Chothia** | GYTFTSY | YPGDGS | YYGSSFDY | RASQDISNYLN | YSSRLHS | QQGSTLPFT |
| | **IMGT** | GYTFTSYY | IYPGDGSS | ASYYGSSFDY | QDISNY | YSS | QQGSTLPFT |
| | **Paratome** | YTFTSYYIQ | WIGWIYPGDGSSKY | SYYGSSFDY | QDISNY | FLIYYSSRLHS | QQGSTLPF |
| **HM268** | **Kabat** | SYYIQ | WIYFGDASTKYNEKFKG | YYGSYFDY | RASQDISNYLN | STSRLHS | QQGSTLPFT |
| | **Chothia** | GYTFTSY | YFGDAS | YYGSYFDY | RASQDISNYLN | STSRLHS | QQGSTLPFT |
| | **IMGT** | GYTFTSYY | VYFGDAST | ASYYGSYFDY | QDISNY | STS | QQGSTLPFT |
| | **Paratome** | YTFTSYYIQ | WIGWIYFGDASTKY | SYYGSYFDY | QDISNY | LLIYSTSRLHS | QQGSTLPF |
| **HM297** | **Kabat** | DYNVD | DIDPNNGGTIYNQKFKD | WRGAMDY | RASQDIDNYLK | DTSRLHS | QQGYTLPWT |
| | **Chothia** | GYTFTDY | DPNNGG | WRGAMDY | RASQDIDNYLK | DTSRLHS | QQGYTLPWT |
| | **IMGT** | GYTFTDYN | IDPNNGGT | ARWRGAMDY | QDIDNY | DTS | QQGYTLPWT |
| | **Paratome** | YTFTDYNVD | WIGDIDPNNGGTIY | RWRGAMDY | QDIDNY | LLIYDTSRLHS | QQGYTLPW |

The variable region (Fv) of the antibody of the present invention may be the Fv region of an antibody derived from an animal other than human (for example, mouse, rabbit, rat, hamster, guinea pig, goat, sheep, donkey, llama, camel, chicken, ostrich, shark, etc.) or it may be a humanized Fv region of the heavy chain and/or the light chain of an antibody derived from a non-human animal. Humanization may be performed, for example, by transplanting the CDRs of VH and VL of non-human animal-derived antibodies into the frameworks of VH and VL of human antibodies (Nature, 332, 323-327, 1988). Humanized antibody may be one in which CDR sequences are retained. Alternatively, humanized antibody may also be one in which antigen binding is improved by, for example, identifying the amino acid residues directly involved in binding to antigen, the amino acid residues interacting with CDRs and the amino acid residues involved in retaining the three-dimensional structure of CDRs, and replacing these residues with amino acid residues of non-humanized antibodies (MABS, 8(7), 1302-1318, 2016).

Table II below shows exemplary sequences of the heavy chain variable region (VH region; position 1 to 117; according to EU numbering; hereinafter, the same shall apply) of mouse-derived antibodies exhibiting PD-1 agonist activity, as well as examples of their humanized VH region sequences.

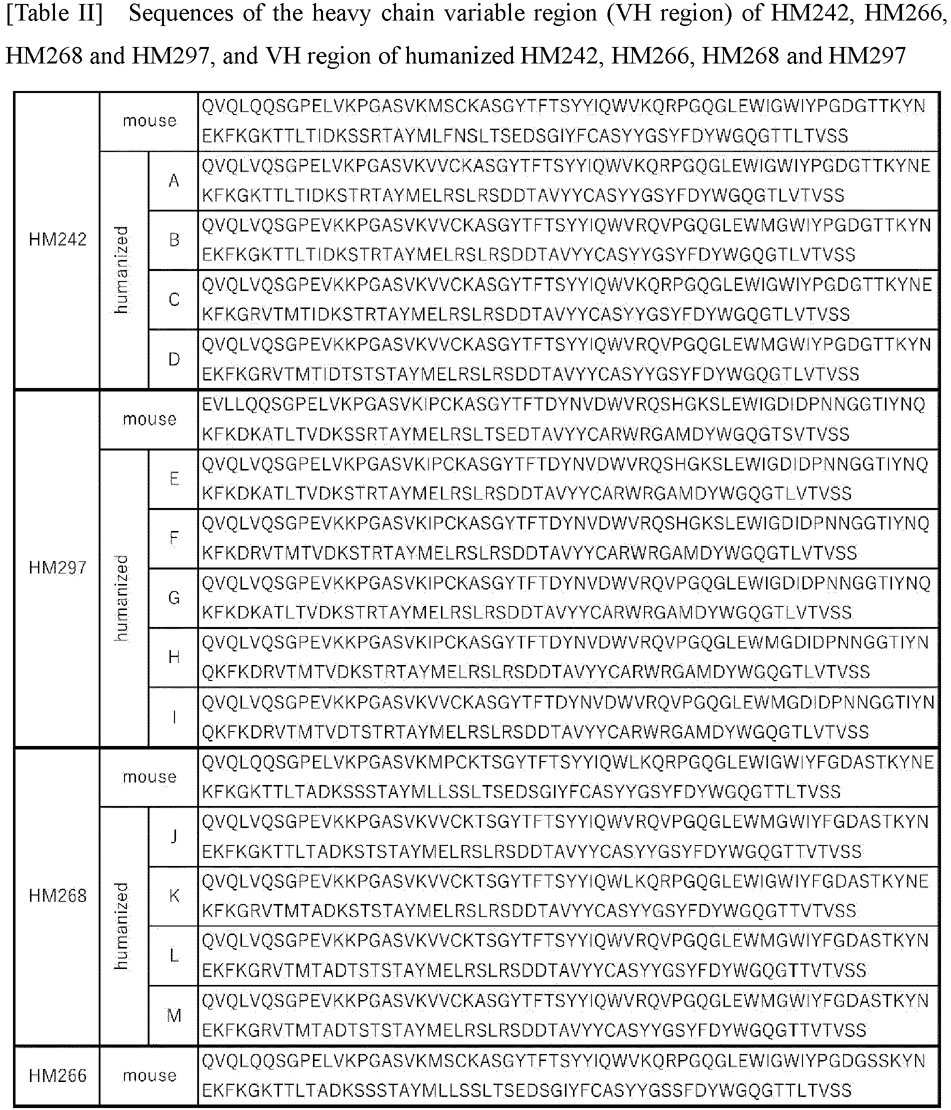

Table III below shows exemplary sequences of the light chain variable region (VL region; position 1 to 107) of mouse-derived antibodies exhibiting PD-1 agonist activity, as well as examples of their humanized VH region sequences.

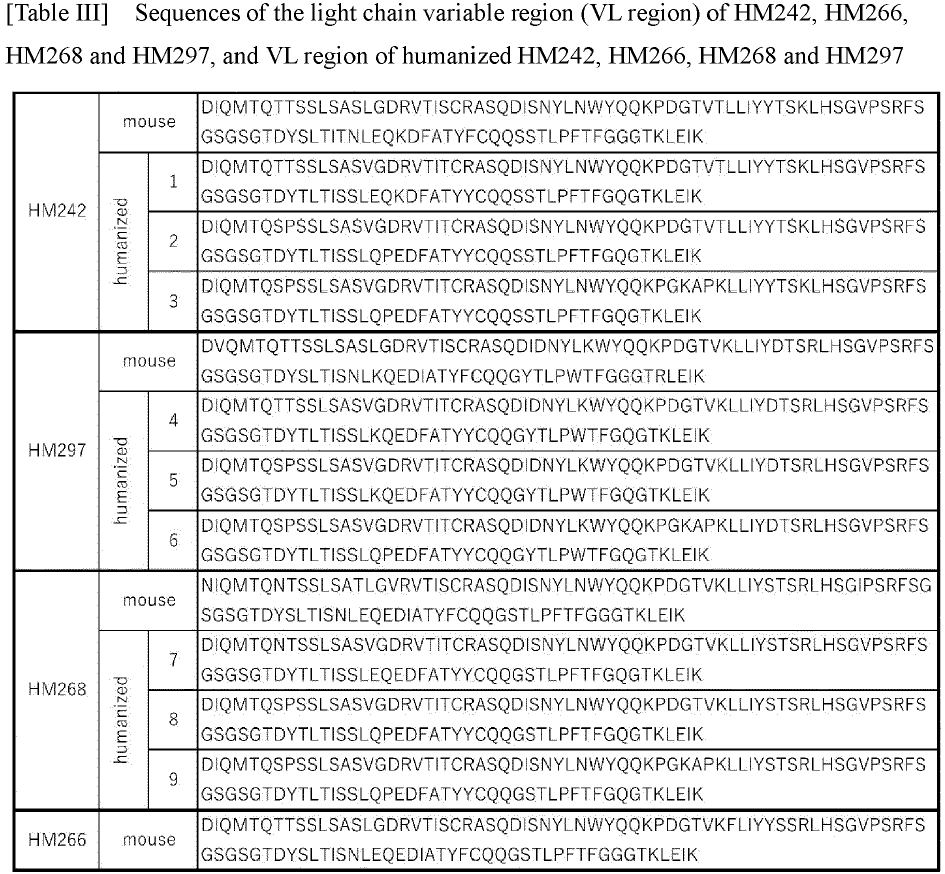

The antibody of the present invention may be one having the heavy chain variable region and/or the light chain variable region as shown in Tables II and/or Table III above. In the antibody of the present invention, the heavy chain variable region and the light chain variable region may individually comprise a sequence having at least 90%, 90-95%, 95-99% or 99% or more identity with the sequences as shown in Tables II and III above.

The antibody of the present invention may be one which has an improved affinity to human Fc receptors. The Fc receptor is preferably Fcγ receptor (FcyR), more preferably FcyRII, and still more preferably FcγRIIB. The binding affinity of the antibody of the present invention to human Fc receptors may be evaluated by a flow cytometer-based binding test for human Fc receptor-expressing cell line, or an human Fc receptor binding affinity test using surface plasmon resonance technology with Biacore 8K (Cytiva). The improvement in the affinity of the antibody of the present invention to human Fc receptors may be evaluated by either of the methods mentioned above. Preferably, the improvement is evaluated by flow cytometer, and more preferably, the improvement is evaluated by both methods. When measured by the human FcyRIIB binding affinity test using surface plasmon resonance technology, the affinity of the antibody of the present invention to human FcyRIIB can be expressed as an equilibrium dissociation constant (K_{D}) ratio to an antibody having the Fc region of human IgG1-K322A (reference antibody). The antibody of the present invention has 1.5-fold or more affinity than the reference antibody, preferably 2-fold or more affinity than the reference antibody, and more preferably 2.5-fold or more affinity than the reference antibody (see Example described later). When measured by the flow cytometer-based binding test for human Fc receptor-expressing cell line, the affinity of the antibody of the present invention to human FcyRIIB can be expressed as a GMFI ratio to an antibody having the Fc region of human IgG1-K322A (reference antibody). Under measuring conditions where a GMFI value without antibody addition is about 40 and a GMFI value with addition of the reference antibody is 300-1500, the antibody of the present invention shows 2-fold or more GMFI, preferably 5-fold or more GMFI, and more preferably 20-fold or more GMFI as compared with the case where the reference antibody having the same Fv region is added.

The antibody of the present invention may also be one which has an improved affinity to human FcγRIIIA. When measured by the Fc receptor binding affinity test using surface plasmon resonance technology, the affinity of the antibody of the present invention to human FcγRIIIA(V158) can be expressed as an equilibrium dissociation constant (K_{D}) ratio to an antibody having the Fc region of human IgG1-K322A (reference antibody). The antibody of the present invention has 1.5-fold or more affinity than the reference antibody, preferably 2-fold or more affinity than the reference antibody, more preferably 2.5-fold or more affinity than the reference antibody, and still more preferably 4-fold or more affinity than the reference antibody (see Example described later). When measured by the flow cytometer-based binding test for human Fc receptor-expressing cell line, the affinity of the antibody of the present invention to human FcγRIIIA(V158) can be expressed as a GMFI ratio to an antibody having the Fc region of human IgG1-K322A (reference antibody). Under measuring conditions where a GMFI value without antibody addition is about 10 and a GMFI value with addition of the reference antibody is 6000-15000, the antibody of the present invention shows 1.5-fold or more GMFI, preferably 2-fold or more GMFI, more preferably 4-fold or more GMFI, and still more preferably 5.0-fold or more GMFI as compared with the case where the reference antibody having the same Fv region is added.

The antibody of the present invention may be one having the Fc region of a human antibody (for example, IgG1, IgG4, etc.) and, preferably, the Fc region (position 216 to 447) of the human antibody is modified so that the affinity to human Fc receptors (at least one of human FcyRIIA, human FcγRIIB, human FcyRIIIA) is improved. By modifying the Fc region of an antibody, its affinity to human Fc receptors can be improved. Alternatively, the affinity of an antibody to human Fc receptors can also be improved by defucosylation treatment.

One example of the amino acid sequence of the Fc region of human IgG1 is shown in SEQ ID NO: 47, and one example of the amino acid sequence of the Fc region of human IgG4 is shown in SEQ ID NO: 64. As a means to improve the affinity of the antibody of the present invention to human Fc receptors, a mutation(s) may be introduced, for example, to at least one position, preferably a combination of positions, selected from the group of amino acid positions consisting of 233, 234, 236, 237, 238, 239, 267, 268, 271, 296, 323, 326, 328, 330 and 332 (according to EU numbering; hereinafter, the same shall apply) in the amino acid sequence of the Fc region of human IgG1 as shown in SEQ ID NO: 47. Further, such mutation(s) may be combined with defucosylation. When mutations are introduced into a combination of positions, for example, it may be selected from among 236/268, 239/268, 239/268/328/332, 239/267/268, 239/267/268/328, 239/267/268/332, 239/268/328, 239/268/332, 239/267/268/328/332, 233/237/238/268/271/330 and 267/328, preferably from among 239/268, 239/268/328/332, 239/267/268, 239/267/268/328332, 239/268/332, 233/237/238/268/271/330 and 267/328, and more preferably from among 239/268/328/332, 233/237/238/268/271/330 and 267/328. G at position 236 may be mutated to, for example, D, E, N, Q, F, H, I, K, L, M, P, R, S, T, V, W, Y or A, preferably to D, E, N or Q, and more preferably to D. H at position 258 may be mutated to, for example, D, E, N, Q, A or G, preferably to D, E, N or Q, and more preferably to D. S at position 239 may be mutated to, for example, D, E, N, Q, F, T, H, Y, G or L, preferably to D, E, N or Q, and more preferably to D. L at position 328 may be mutated to, for example, Y, E, F, H, I, Q, W, D, T, S, M, A, V, N or H, preferably to Y, F, D, E, N or Q, and more preferably to Y. I at position 332 may be mutated to, for example, E, D, F, L, R, S, T, D, N, Q, H, Y, A or M, preferably to E, T or M, and more preferably to E. E at position 233 may be mutated to, for example, D. G at position 237 may be mutated to, for example, W, F, A, D, E, L, M or Y, preferably to D or E, and more preferably to D. P at position 238 may be mutated to, for example, D. P at position 271 may be mutated to, for example, G. A at position 330 may be mutated to, for example, K, R or M, preferably to R. S at position 267 may be mutated to, for example, E, V, Q, A or L, preferably to E or G, and more preferably to G. K at position 326 may be mutated to, for example, L, Q, N, M, D, S, T or A. L at position 234 may be mutated to, for example, D, E, N, Q, T, H, I, V, F, W or Y. V at position 323 may be mutated to, for example, I, L or M. Y at position 296 mat be mutated to, for example, D.

When mutation(s) is/are to be introduced into the Fc region of human IgG1, they may be combined with other mutations depending on the purpose, as exemplified by K322A (a mutation known to suppress complement-dependent cytotoxicity (CDC) activity by decreasing the binding of complement C1q) or E293A (a mutation known to suppress ADCC activity by decreasing the binding to FcyRIIIA).

The combination of mutations to be introduced into the Fc region of human IgG1 may be any one of the following combinations: G236D/H268D, S239D/H268D, S239D/H268D/L328Y/I332E, G236D/H268D/K322A, S239D/H268D/K322A, S239D/S267G/H268D/K322A, G236D/H268D/E293A/K322A, S239D/H268D/K322A/L328Y/I332E, S239D/H268D/E293A/K322A, S239D/S267G/H268D/K322A/L328Y, S239D/S267G/H268D/K322A/I332E, S239D/H268D/K322A/L328Y, S239D/H268D/K322A/I332E, S239D/S267G/H268D/K322A/L328Y/I332E, E233D/G237D/P238D/H268D/P271G/A330R, or S267E/L328F. Preferably, the combination may be any one of the following combinations: S239D/H268D/K322A, S239D/S267G/H268D/K322A, G236D/H268D/E293A/K322A, S239D/H268D/K322A/L328Y/I332E, S239D/H268D/E293A/K322A, S239D/S267G/H268D/K322A/I332E, S239D/H268D/K322A/I332E, E233D/G237D/P238D/H268D/P271G/A330R, or S267E/L328F.

When mutations are to be combined with defucosylation in the Fc region of human IgG, the combination of mutations in the Fc region should be such that defucosylation improves affinity for human Fc receptor, especially human FcγRIIIA, 1.5-fold or more affinity than the fucosylated IgG having same mutations, preferably 2-fold or more affinity than the fucosylated IgG having same mutations, more preferably 4-fold or more affinity than the fucosylated IgG having same mutations. Preferred mutation and defucosylation combinations include, but are not limited to, S239D/S267G/H268D/K322A and defucosylation combinations.

As an exemplary means to improve the affinity to human Fc receptors, a mutation may be introduced at any one or more of the following positions: 236, 239, 268, 328, or 332 in the amino acid sequence of the Fc region of human IgG4 as shown in SEQ ID NO: 64, for example. Preferably, a mutation may be introduced at a combination of those positions. Further, such mutations may be combined with defucosylation.

When a mutation is to be introduced into the Fc region of human IgG4, it may be combined with other mutations such as S228P that is known to be effective for improving antibody stability.

When mutations are to be combined, the combination may be selected from among S228P/G236D/Q268D, S228P/S239D/Q268D and S228P/S239D/Q268D/L328Y/I332E, more preferably from between S228P/S239D/Q268D and S228P/S239D/Q268D/L328Y/I332E.

Table IV shows exemplary sequences of Fc regions of human IgG1 (WT and K322A) and IgG4 (WT and S228P), as well as examples of the sequences of Fc regions in their Fc region modified forms with mutations introduced.

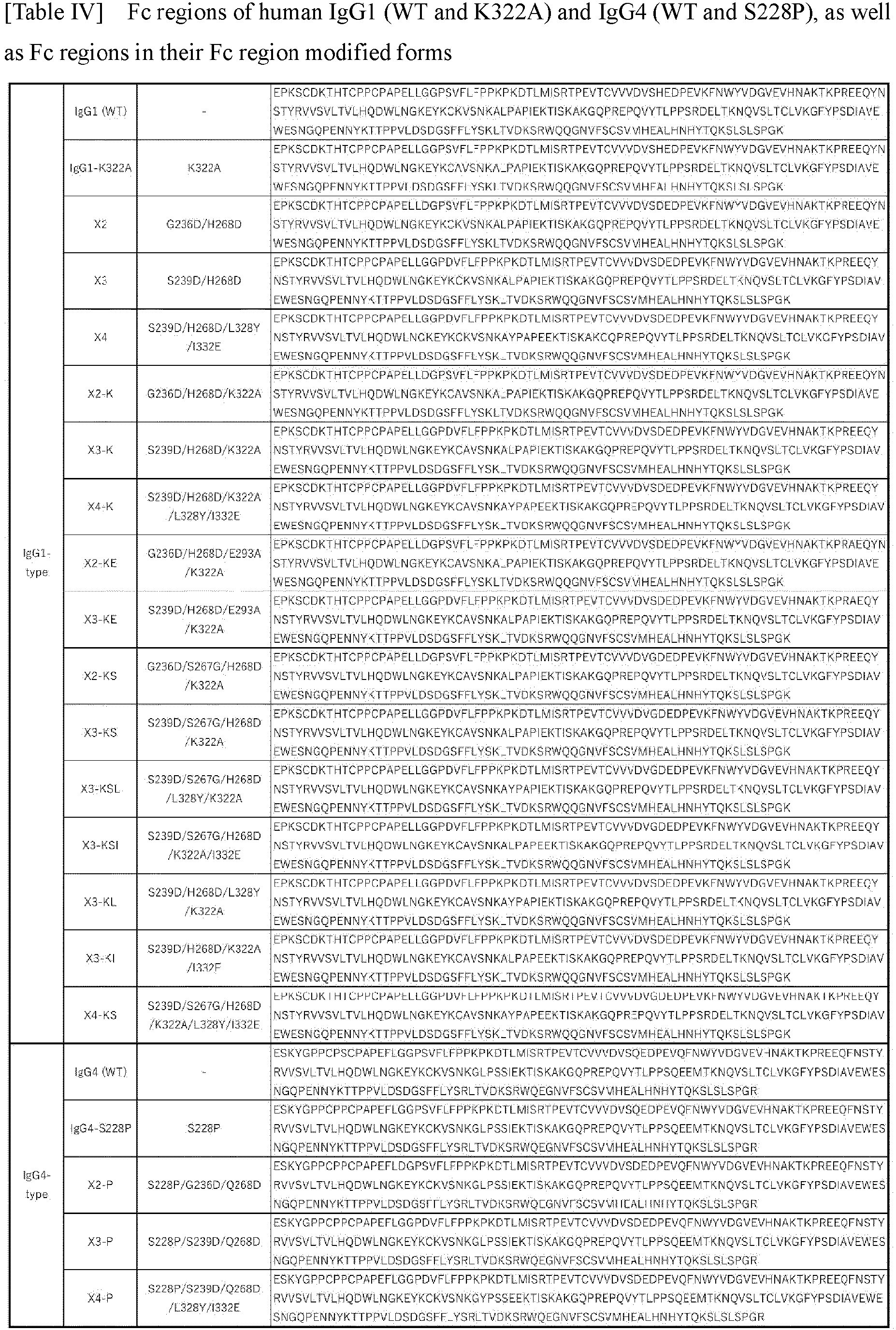

The antibody of the present invention may advantageously be one which has a high ratio of monomers having a four-chain structure consisting of two light chains and two heavy chains. Monomer ratio may be measured by SEC-HPLC (see Example described later). Monomer ratio is preferably at least 80%, more preferably at least 90%.

The antibody of the present invention may further undergo functional modifications, such as introduction of amino acid mutations other than those described above, subclass substitution, sugar chain modification, etc.

The antibody of the present invention preferably has an ADCC activity higher than that of IgG1 or IgG1-K322A in which the same Fv region is employed. Compared to antibodies with low ADCC activity, antibodies with high ADCC activity exhibit a high suppressive action on antigen-stimulated T cells. ADCC activity may be examined as follows: for example, in a system using Fc receptor expressing gene-modified Jurkat T cells instead of effector cells, signals from the Fc receptors activate the luciferase gene integrated downstream of NFAT response sequence to thereby generate luciferase, which is then quantified with a luminometer (see Example described later).

The antibody of the present invention preferably comprises a variable region in which each of the Fab regions of a heavy chain and a light chain of an antibody (showing PD-1 agonist activity) derived from a non-human animal (e.g., mouse) has been humanized, and the Fc region of an Fc region-modified form of human IgG1 or IgG4. Exemplary amino acid sequences of the heavy chain and the light chain of preferable antibodies of the present invention and examples of nucleic acid sequences encoding those amino acid sequences are shown in Table V below. The antibody of the present invention may be one which has the heavy chain sequence and/or the light chain amino acid sequence as shown in Table V below. In the antibody of the present invention, the heavy chain and the light chain thereof may comprise an amino acid sequence having at least 90%, 90-95%, 95-99% or more than 99% identity with the amino acid sequence as shown in Table V below. Further, nucleotides encoding the antibody of the present invention may also be nucleotides encoding these heavy chain and light chain amino acid sequences, and such nucleotides may be altered from the nucleotide sequences as shown in Table V depending on the amino acid sequence of interest. The designing of nucleotide sequences in accordance with the amino acid sequences of interest is known to one of ordinary skill in the art, and it is desirable that nucleotide sequences (codons) encoding individual amino acids be optimized in accordance with the amino acid sequences of interest.

Examples of the antibody of the present invention include HM242-C3-X3-K, HM242-C3-X3-KE, HM242-C3-X3-KI, HM242-C3-X3-KSI, HM242-C3-X4-K, HM242-D3-X3-K, HM242-D3-X3-KE, HM242-D3-X3-KI, HM242-D3-X3-KS, HM242-D3-X3-KSI, HM242-D3-X4-K, HM268-K8-X3-K, HM268-K8-X3-KE, HM268-K8-X3-KI, HM268-K8-X3-KS, HM268-K8-X3-KSI and HM268-K8-X4-K. HM242-C3-X3-KI, HM242-C3-X3-KSI, HM242-D3-X3-KI, HM242-D3-X3-KS, HM242-D3-X3-KSI, HM268-K8-X3-KI, HM268-K8-X3-KS and HM268-K8-X3-KSI are preferred, with HM242-D3-X3-KS, HM268-K8-X3-KI, HM268-K8-X3-KS and HM268-K8-X3-KSI being the most preferred.

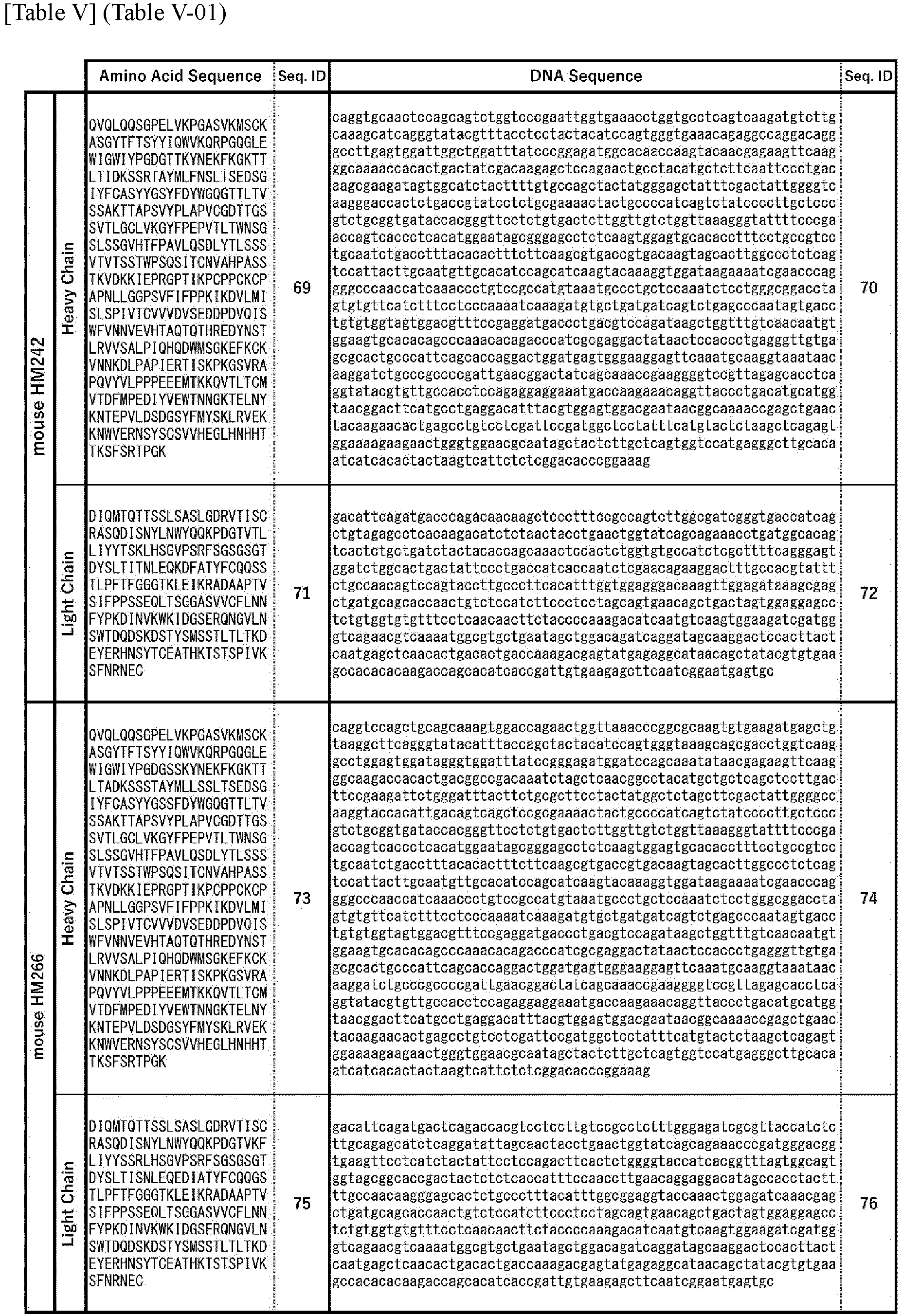

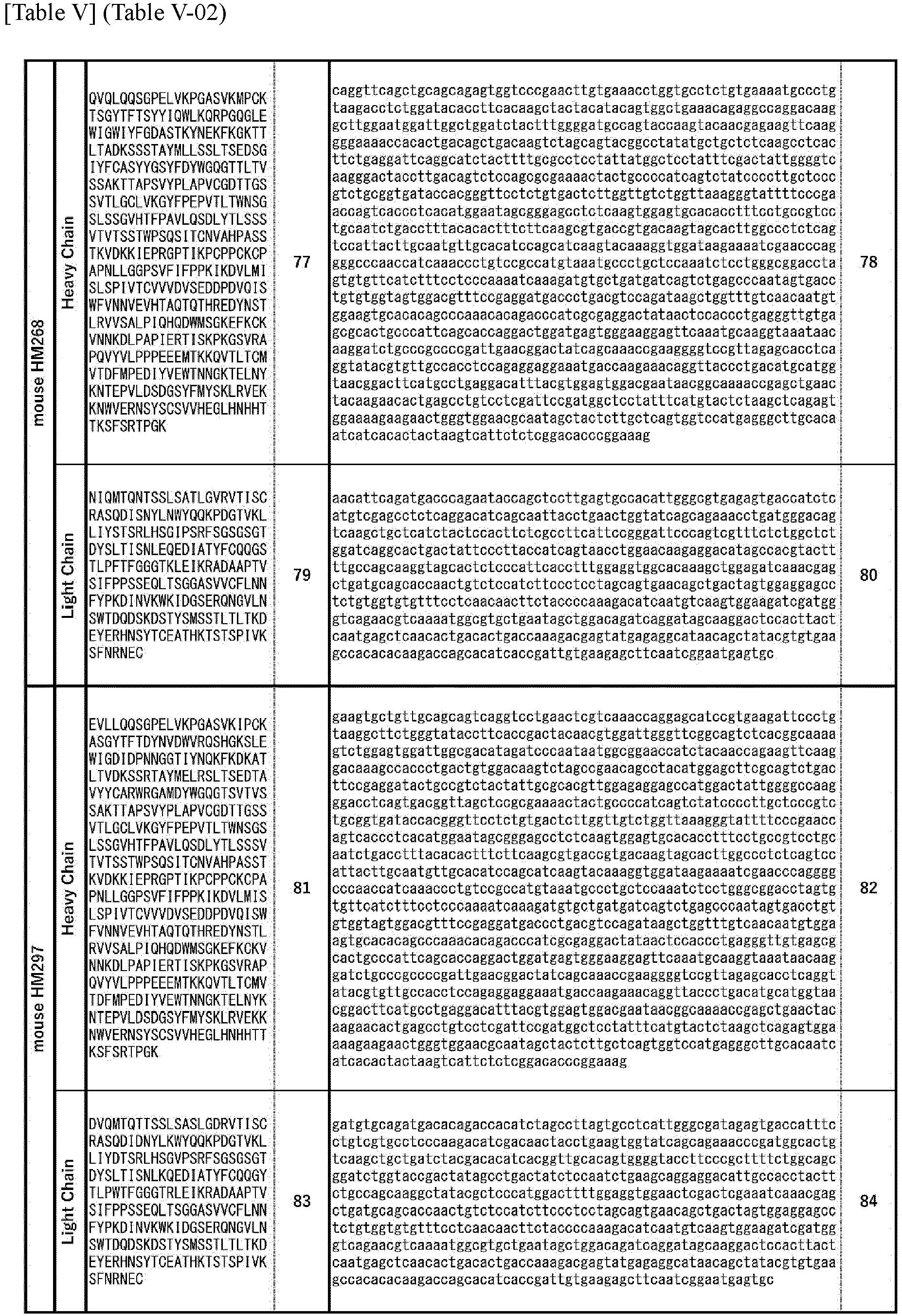

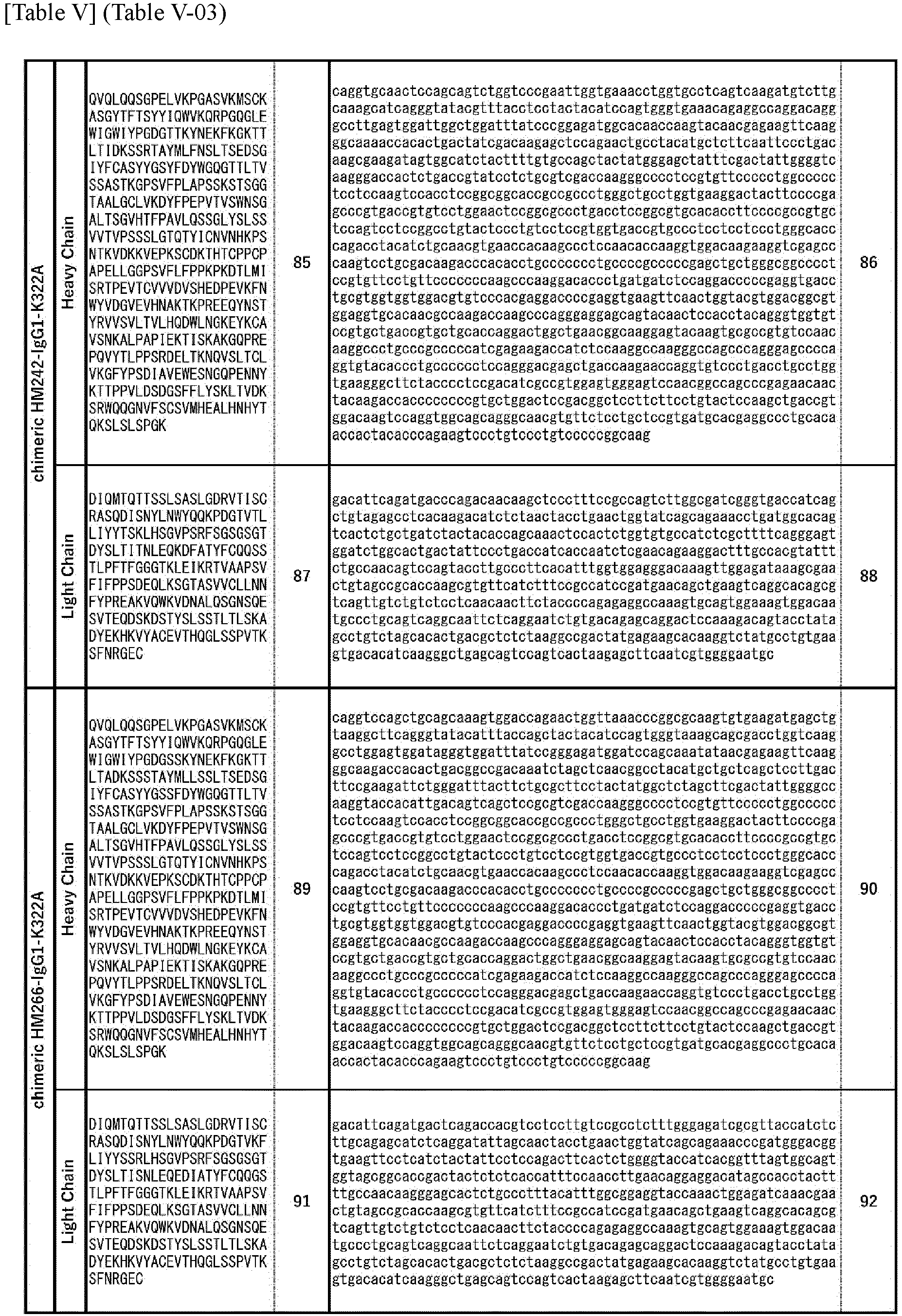

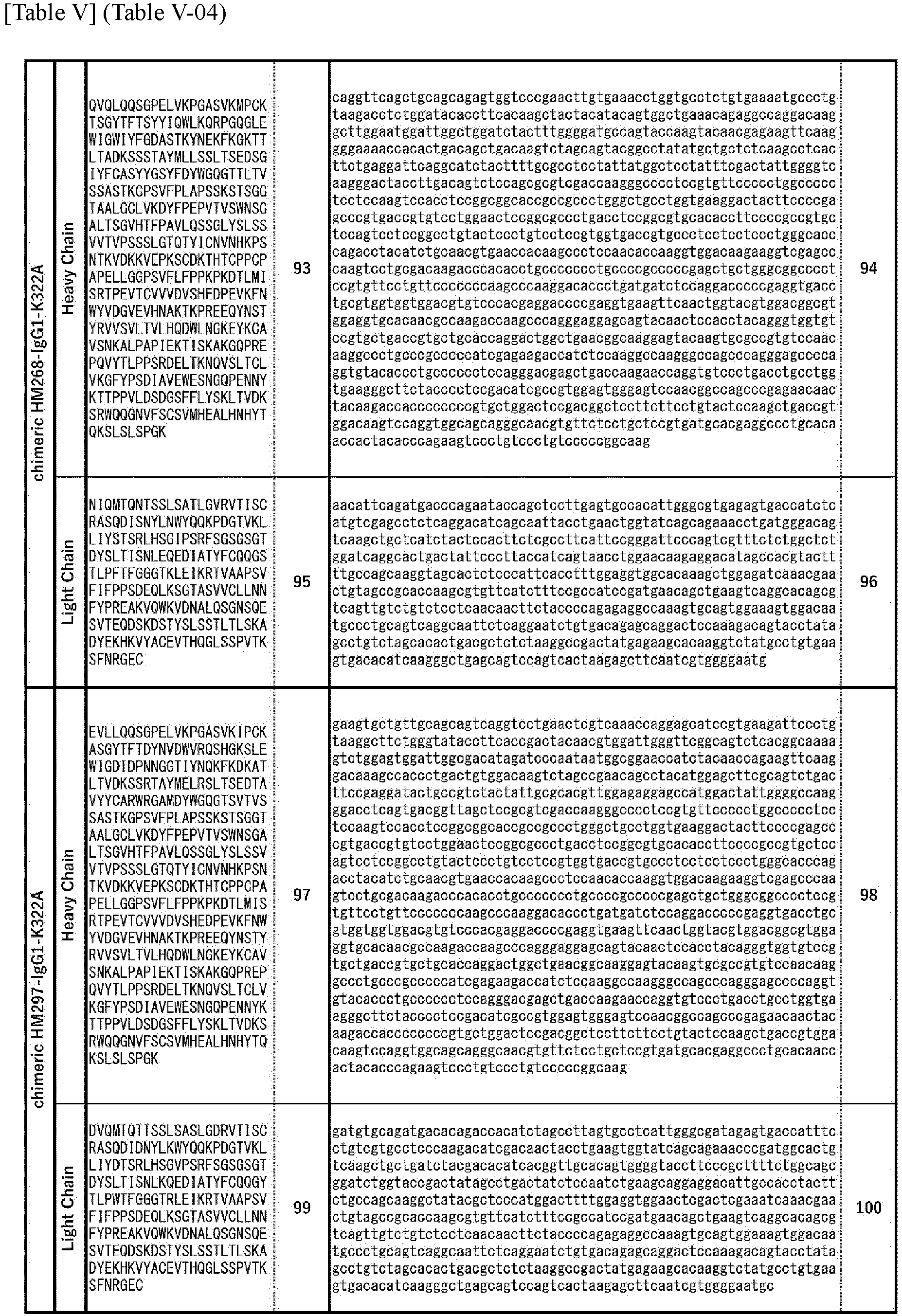

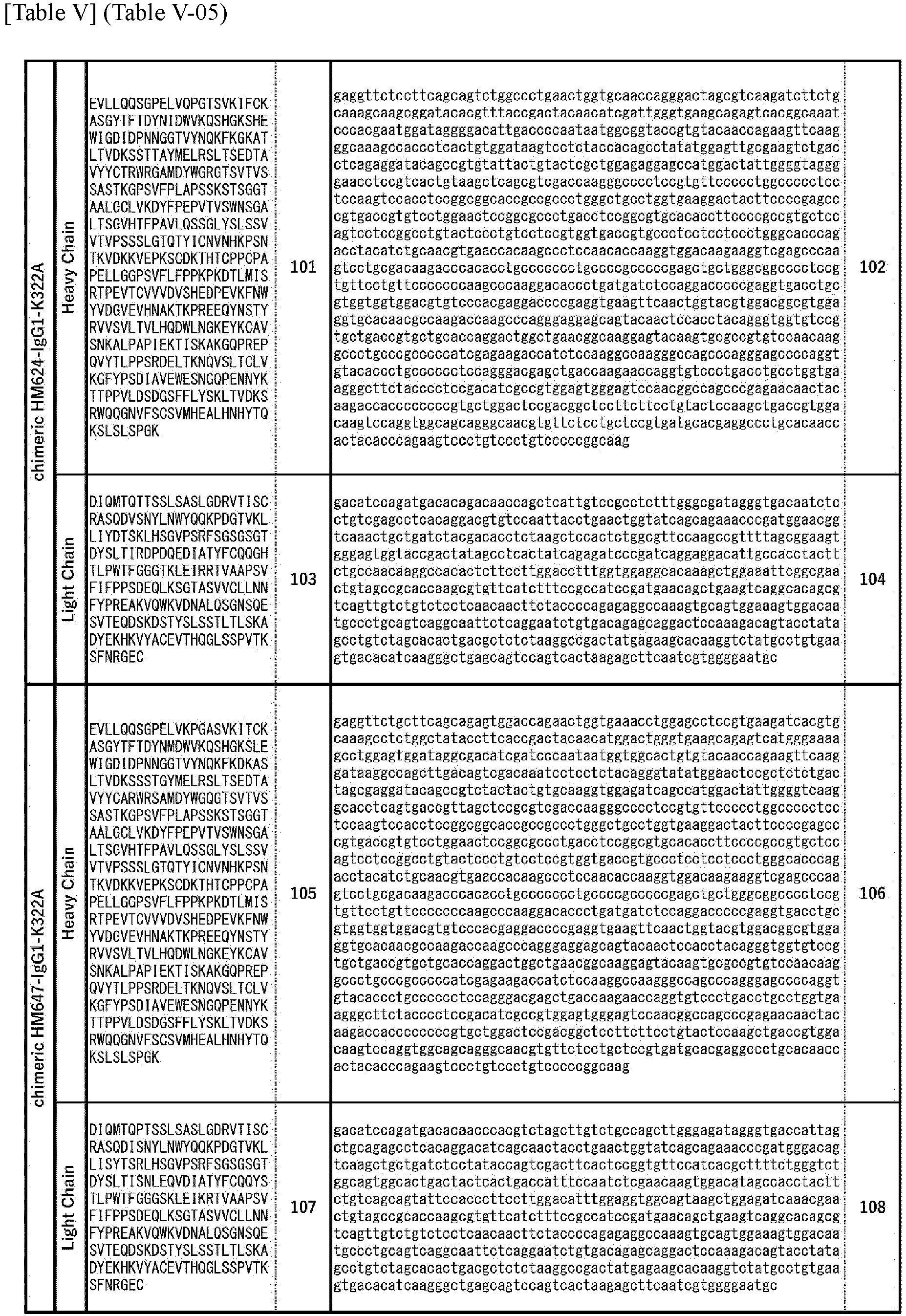

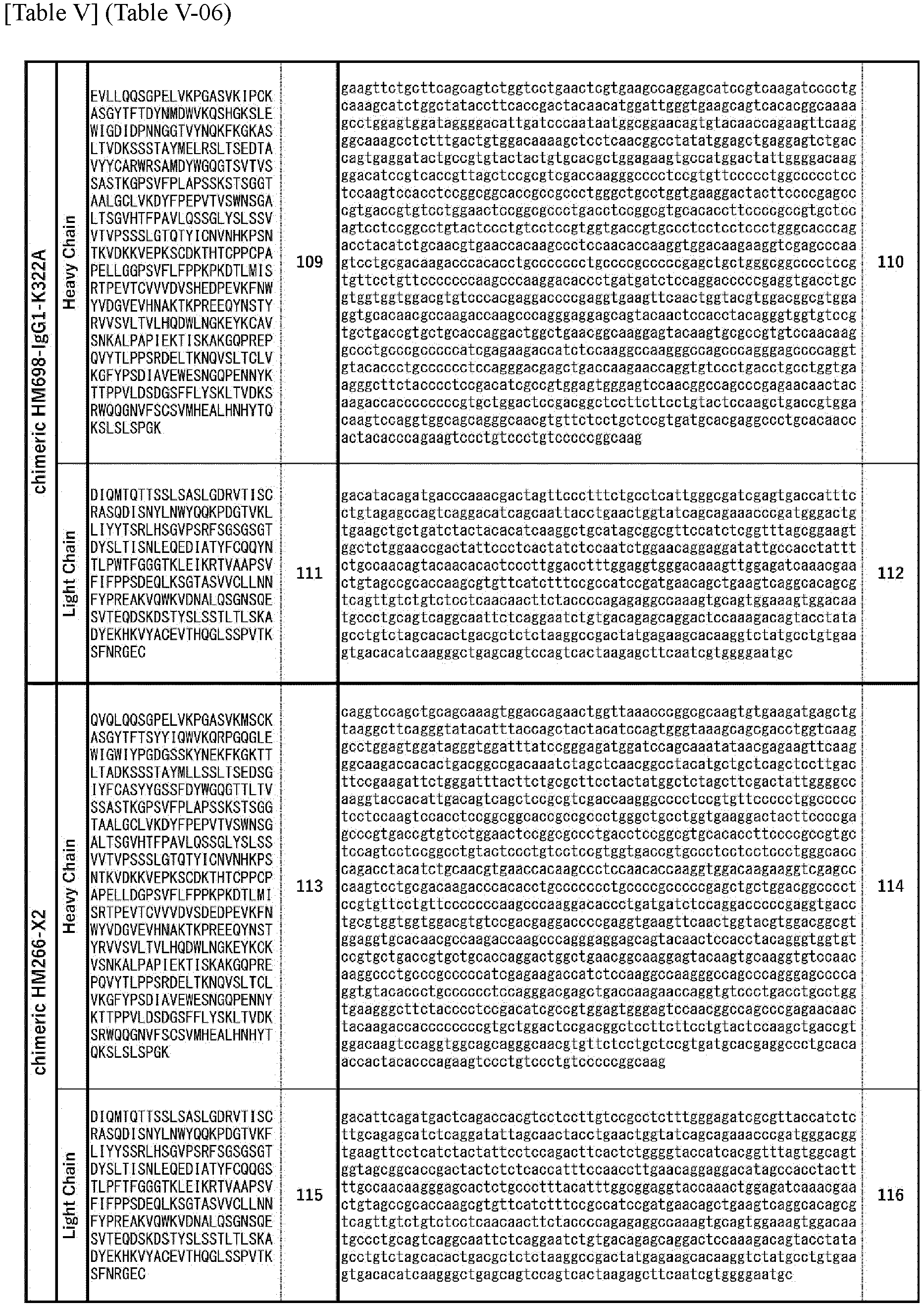

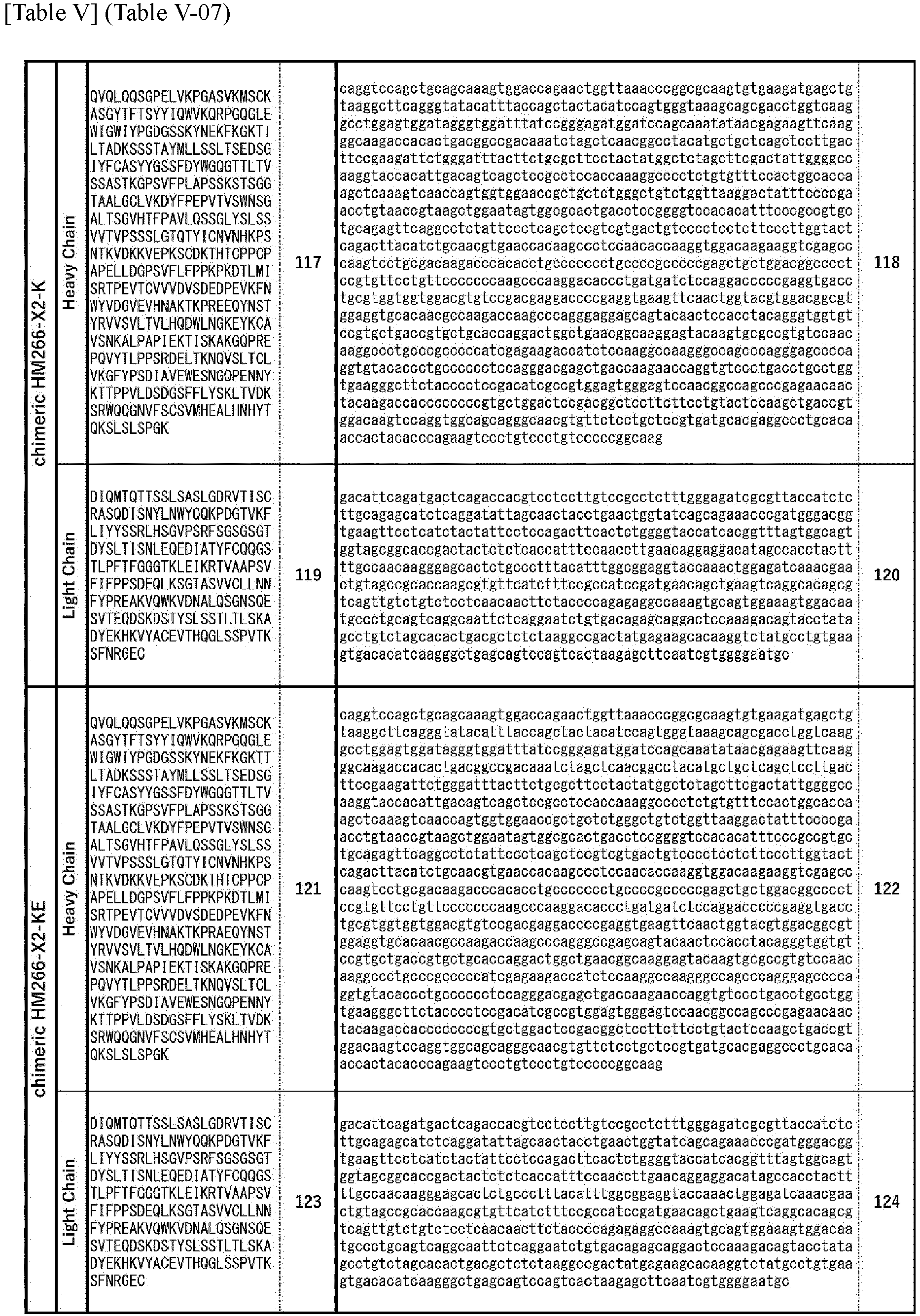

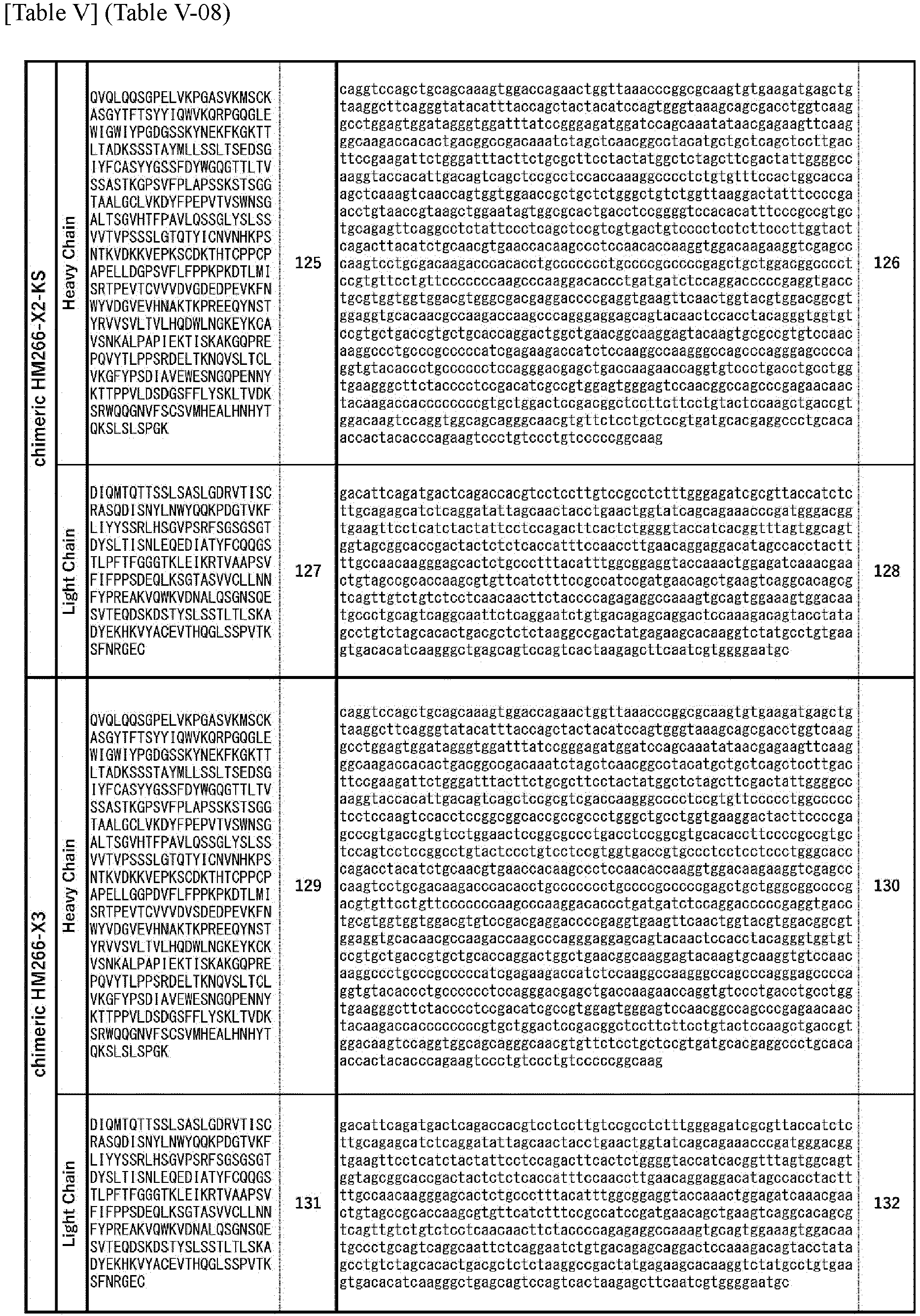

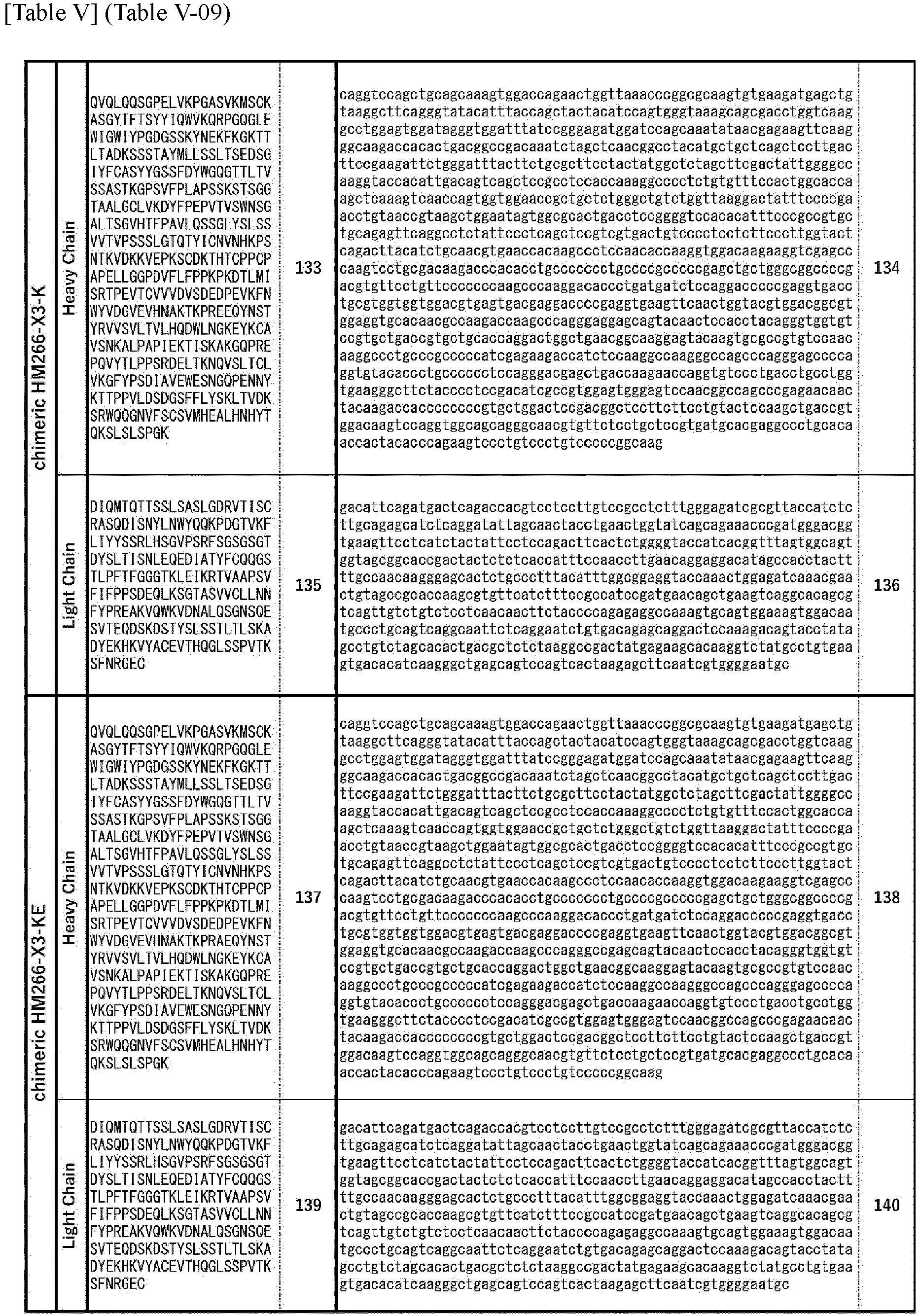

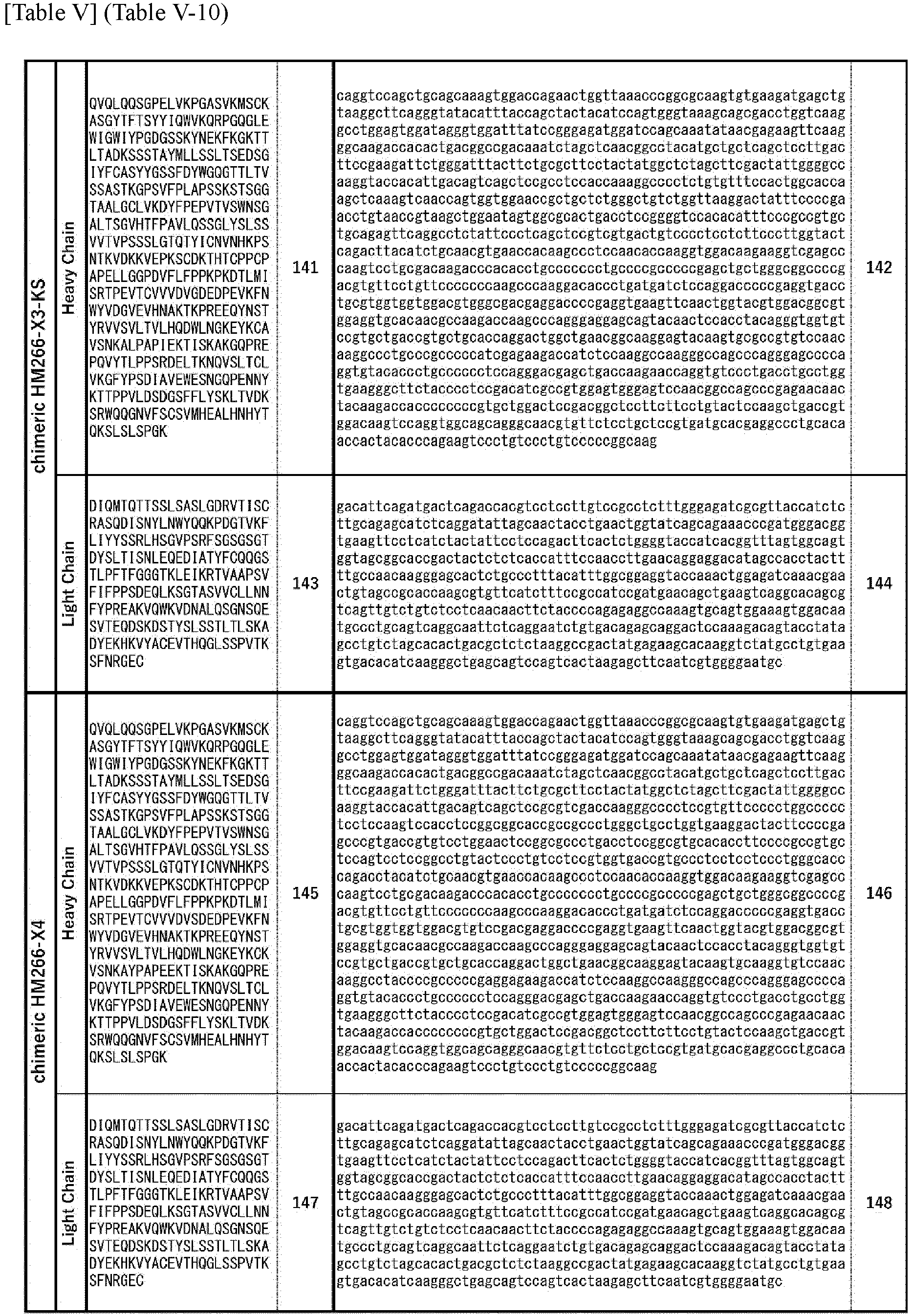

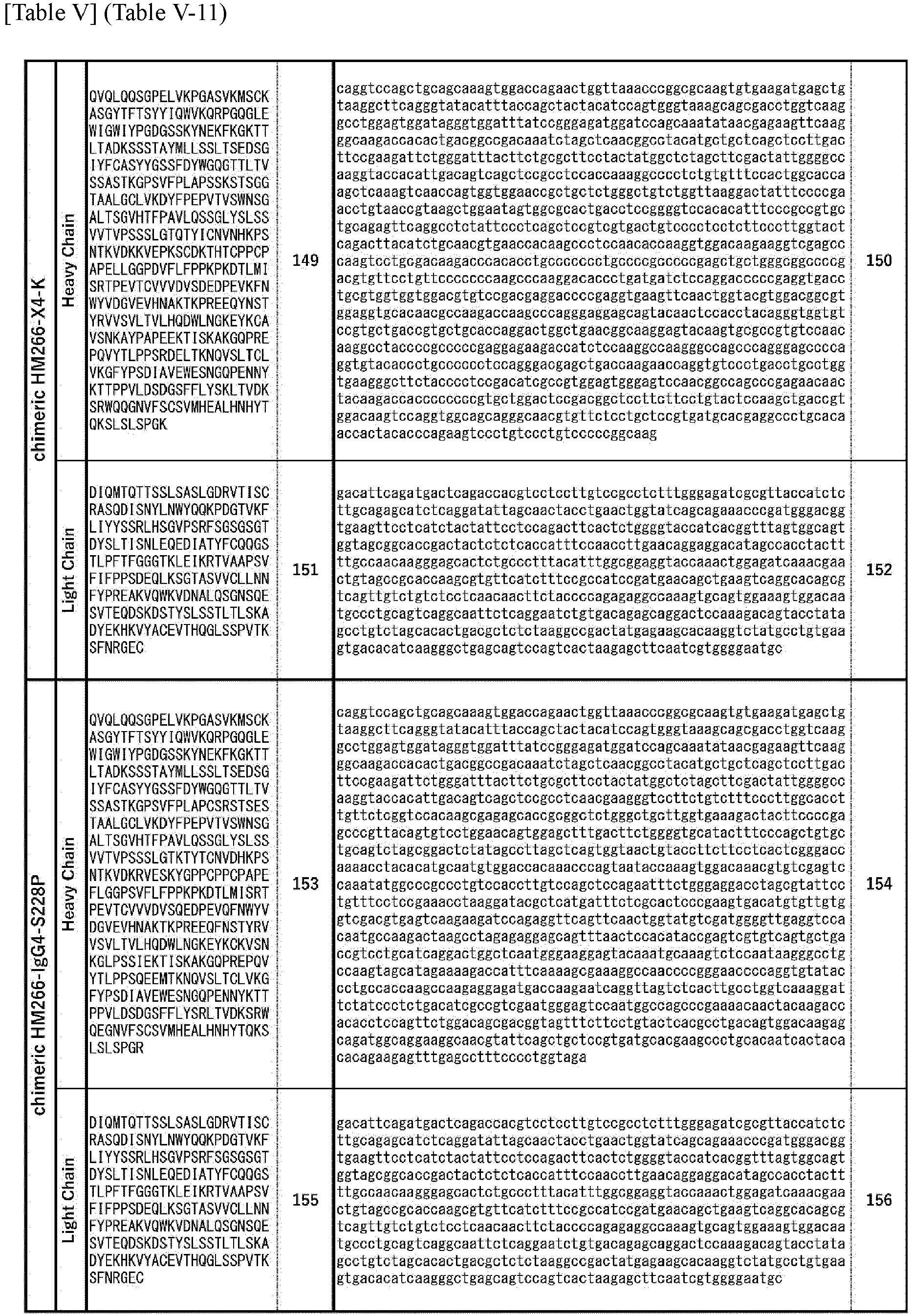

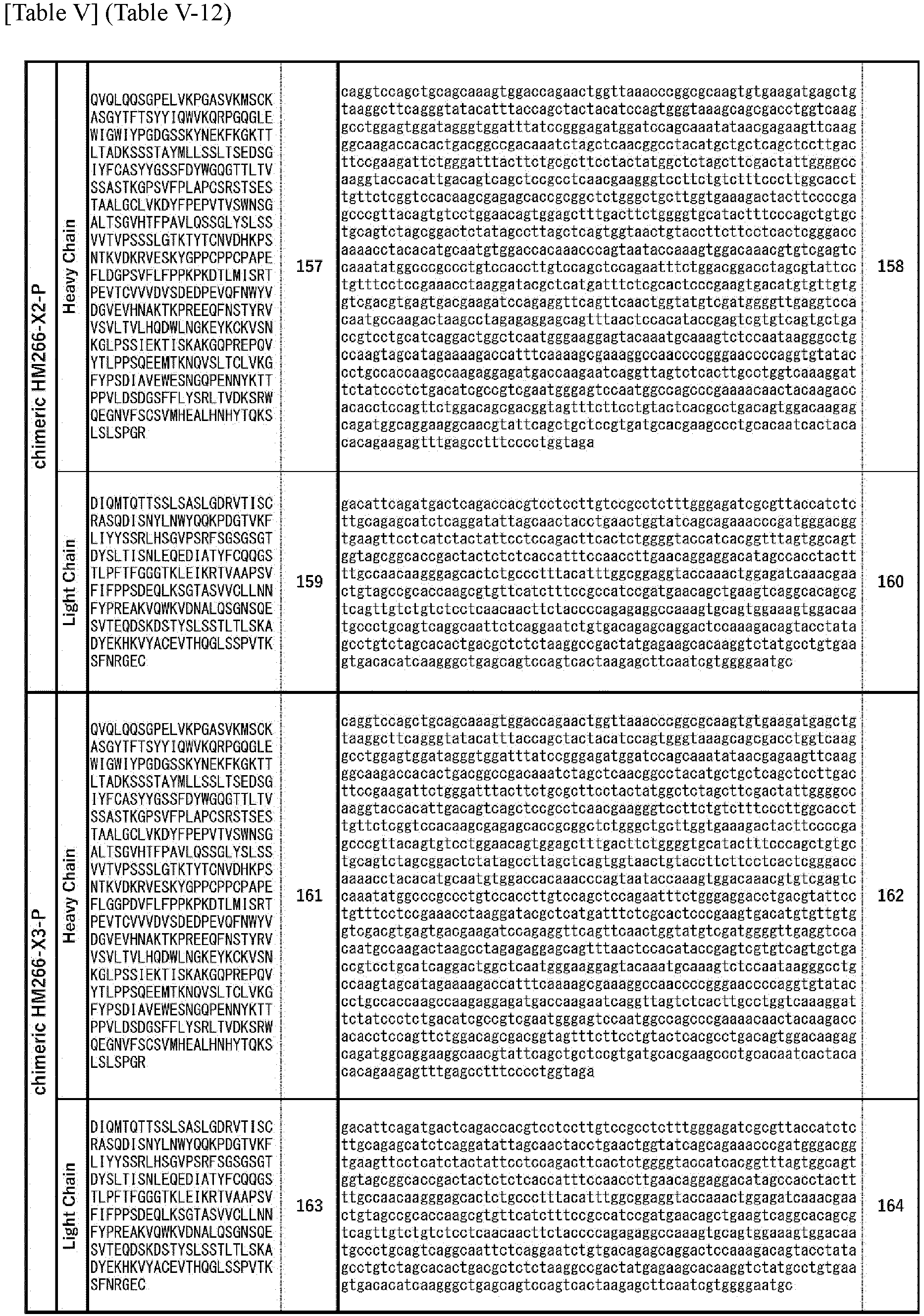

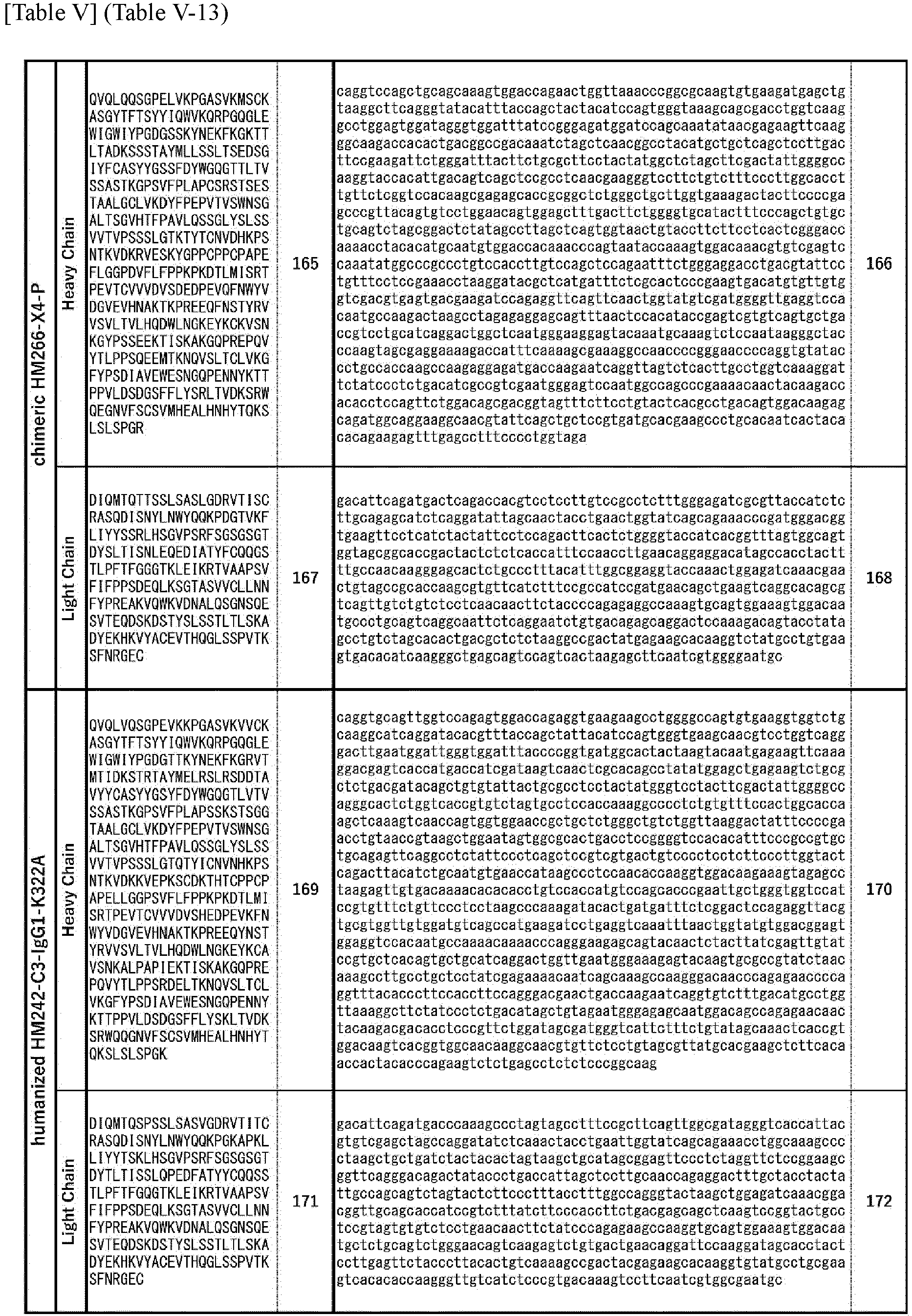

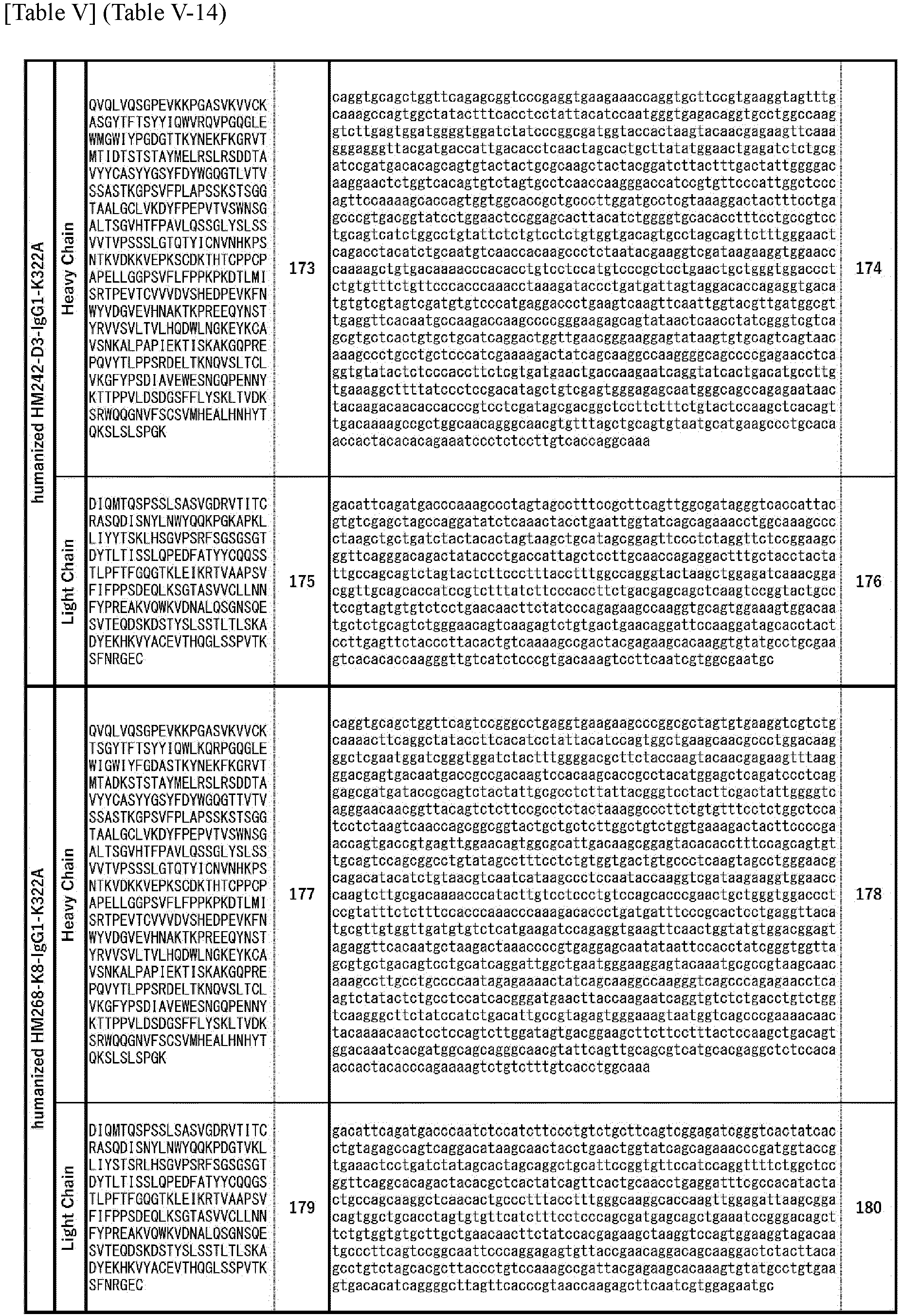

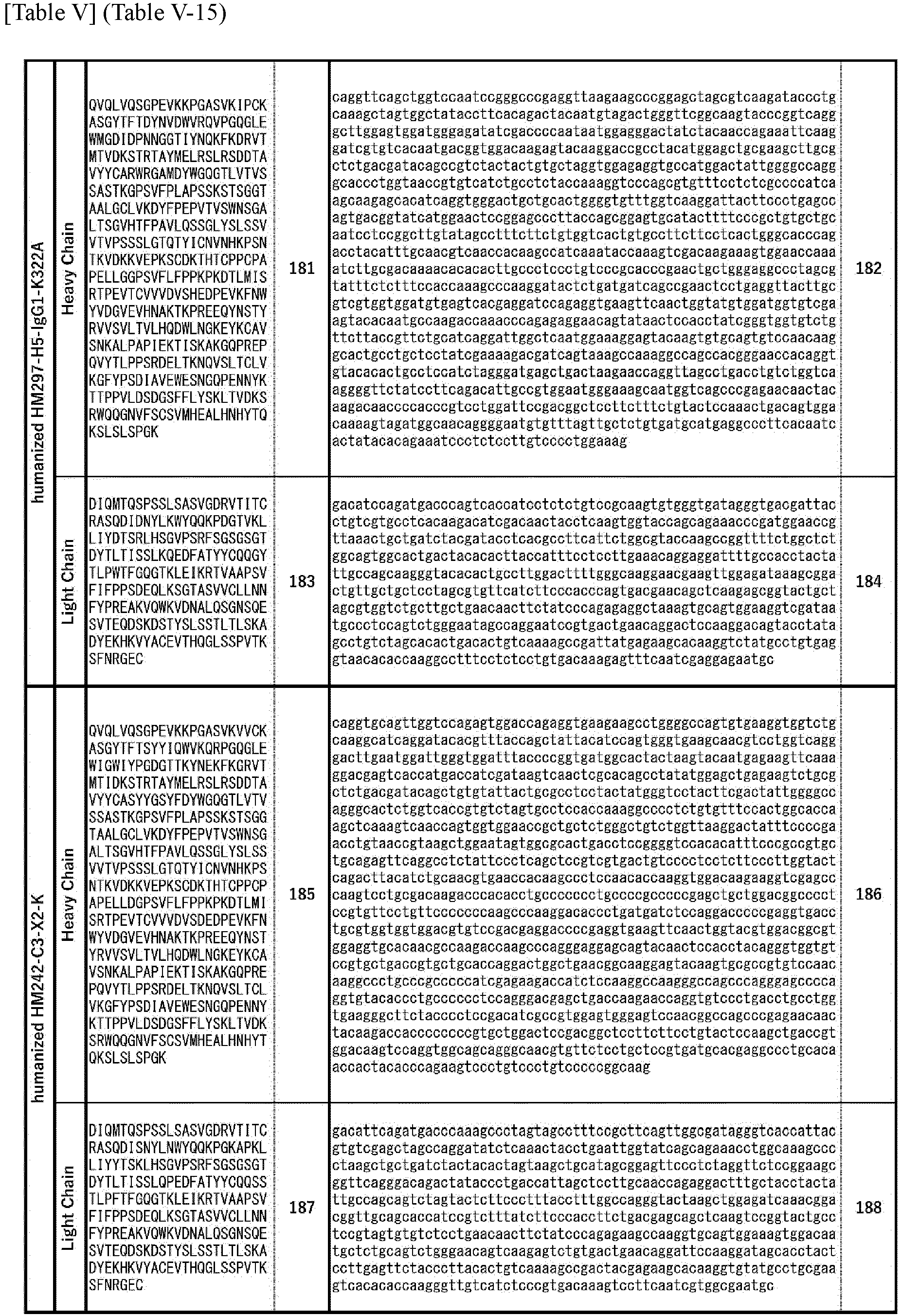

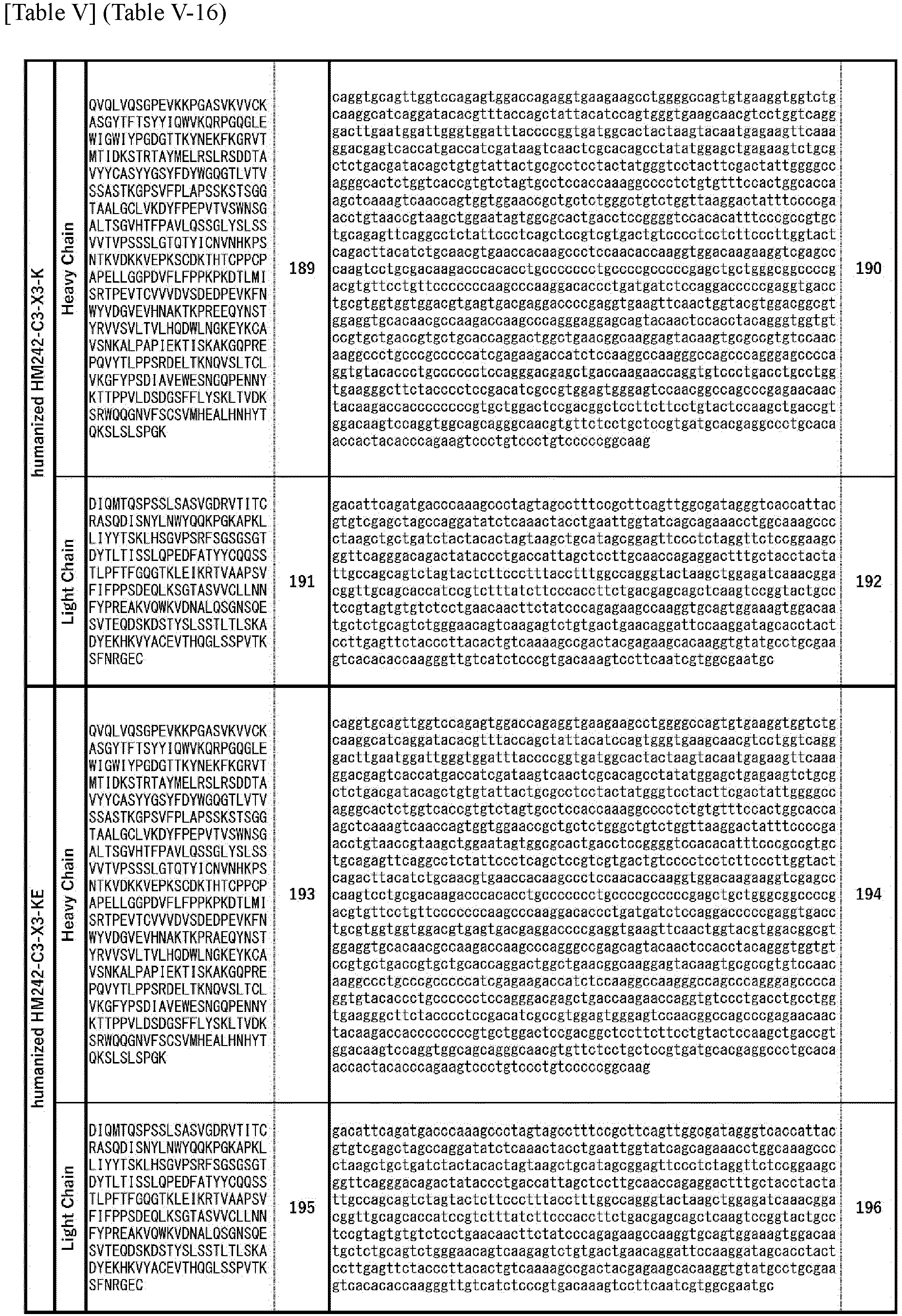

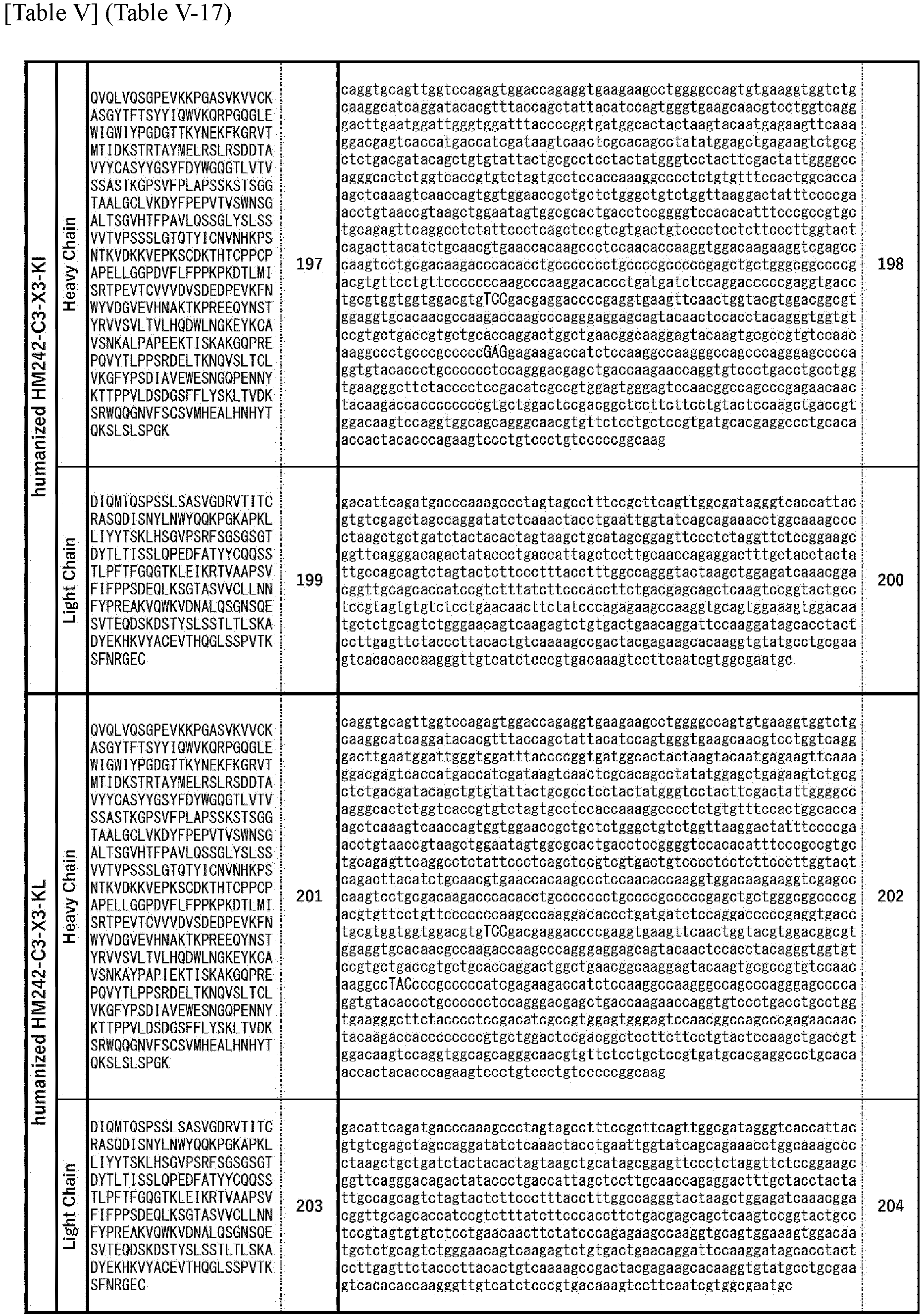

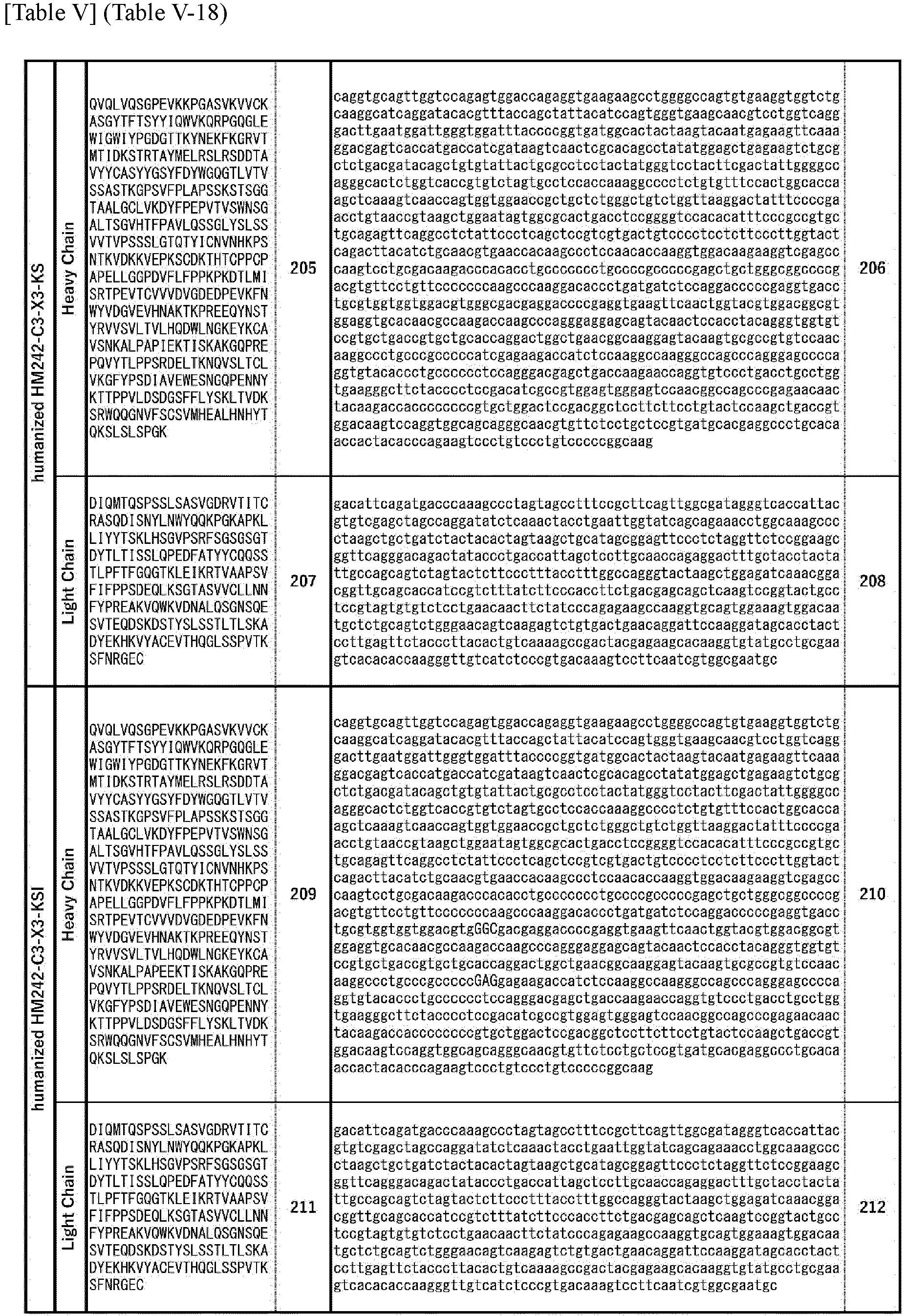

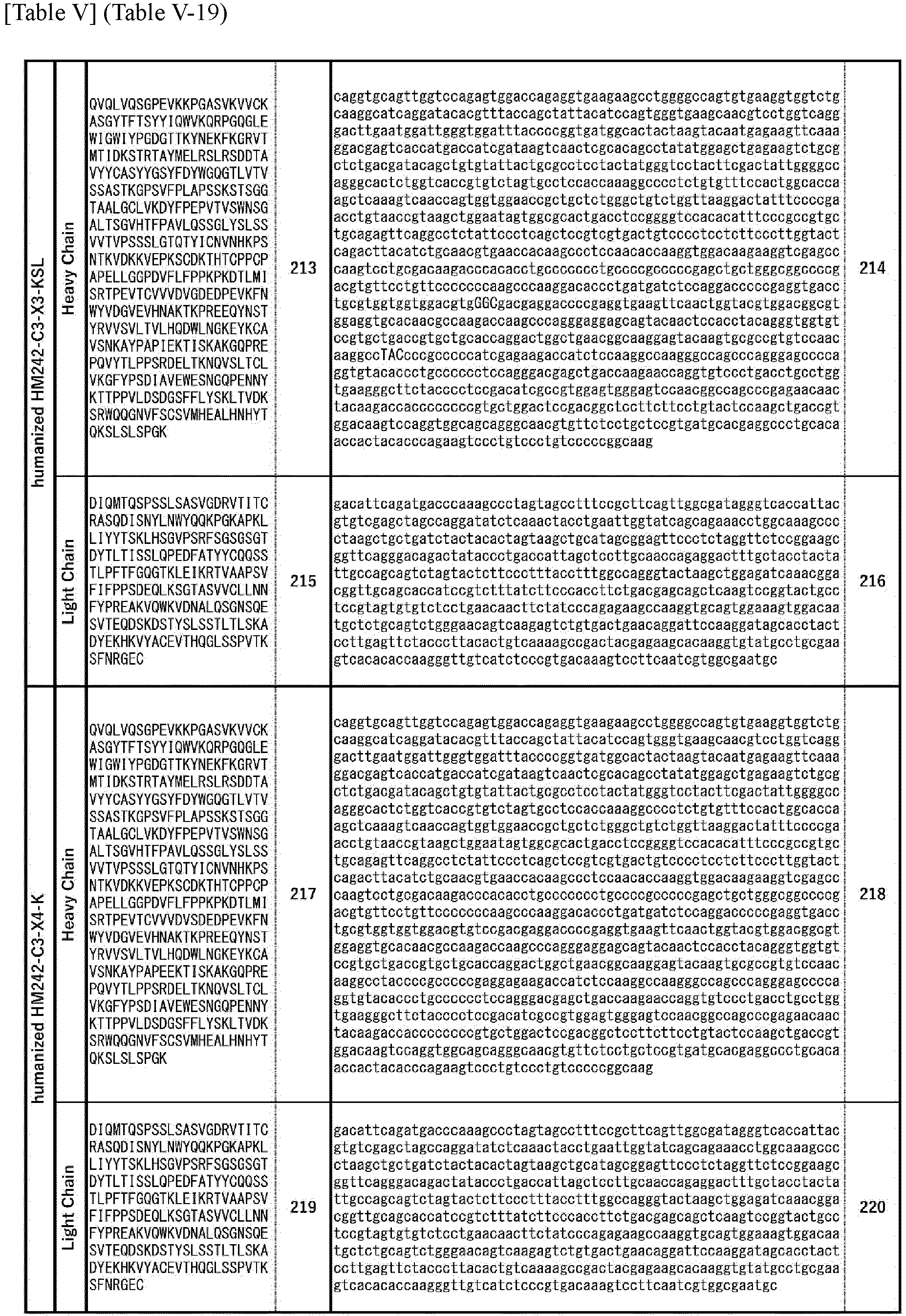

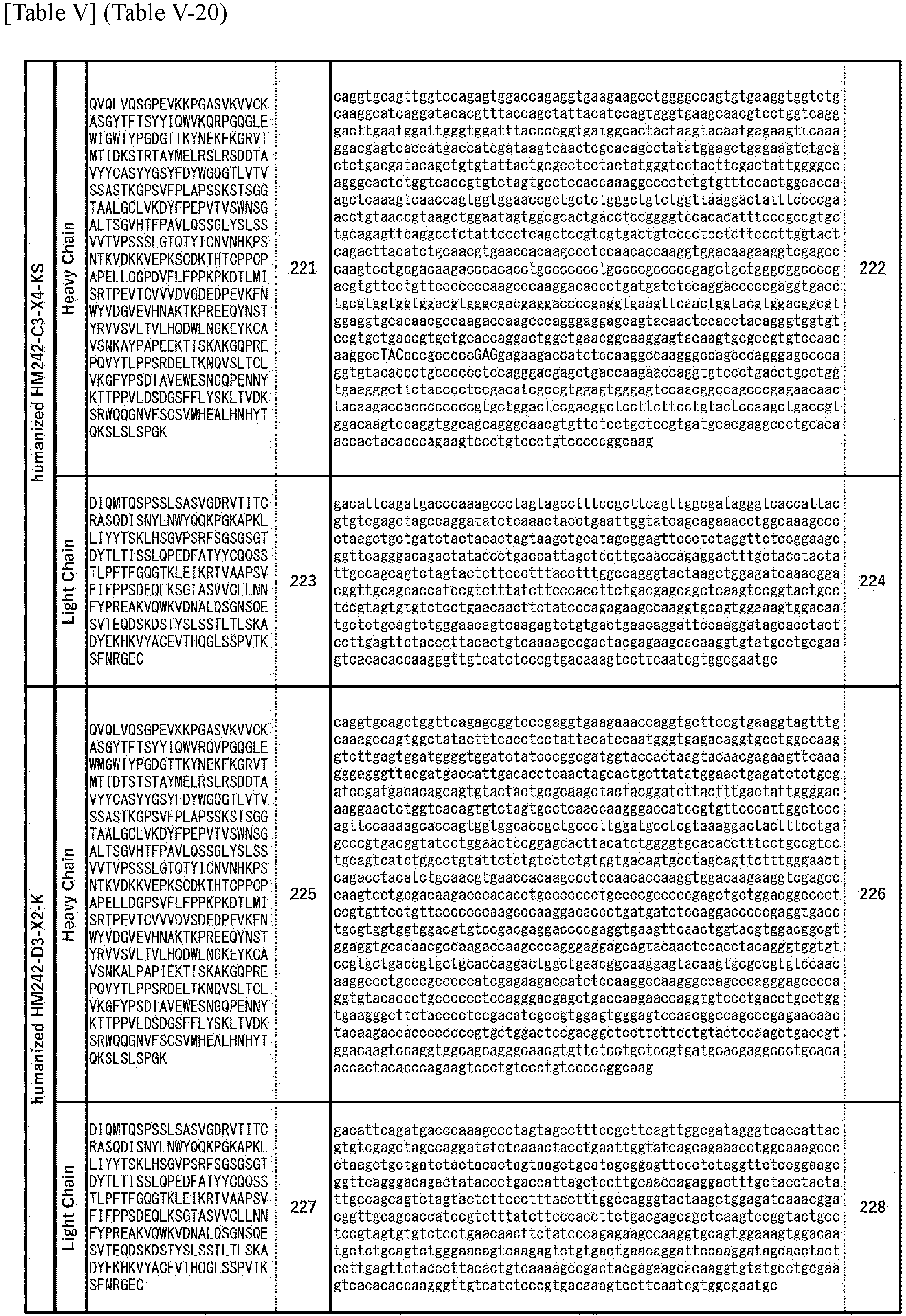

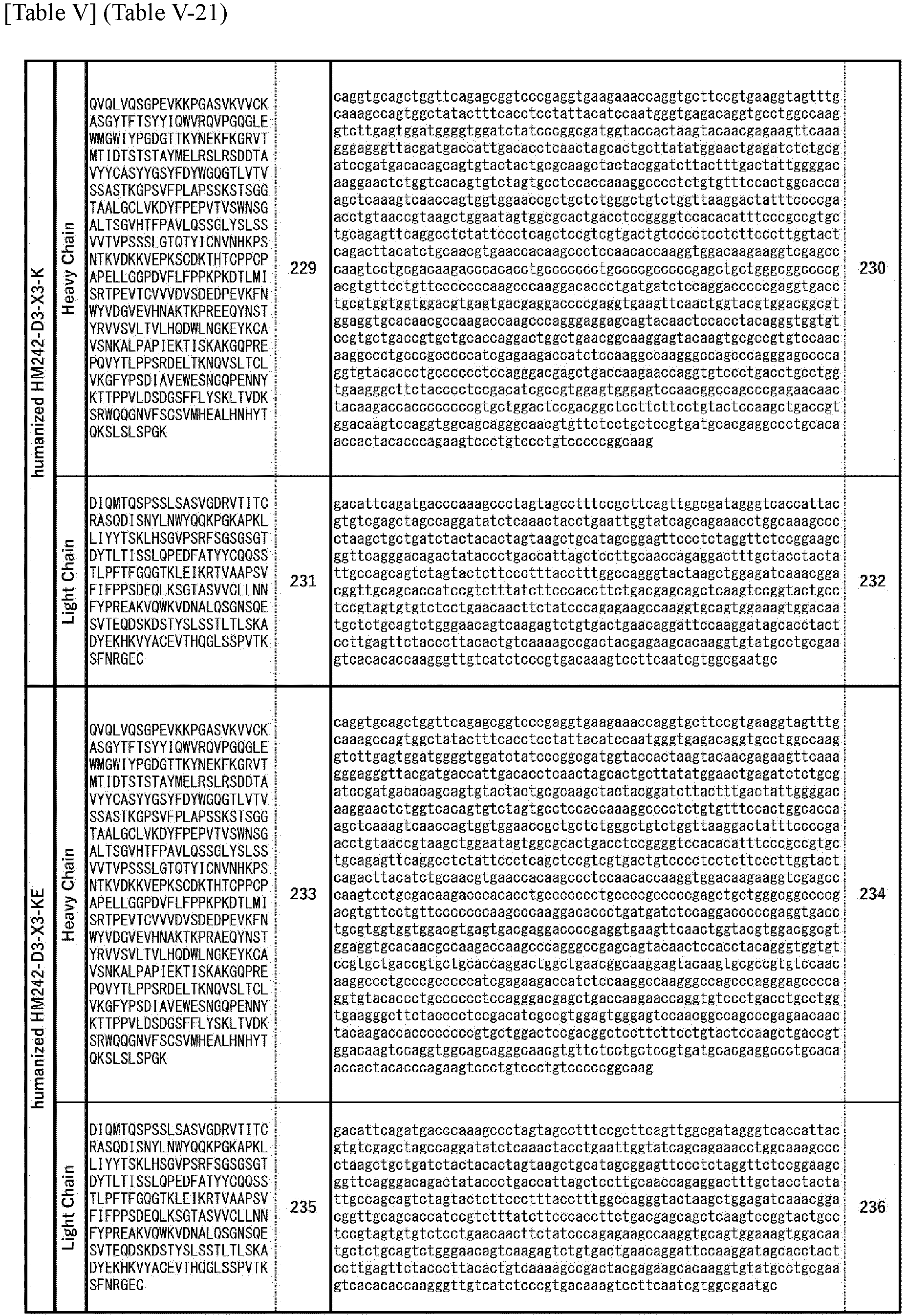

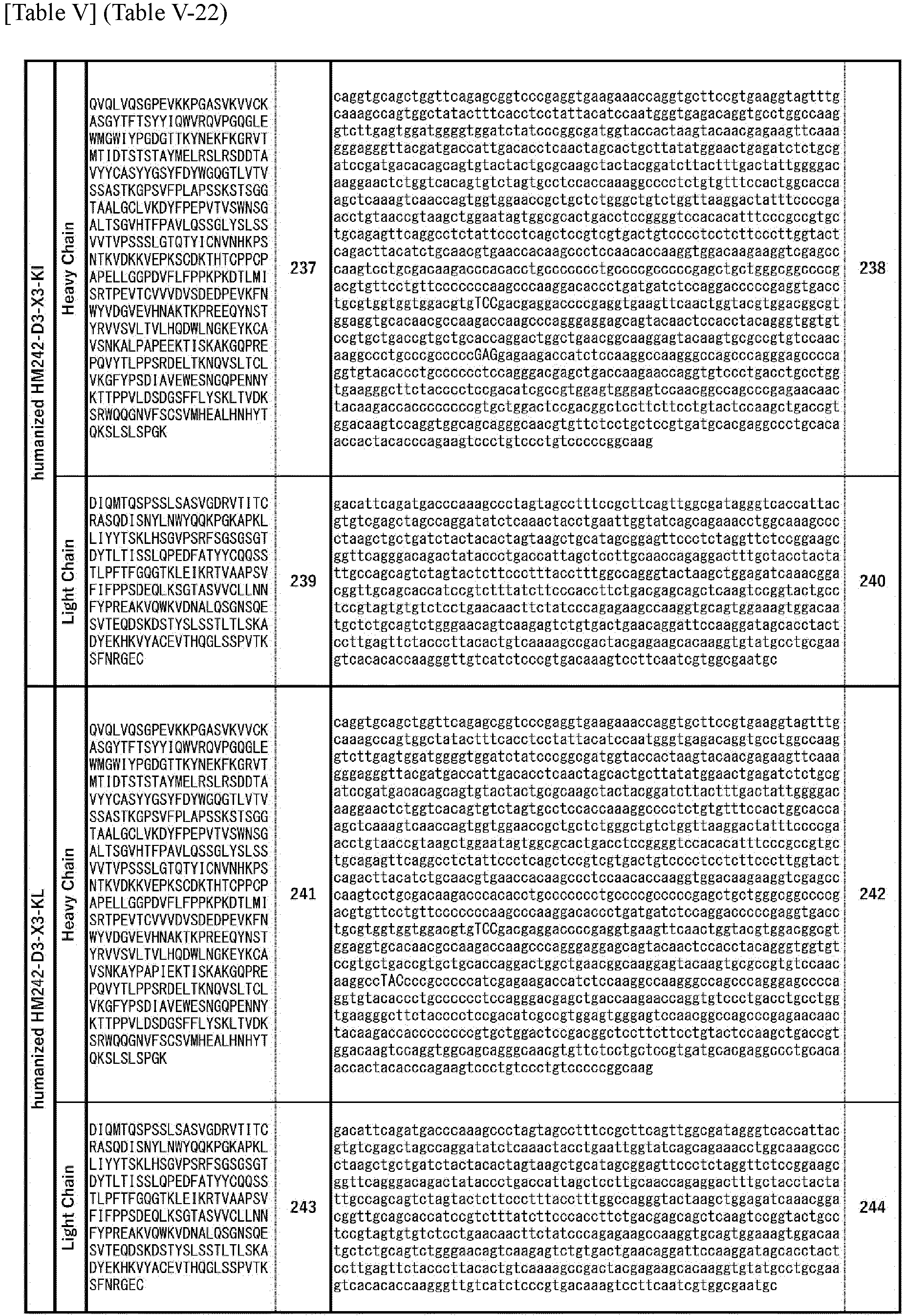

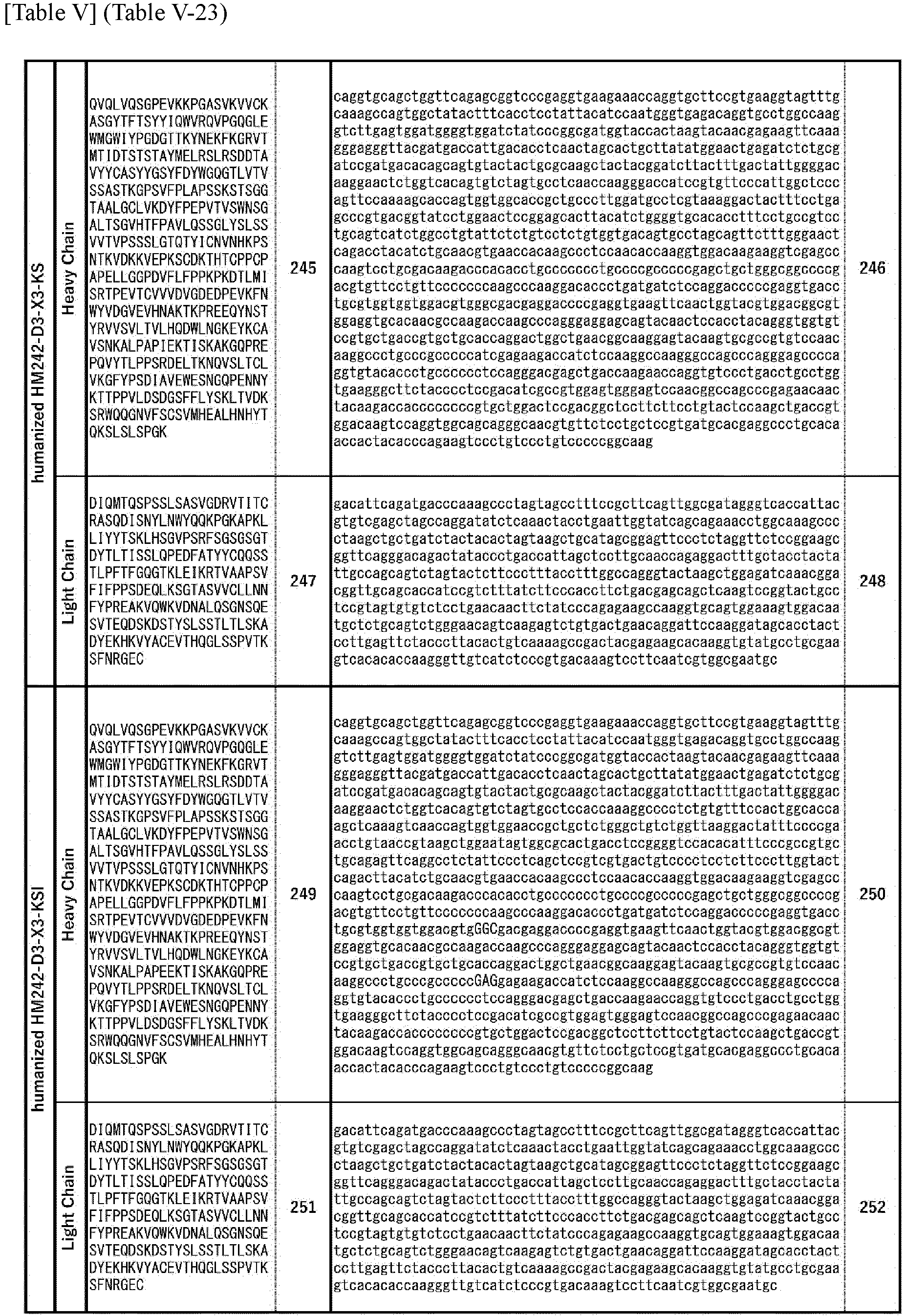

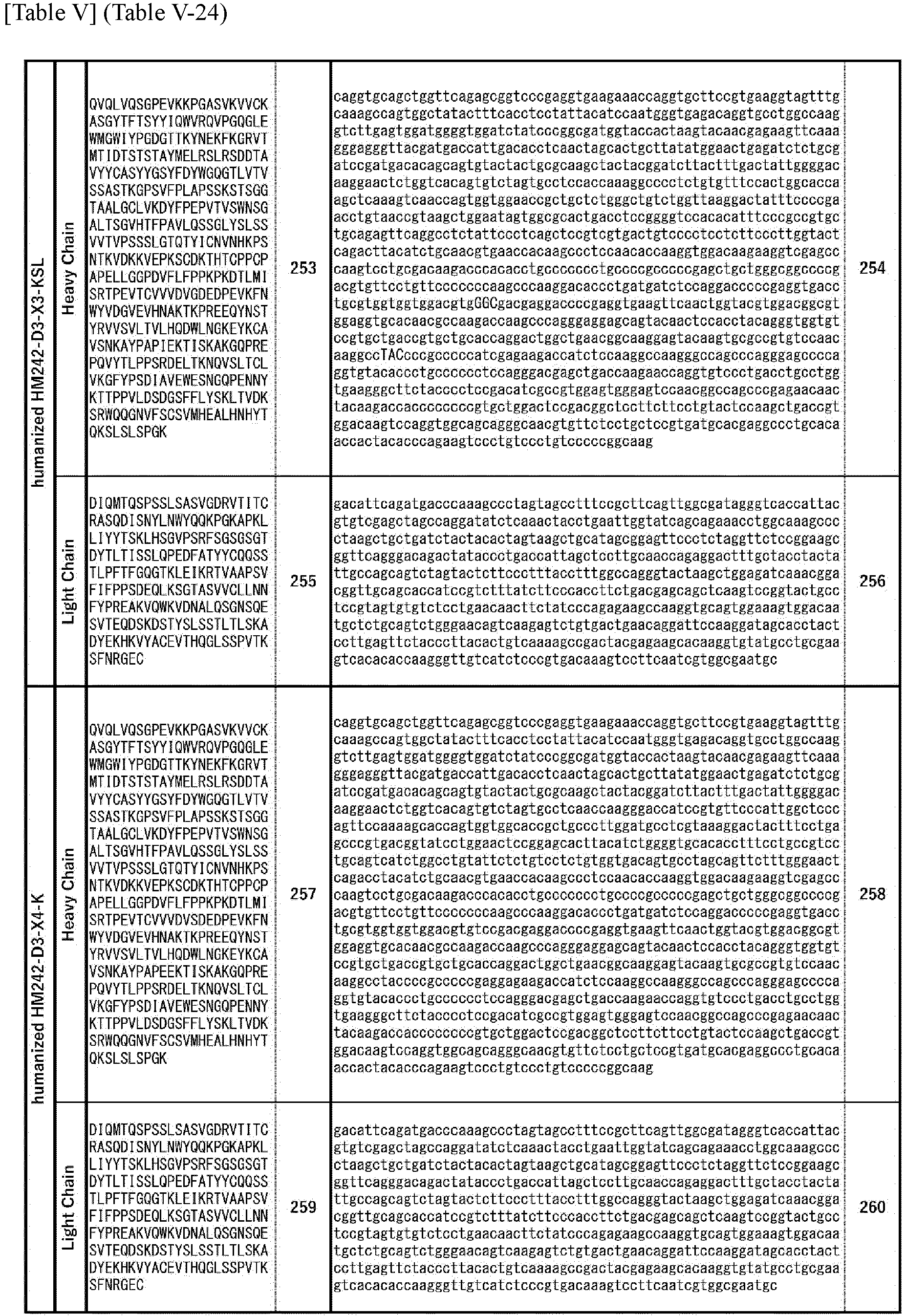

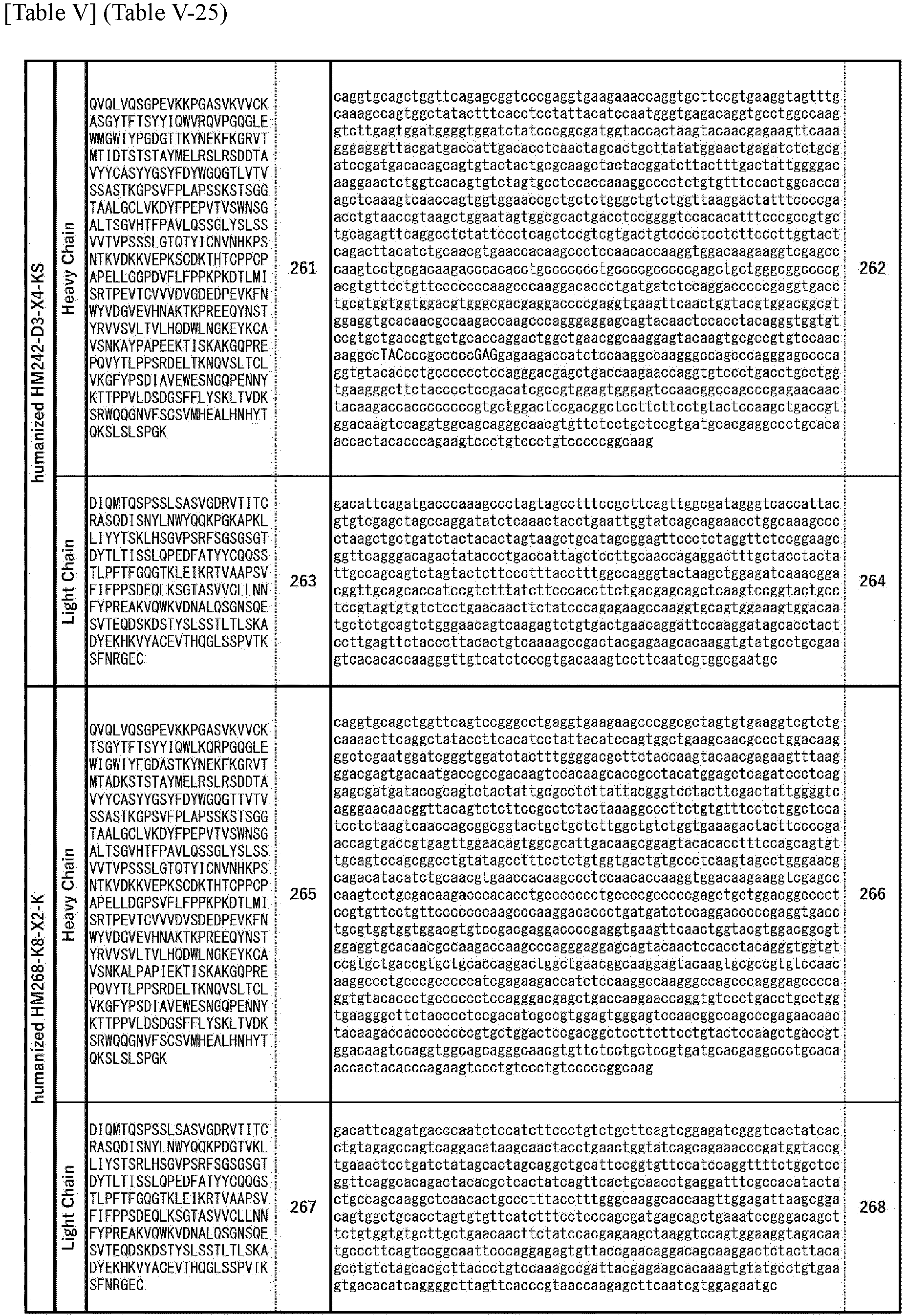

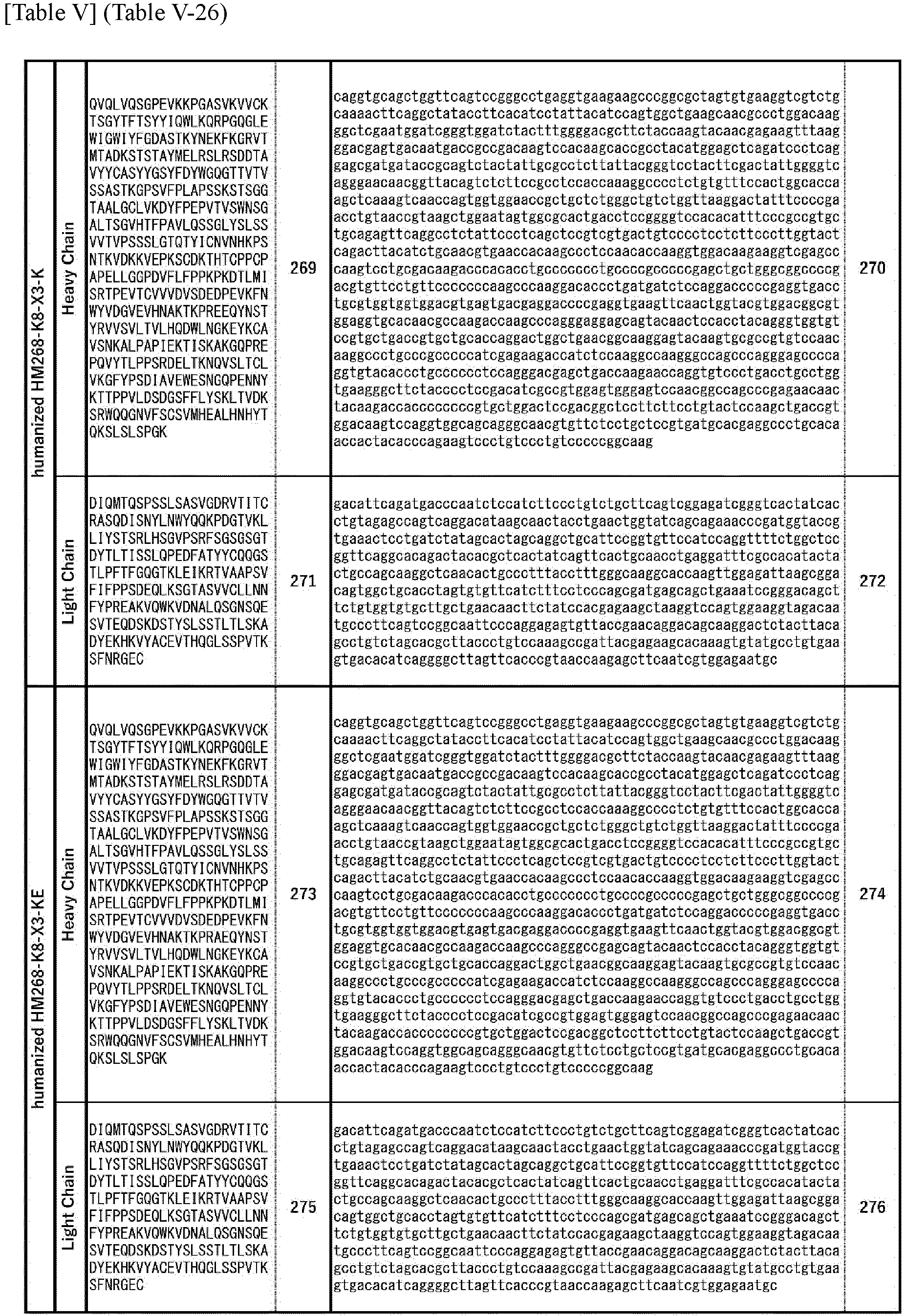

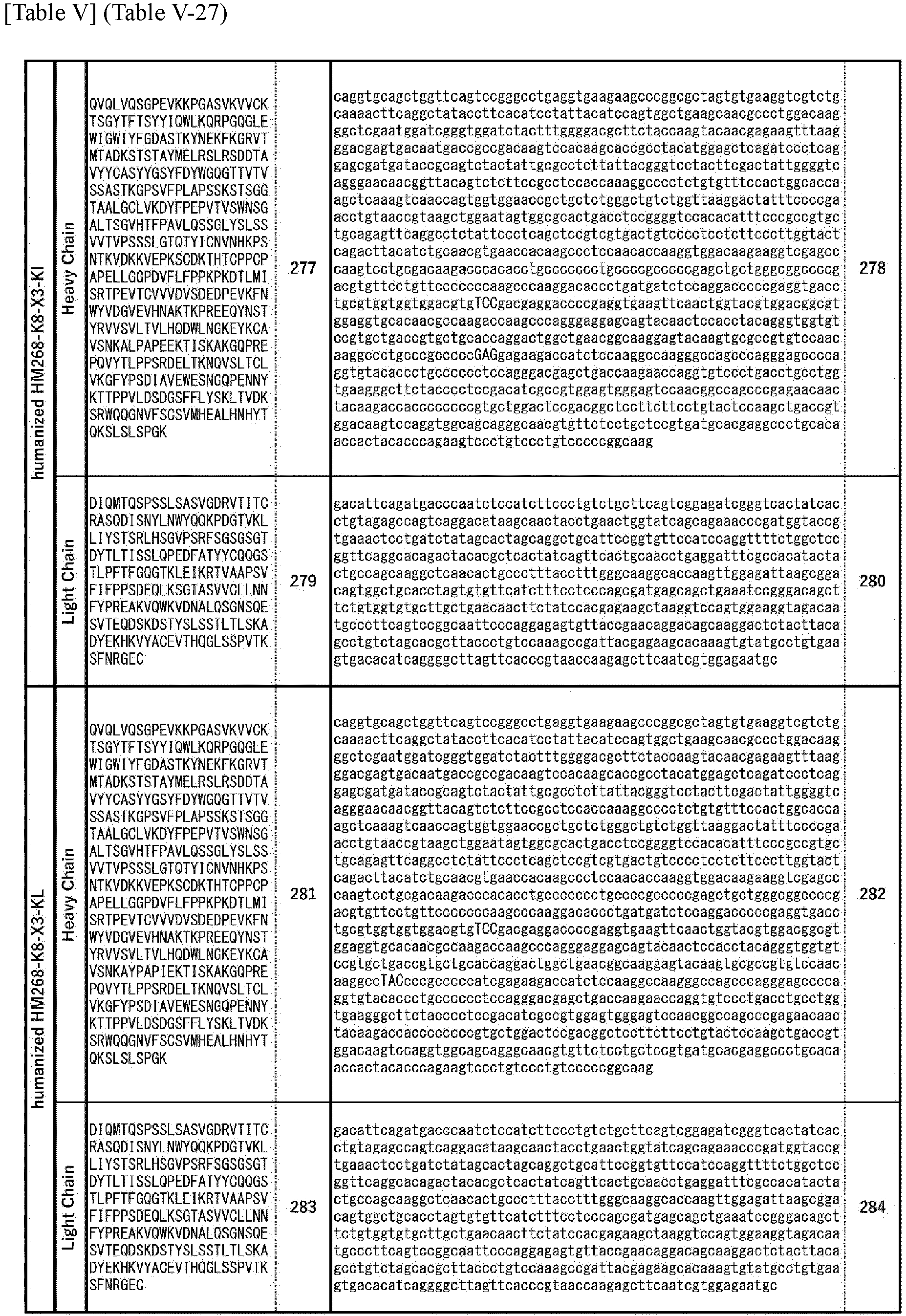

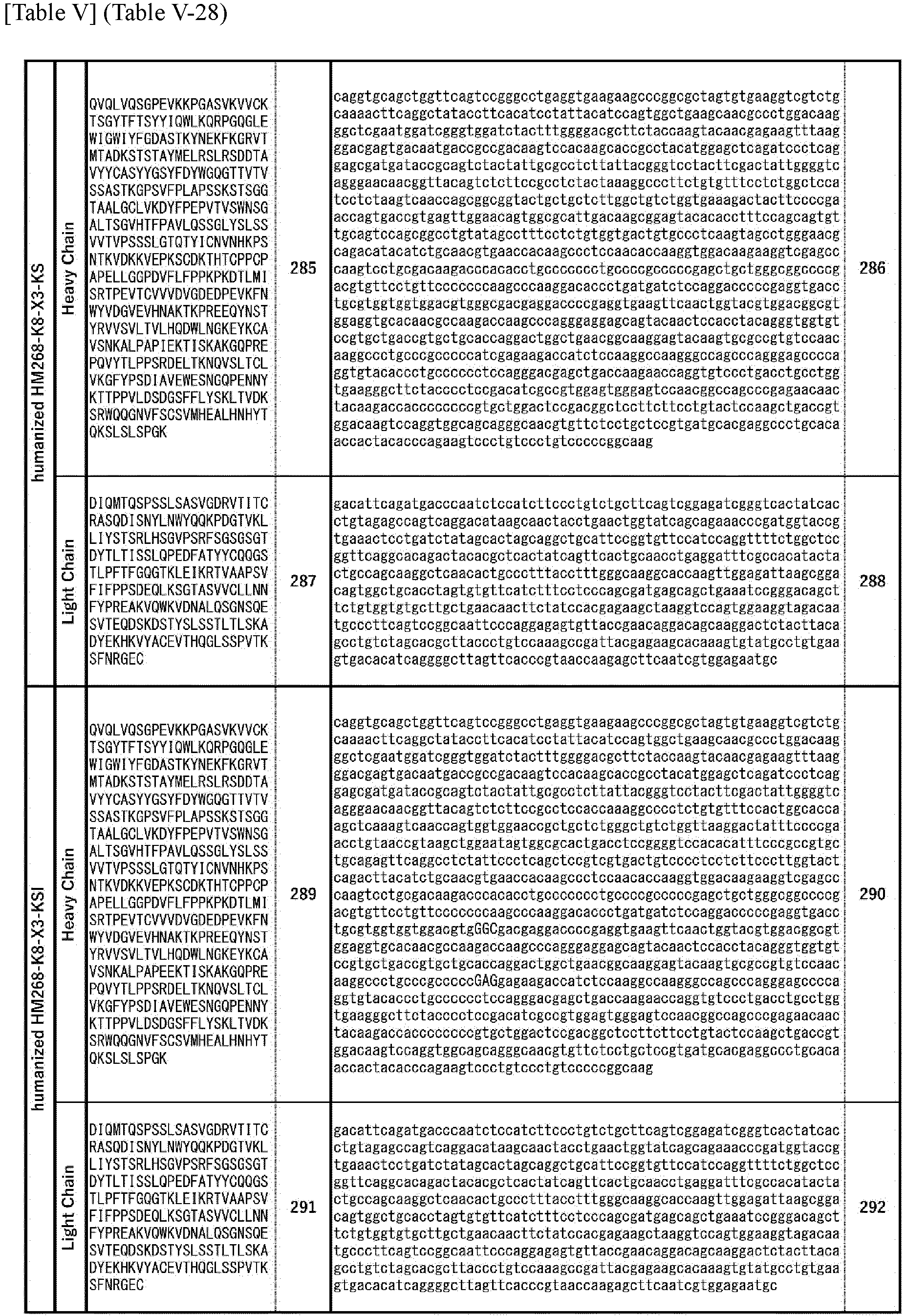

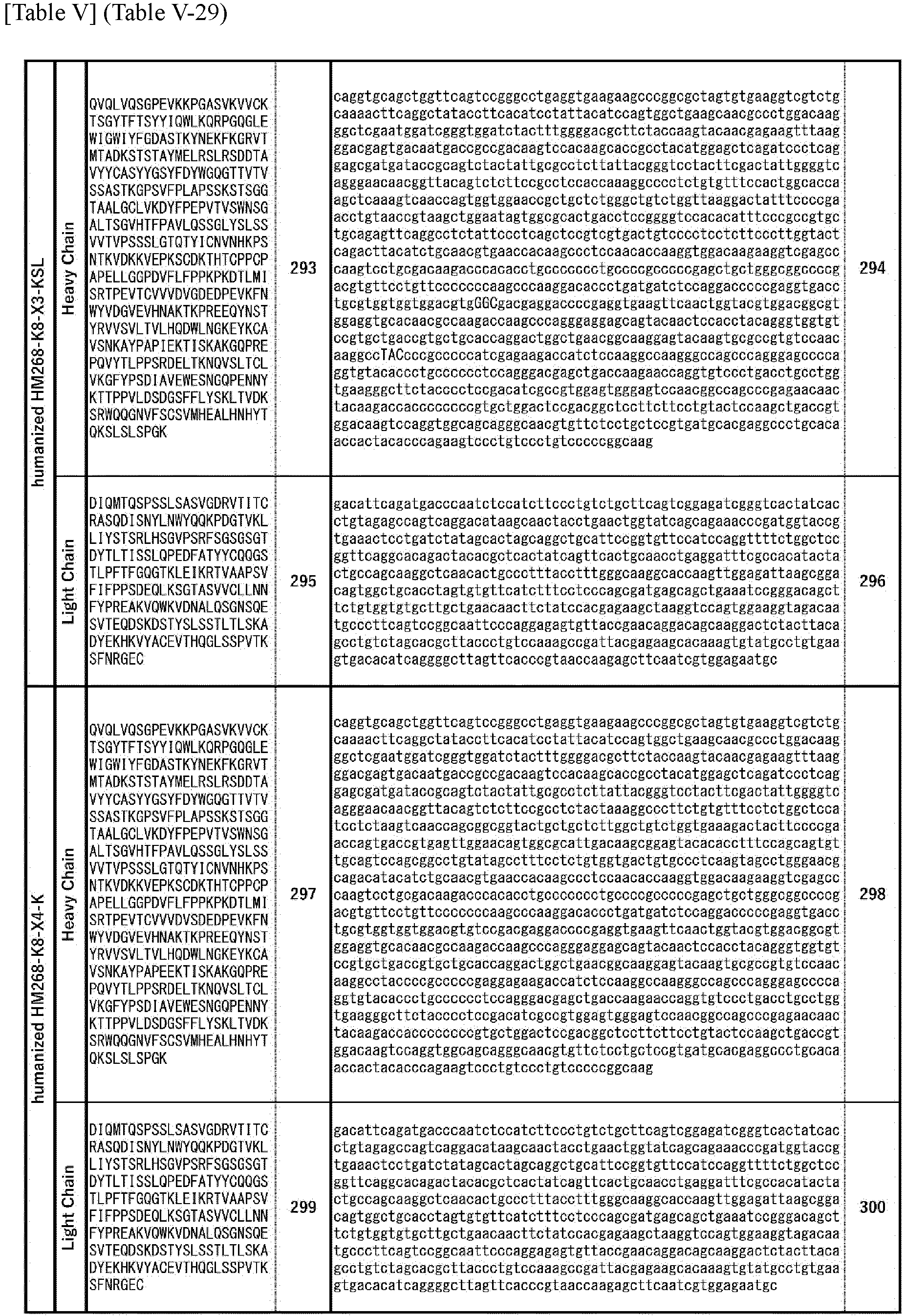

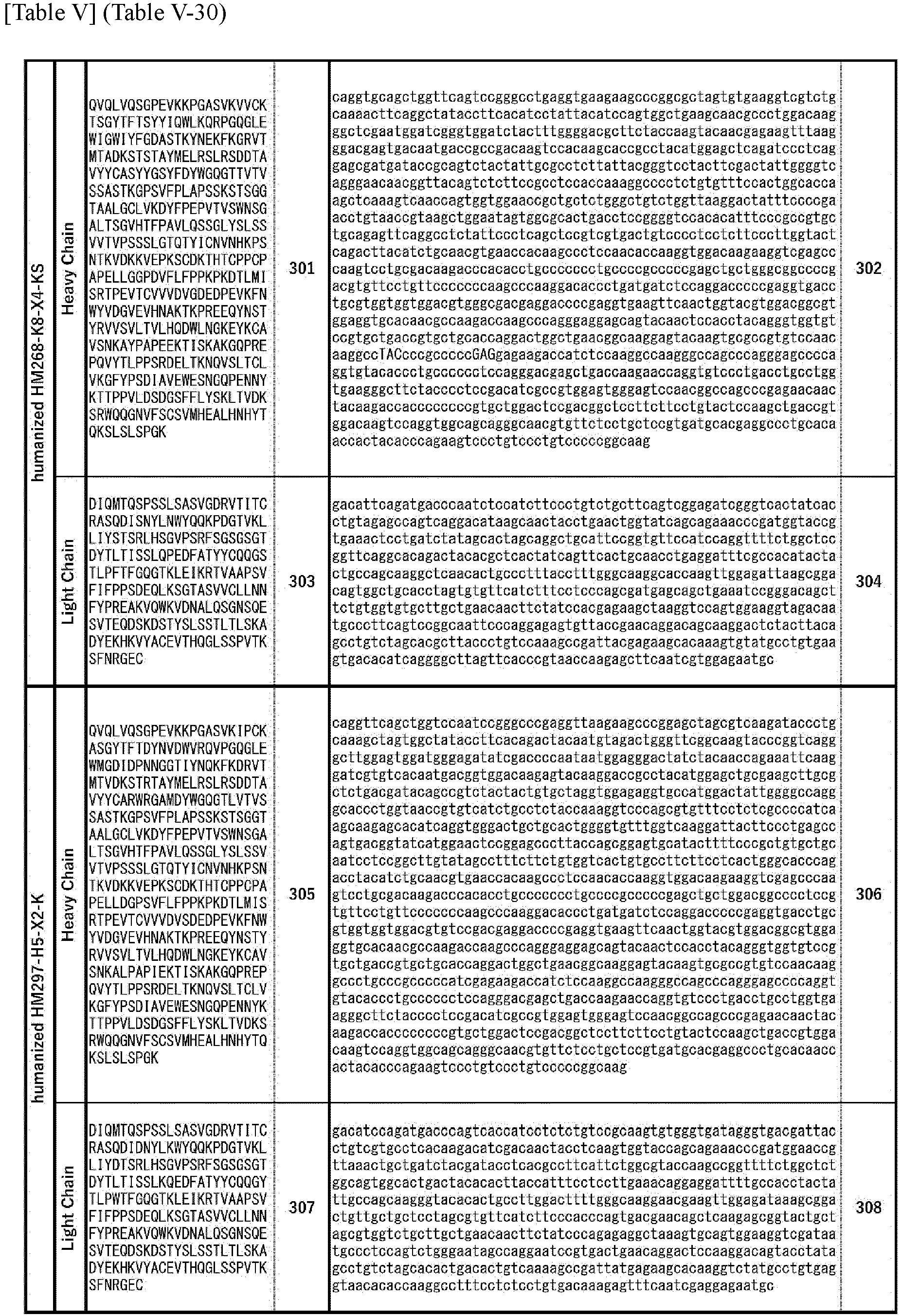

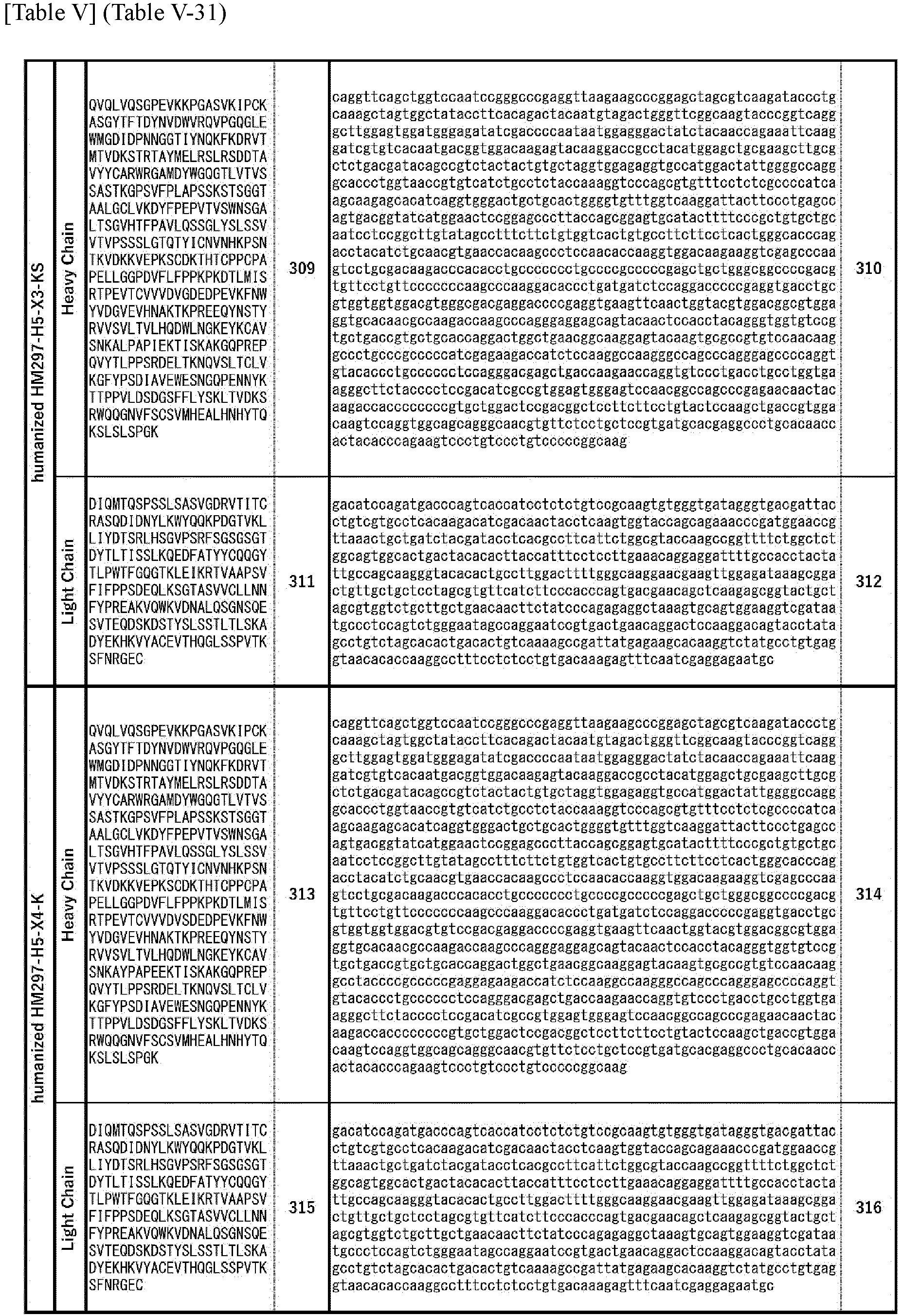

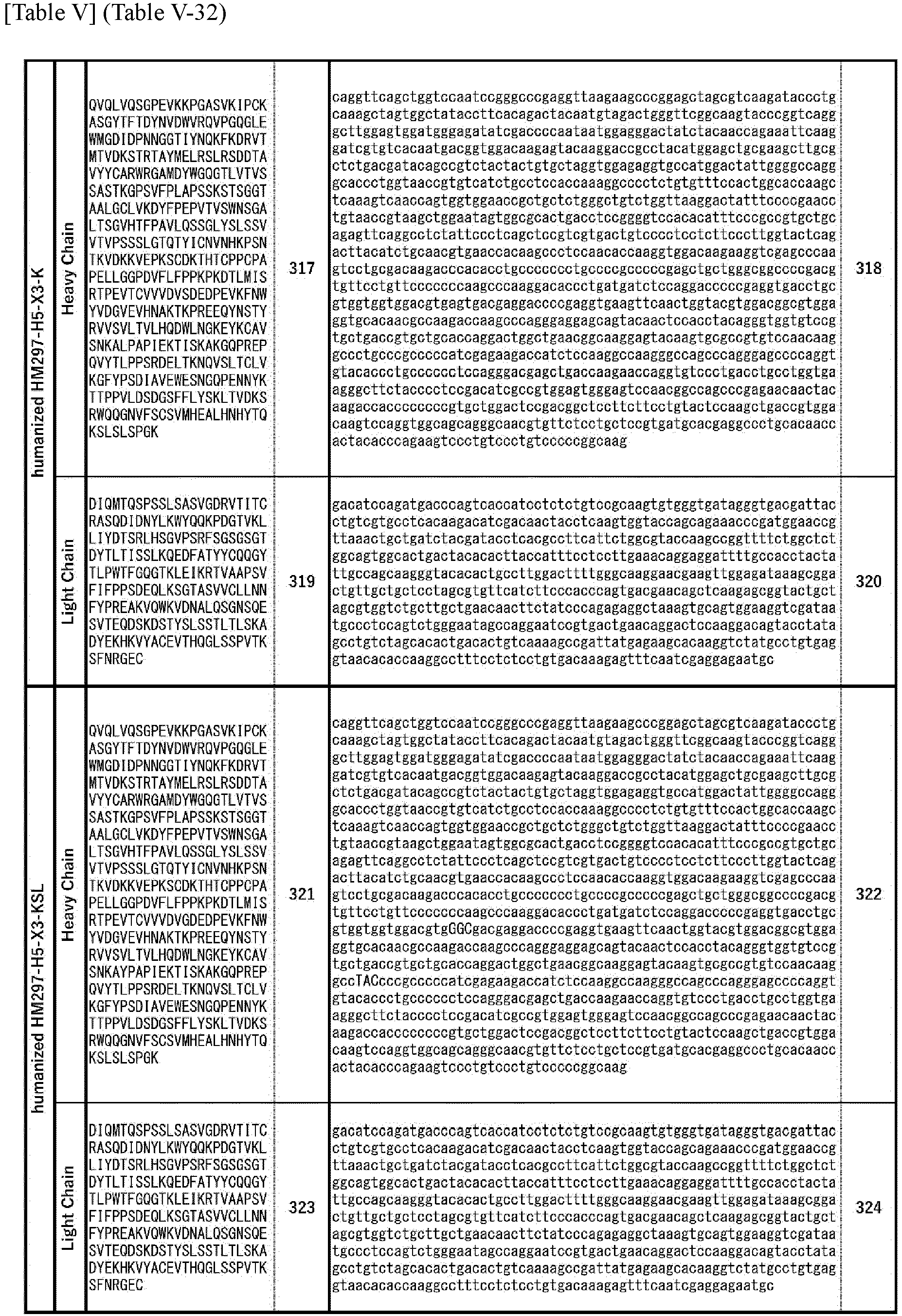

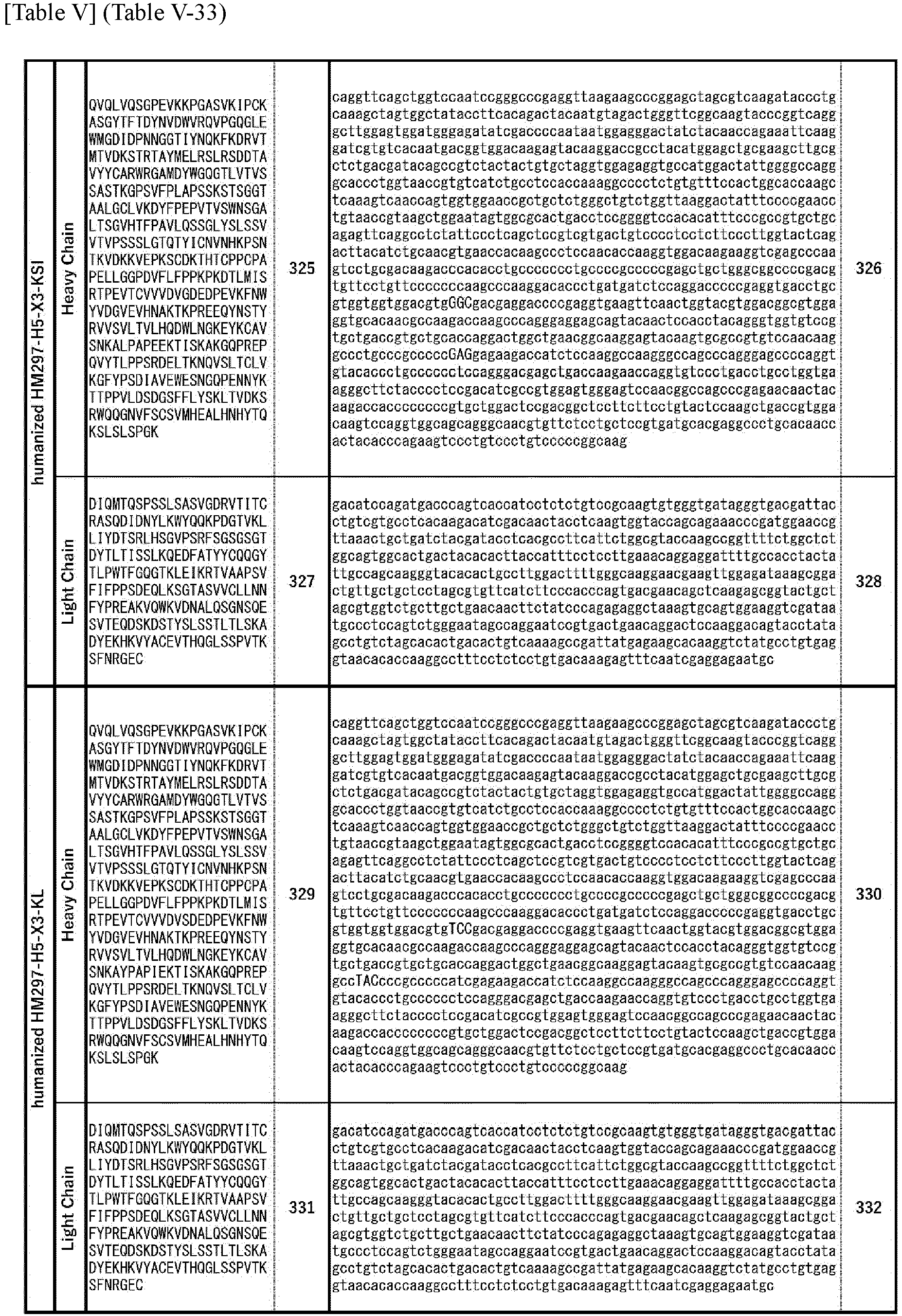

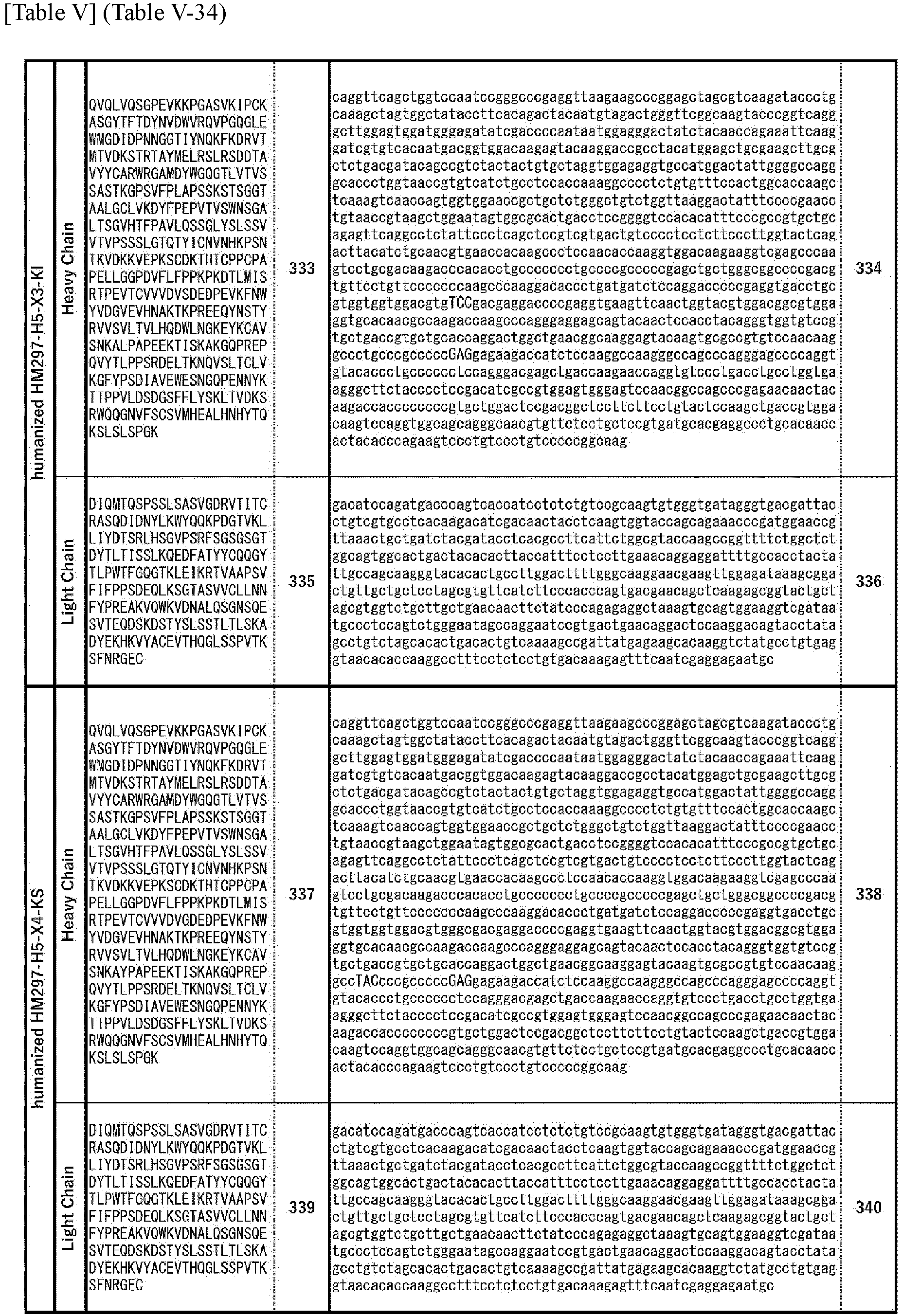

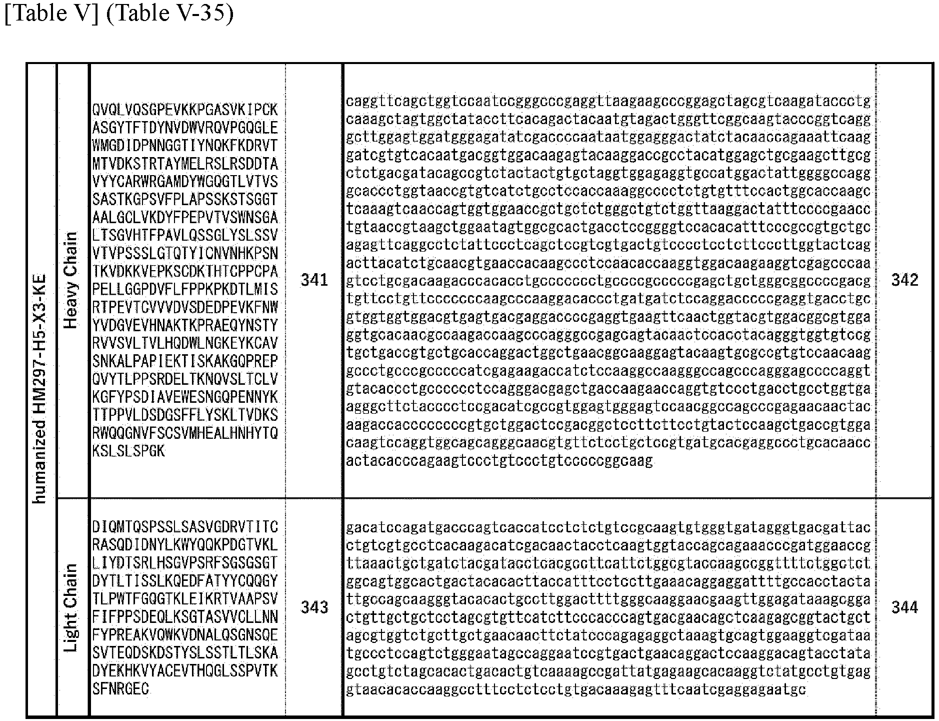

As used herein, the "functional fragment" typically means a molecule which has one or plurality of the scFv, Fv, F(ab')2, Fab' or Fab of the antibody of the present invention, and such molecule preferably binds to Fc receptors. Specific examples of functional fragment include, but are not limited to, an antibody-drug complex composed of the antibody of the present invention and a drug; a polypeptide having the scFv of the antibody of the present invention and the scFv of an anti-Fc receptor antibody; and a fusion protein having the scFv of the antibody of the present invention and an Fc region. When a functional fragment has an Fc region (for example, when a functional fragment is a fusion protein of the scFv of the antibody of the present invention and an Fc region), it is desirable that the Fc region is the Fc region of the Fc region-modified forms described in the present specification. The protein improvement and codon optimization techniques mentioned above may also be applied to functional fragments.

The antibody of the present invention and a functional fragment thereof may be prepared as a recombinant antibody or a functional fragment thereof by genetic engineering techniques. Briefly, a DNA encoding a heavy chain gene and a light chain gene of the antibody of the present invention or a DNA encoding a functional fragment of the antibody of the present invention is synthesized, inserted into an expression vector (e.g., plasmid, bacteriophage, or virus), and then introduced into host cells (e.g., CHO cells, HEK cells, etc.). By culturing the host cells, recombinant antibodies or functional fragments thereof can be obtained from the culture. Codon optimization is preferable in synthesizing a DNA encoding a heavy chain gene and a light chain gene of an antibody or a DNA encoding a functional fragment of an antibody. The heavy chain gene and the light chain gene of the antibody may be inserted into either the same expression vector or different expression vectors. The expression vector may comprise promoters, enhancers, polyadenylation signals, replication origins, selectable marker genes, and so forth. Further, the expression vector may include secretory signal peptide sequences.

The ratio of the heavy chain gene and the light chain gene of the antibody to be inserted into the expression vector is preferably 1: 1 but may be appropriately changed in order to improve the expression level of the antibody. Introduction of the recombinant expression vector into host cells may be performed by known methods such as the calcium phosphate method, the DEAE-dextran method, microinjection, lipofection, electroporation, transduction, scrape loading, the shotgun method, etc. When host cells into which a recombinant expression vector has been introduced are cultured, a recombinant antibody or a functional fragment thereof is produced in the culture. Appropriate culture conditions (medium, culture time, culture temperature, CO₂ concentration, etc.) can be appropriately selected by one of ordinary skill in the art. The thus produced antibody or a functional fragment thereof may be recovered by known methods of protein separation/purification using isoelectric point, size, solubility (in water, organic solvent, etc.), affinity to a specific substance (substrate, coenzyme, etc. in the case of an enzyme), and so forth. In order to obtain a defucosylated form of a recombinant antibody or a functional fragment thereof, 2-deoxy-2-fluoro-L-fucose is added to the medium and host cells are cultured therein, or alternatively, fucosyltransferase 8 (FUT8)-deficient cells are used as host cells. Other methods may also be used. The defucosylated form may be separated and purified by the same method as described above.

The antibody of the present invention or a functional fragment thereof exerts a more potent T cell-suppressive action than CTLA-4-Ig in human T cell / B cell mixed lymphocyte reaction (MLR), and is capable of exhibiting an anti-inflammatory action mediated by suppression of the function of human T cells (see Example described later).

The antibody of the present invention or a functional fragment thereof may be used for treatment and/or prevention of inflammatory diseases. As used herein, inflammatory diseases refer to diseases caused by excessive inflammation resulting, for example, from wounds, chemical substances, infections, or recognition of self-tissue by immune cells. Inflammatory diseases include autoimmune diseases and diseases classified as type I-IV allergies. Specific examples of such inflammatory diseases include, but are not limited to, Behcet's disease, systemic lupus erythematosus, lupus nephritis, cutaneous lupus erythematosus, chronic discoid lupus erythematosus, serum sickness, systemic sclerosis (systemic scleroderma, progressive systemic sclerosis), multiple sclerosis, scleroderma, polymyositis, dermatomyositis, periarteritis nodosa (polyarteritis nodosa), aortitis syndrome (Takayasu arteritis), malignant rheumatoid arthritis, rheumatoid arthritis, arthritis, juvenile idiopathic arthritis, spondylarthritis, mixed connective tissue disease, Sjogren's syndrome, adult-onset Still's disease, vasculitis, allergic granulomatous angiitis, hypersensitivity angiitis, rheumatoid vasculitis, large-vessel vasculitis, ANCA-associated vasculitis (e.g., microscopic polyangiitis, granulomatosis with polyangiitis, and eosinophilic granulomatosis with polyangiitis), Cogan's syndrome, RS3PE, temporal arteritis, polymyalgia rheumatica, fibromyalgia, antiphospholipid antibody syndrome, eosinophilic fasciitis, IgG4-related diseases (e.g., primary sclerosing cholangitis, autoimmune pancreatitis, etc.), Guillain-Barre syndrome, myasthenia gravis, chronic atrophic gastritis, hepatitis, autoimmune hepatitis, nonalcoholic steatohepatitis, primary biliary cirrhosis, Goodpasture's syndrome, glomerulonephritis, rapidly progressive glomerulonephritis, megaloblastic anemia, hemolytic anemia, autoimmune hemolytic anemia, Coombs positive autoimmune hemolytic anemia, pernicious anemia, autoimmune neutropenia, idiopathic thrombocytopenic purpura, Basedow's disease (Graves' disease (hyperthyroidism)), Hashimoto's thyroiditis, autoimmune adrenal insufficiency, primary hypothyroidism, Addison's disease (chronic adrenal insufficiency), idiopathic Addison's disease, type I diabetes mellitus, slowly progressive insulin-dependent diabetes mellitus (latent adult onset autoimmune diabetes), localized scleroderma, psoriasis, psoriatic arthritis, bullous pemphigoid, pemphigus, pemphigoid, herpes gestationis, linear IgA bullous skin disease, epidermolysis bullosa acquisita, alopecia areata, vitiligo, vitiligo vulgaris, Stevens-Johnson syndrome, fixed drug eruption, neuromyelitis optica, chronic inflammatory demyelinating polyneuropathy, multifocal motor neuropathy, sarcoidosis, giant cell arteritis, amyotrophic lateral sclerosis, Harada disease, autoimmune optic neuropathy, idiopathic azoospermia, spontaneous abortion, recurrent fetal loss, infertility, infertility related to lack of fetal-maternal tolerance, inflammatory bowel diseases (e.g., ulcerative colitis, Crohn's disease), celiac disease, ankylosing spondylitis, asthma, severe asthma, infections (viral, bacterial, fungal, parasitic), chronic obstructive pulmonary disease, urticaria, chronic urticaria, transplantation immunity, familial Mediterranean fever, eosinophilic sinusitis, eosinophilic gastrointestinal disease, dilated cardiomyopathy, systemic mastocytosis, inclusion body myositis, pelvic inflammatory disease, Alzheimer's disease, Peyronie's disease, gallbladder disease, pilonidal disease, peritonitis, surgical adhesions, stroke, Lyme disease, meningoencephalitis, autoimmune uveitis, non-infectious uveitis, hypersensitivity, chronic allergic diseases (atopic dermatitis, fever, allergic rhinitis), food allergy, conjunctivitis, anaphylactic shock, contact dermatitis, fibrosing alveolitis, hypersensitivity pneumonitis, allergic bronchopulmonary aspergillosis, allergic encephalitis, IgA nephropathy, Meniere's disease, cholangitis, pancreatitis, Trauma (surgery) , acute and chronic graft-vs-host disease, transplant rejection, heart diseases (ischemic diseases such as myocardial infarction, atherosclerosis), intravascular coagulation, osteoporosis, osteoarthritis, periodontitis, hypoxia, gestational herpes, acquired hypogonadism, hypoparathyroidism, and cytokine storm.

The antibody of the present invention or a functional fragment thereof may be used for treatment and/or prevention of inflammatory diseases, especially those diseases in which T cells and autoantibodies are involved, such as systemic lupus erythematosus, lupus nephritis, multiple sclerosis, systemic sclerosis (systemic scleroderma, progressive systemic sclerosis), multiple sclerosis, scleroderma, polymyositis, dermatomyositis, rheumatoid arthritis, Sjogren's syndrome, allergic granulomatous angiitis, large-vessel vasculitis, ANCA-associated vasculitis (e.g., microscopic polyangiitis, granulomatosis with polyangiitis, and eosinophilic granulomatosis with polyangiitis), antiphospholipid antibody syndrome, IgG4-related diseases (e.g., primary sclerosing cholangitis, autoimmune pancreatitis, etc.), idiopathic thrombocytopenic purpura, Basedow's disease (Graves' disease (hyperthyroidism)), Hashimoto's throiditis, psoriasis, psoriatic arthritis, bullous pemphigoid, pemphigus, pemphigoid, alopecia areata, vitiligo, vitiligo vulgaris, Stevens-Johnson syndrome, fixed drug eruption, neuromyelitis optica, inflammatory bowel diseases (e.g., ulcerative colitis, Crohn's disease), asthma, and acute and chronic graft-vs-host disease.

The present invention provides a pharmaceutical composition comprising an agonist antibody to human PD-1 (anti-human PD-1 agonist antibody) or a functional fragment thereof, wherein the antibody or a functional fragment thereof binds to domain #7 of human PD-1 as shown in SEQ ID NO: 9, and a pharmaceutically acceptable carrier. The pharmaceutical composition of the present invention may be used as a pharmaceutical drug.

The pharmaceutical composition of the present invention may be administered to subjects (human or non-human animal) systemically or locally by an oral or parenteral route.

The pharmaceutical composition of the present invention may comprise an effective amount of the anti-human PD-1 agonist antibody or a fragment thereof, and may be formulated into a preparation by mixing, dissolving, emulsifying, encapsulating, lyophilizing, etc. with a pharmaceutically acceptable carrier.

The pharmaceutical composition of the present invention is suitable for either oral or parenteral administration. Preferable preparations for oral administration include, but are not limited to, liquids which have an effective amount of the anti-human PD-1 agonist antibody or a functional fragment thereof dissolved in a diluent such as water or physiological saline; capsules, granules, powders or tablets containing an effective amount of said antibody or fragment as a solid or granules; suspensions which have an effective amount of said antibody or fragment is suspended in an appropriate dispersion medium; and emulsions prepared by first dissolving an effective amount of said antibody or fragment in a solution which is then dispersed and emulsified in an appropriate dispersion medium.

For parenteral administration, the anti-human PD-1 agonist antibody or a functional fragment thereof may be formulated into injections (including lyophilized formulation), suspensions, emulsions, creams, ointments, inhalants, suppositories or the like, together with pharmaceutically acceptable solvents, excipients, binders, stabilizers, dispersants and the like. In the formulation of injections, the anti-human PD-1 agonist antibody or a functional fragment thereof may be dissolved in an aqueous solution (preferably Hanks solution or Ringer solution) or a physiologically compatible buffer such as physiological saline buffer. Further, the pharmaceutical composition of the present invention may take the form of suspension, solution or emulsion, etc. in an oily or aqueous vehicle. Alternatively, the anti-human PD-1 agonist antibody or a functional fragment thereof may be produced in the form of a powder, which may be prepared into an aqueous solution or suspension with sterile water or the like before use. For administration by inhalation, the anti-human PD-1 agonist antibody or a functional fragment thereof may be pulverized to prepare a powder mixture with an appropriate base such as lactose or starch. Suppository formulations may be prepared by mixing the anti-human PD-1 agonist antibody or a functional fragment thereof with a routinely used suppository base such as cocoa butter. Further, the pharmaceutical composition may be formulated as a sustained release preparation by encapsulating in a polymer matrix or the like. Parenteral administration includes, but are not limited to, intravenous, intramuscular, subcutaneous, rectal, nasal, intraoral, and transdermal administration.

The dose may be about 0.1 to 100 mg/kg (body weight) per human adult, and this dose may be administered once or multiple times at intervals of about 1 day to 6 months.

The pharmaceutical composition of the present invention may be used alone. Alternatively, the composition may be used in combination with other therapeutics such as antihistamines, antiallergics, vasoconstrictive nasal sprays, steroids, small molecule immunosuppressants (cyclosporine, tachlorimus, etc.), antibody preparations (e.g., anti-IgE antibody, anti-IL-4 antibody, anti-IL-5 antibody, anti-IL-6 antibody, anti-IL-13 antibody, anti-IL-4 receptor antibody, anti-IL-5 receptor antibody, anti-IL-22 antibody, anti-IL-25 antibody, anti-IL-33 antibody, anti-TNF-α antibody, anti-TSLP antibody, anti-BAFF antibody, etc.), and recombinant soluble fusion proteins (CTLA-4-Ig, etc.). From such a combined use, synergism of drug efficacies can be expected.

### EXAMPLES

Hereinbelow, the present invention will be described more specifically with reference to the following Example.

### Methods

### Commercially Available Antibodies, Commercially Available Fusion Proteins, and Known Antibodies

Regarding commercially available antibodies and recombinant protein: Anti-human PD-1 antibody (clone: EH12.2H7, BioLegend), anti-human PD-1 antibody (clone: J116, Invitrogen), anti-human PD-1 antibody (clone: MIH4, Invitrogen), control mouse IgG1 (clone: MOPC-21, BioLegend), control human IgG1 (clone: QA16A12, BioLegend), control human IgG4 (clone: QA16A15, BioLegend), and recombinant human CTLA-4-Fc region chimeric protein (CTLA-4-Ig, Cat#: 591908, BioLegend) were used. Regarding known antibodies: 949 (anti-human PD-1 antibody described in WO2011/110621, its sequence of Fc region is human IgG4-S228P), PD1AB-6 (PD1AB-6-IgG1-K322A described in WO2017/058859), PD1-17 (anti-human PD-1 antibody described in WO2004/056875, its sequence of Fc region is human IgG1), 3.7C6 (anti-human PD-1 antibody described in WO2020/247648, its sequence of Fc region is human IgG1) and Antibody 1 (anti-human PD-1 antibody described in WO2019/168745, its sequence of Fc region is human IgG1) were prepared by the method described in below.

### Acquisition and Purification of Novel Antibodies

Techniques for producing anti-human PD-1 mouse monoclonal antibody are known in the art. For example, the method described in Goding, Monoclonal Antibodies: Principles and Practice, pp. 59-103 (Academic Press, 1986) was used. As immunization host, A/J and BALB/c mice were used. As immunogens, a plasmid vector expressing the full length or a mutant of human PD-1 protein (NCBI accession number: NP_005009.2) (15-50 µg), a fusion protein of recombinant human PD-1 extracellular domain and human IgG1-Fc region (25 µg), and 293T cells so engineered to transiently express human PD-1 or a mutant thereof (5 × 10⁷ cells) were used. One of these immunogens was intramuscularly, intradermally, intraperitoneally or intravenously injected at an interval of 10 to 50 days. When adjuvant was necessary, Sigma adjuvant system (S6322-1VL; Sigma-Aldrich) was used. Three days after the final immunization, splenocytes of the immunization host and P3U1 mouse myeloma cells were fused to prepare hybridomas. Screening for hybridomas producing anti-human PD-1 antibody was performed as follows. Briefly, the culture supernatant of each hybridoma was added to HEK293 cells which had been rendered to express human PD-1. After staining with R-phycoerythrin-labeled goat anti-mouse IgG(H+L) F(ab')2 fragment (115-116-146; Jackson ImmunoResearch) as secondary antibody, flow cytometry was performed for analysis. Finally selected hybridomas were cloned by limiting dilution analysis, and cultured at high density in a cell line bioreactor (Wheaton). From the resultant culture supernatants, anti-human PD-1 antibodies were purified using Ab-Capture ExTra (P-003-10; Protenova).

### Antibody Sequencing

In order to specify the variable region sequence of anti-human PD-1 antibodies, frozen hybridomas were sent to BizComJapan, Inc. to use contract analysis services provided by Fusion Antibodies, Plc.

### Humanization of Anti-Human PD-1 Antibody

To perform humanization, the complementarity determining region (CDR) of mouse antibody and the residues necessary for retaining CDR conformation of mouse antibody were transplanted into the framework of human antibody.

### Expression and Purification of Recombinant Antibodies

A heavy chain expression vector was constructed using a DNA encoding a heavy chain sequence and an expression vector (pcDNA3.4, Thermo Fisher Scientific). Likewise, a light chain expression vector was constructed using a DNA encoding a light chain sequence and an expression vector (pcDNA3.4, Thermo Fisher Scientific). The two vectors were mixed in such a manner that the ratio of heavy chain and light chain would be 1:1, and transfected into CHO cells at 0.8 µg of DNA per ml of culture medium using ExpiFectamine CHO Reagent (Thermo Fisher Scientific). One day after the transfection, ExpiCHO Feed and ExpiFectamine CHO Enhancer were added to the cells, which were then cultured at 32°C for 10 to 12 days. The culture medium was subjected to centrifugation and filtration to remove cells and the supernatant was collected. The amount of antibody in the culture supernatant was measured with Cedex Bio (Roche Diagnostic). The culture supernatant was applied to Protein A column pre-equilibrated with phosphate-buffered saline (PBS), and then the column was washed with PBS. Antibody was eluted with citrate buffer (pH 3.4), and the eluent was neutralized by adding 160 µl of 1M Tris-HCl (pH 9.0) per ml of eluent. After concentration by ultrafiltration, antibody was purified by gel filtration chromatography using Superdex 200 Increase 10/300 GL (Cytiva). Defucosylated antibody was prepared by adding 2-deoxy-2-fluoro-L-fucose (2-DFF) to the culture medium to give a final concentration of 10 to 1000 µM. Comparable levels of FcyRIIIA binding affinity were observed when the final concentration of 2-DFF was within the range of 10 to 1000 µM. Purification of the defucosylated antibody was performed in the same manner as described above.

### Analysis by SDS-PAGE

The purified antibody was suspended in a sample buffer (with reductant and without reductant) and heated at 98°C for 3 minutes. The resultant sample was applied to 4-15% Mini-PROTEAN TGX polyacrylamide gel (Bio-Rad) and electrophoresed at a constant voltage of 200 V for 30 minutes. Staining was performed with SYPRO Ruby Protein Gel Stain (Thermo Fisher Scientific), followed by analysis with LAS500 (Cytiva).

### Analysis by SEC-HPLC

The ratio of monomers contained in samples was measured by SEC-HPLC. As a column, TSKgel G3000SWXL, 5 µm, 7.8 mm × 300 mm (TOSOH) was used. As regards a mobile phase, 84.4 g of potassium dihydrogen phosphate and 66.2 g of dipotassium hydrogen phosphate were dissolved in water to give a 1000 ml solution, which was diluted 10-fold for use. For the ratio of monomers, each corresponding peak area of a sample solution was measured by the automatic integration method, and the proportions of the respective areas were determined by the area normalization method.

### Evaluation of Thermal stability with Differential Scanning Calorimeter

For evaluation of the thermal stability of anti-human PD-1 antibody, denaturation midpoint temperature (Tₘ) and denaturation onset temperature (Tₒₙₛₑₜ), each used as a predictive indicator, were measured. For 400 µl aliquots of anti-human PD-1 antibody sample (diluted to 0.1 mg/ml), calorimetric change that occurred when temperature was raised from 25°C to 100°C at a rate of 200°C/hr was measured with a differential scanning calorimeter (Microcal PEAQ-DSC; Malvern Panalytical). As a reference for measurement, PBS was used. With respect to the resultant data, baseline correction based on the result of buffer measurement and fitting analysis with a non-two-state model were conducted. Then, Tₘ and Tₒₙₛₑₜ for Fab, CH2 and CH3 regions were calculated.

### PD-1 Binding Affinity Measurement Test Using Surface Plasmon Resonance Technique

The binding affinity of each antibody to human PD-1 was measured with Biacore 8K (Cytiva). Briefly, anti-His Tag antibody was immobilized by amine coupling using Amine Coupling Kit and His Capture Kit, onto 8 channels of a sensor chip (provided with 16 flow cells; one channel consisting of two flow cells) which had carboxymethyl dextran immobilized on a gold film. The immobilization was performed by feeding an activation solution (NHS and EDC mixed in equal amounts) at a flow rate of 10 µl/min for 7 minutes, an immobilization solution at a flow rate of 5 µl/min for 7 minutes, and 1 M ethanolamine hydrochloride-NaOH (pH 8.5) at a flow rate of 10 µl/min for 7 minutes. The buffer used at the time of immobilization was 1x HBS-EP+ Buffer. After the immobilization and before applying a sample, Human PD-1/PDCD1 Protein (PD-1) adjusted to 1 µg/ml with 1x HBS-EP+ Buffer was fed to one of the two flow cells of each channel in an amount of 20 µl at a flow rate of 10 µl/min to thereby capture PD-1 on the sensor chip. As regards the other flow cell, 1x HBS-EP+ Buffer was fed in the same manner to prepare a control flow cell. Each antibody was adjusted to 16 nM with 1x HBS-EP+ Buffer, and then subjected to 2-fold 7-step serial dilution using 1x HBS-EP+ Buffer. The resultant sample solution (30 µl) was fed to each flow cell at a flow rate of 10 µl/min and the response was measured. After subtracting the response of control flow cell from the response of the flow cell which was allowed to capture the ligand, K_{D} value was calculated by curve fitting using Biacore 8K Evaluation Software.

### Fc Receptor Binding Affinity Measurement Test Using Surface Plasmon Resonance Technique

The binding affinity of each antibody to Fc receptors [FcyRIIB/C (CD32b/c) (FcyRIIB) (R&D Systems) and Fc gamma RIIIA/CD16a, CF (FcyRIIIA(V158)) (R&D Systems)] was measured with Biacore 8K. Briefly, anti-His Tag antibody was immobilized by amine coupling using Amine Coupling Kit and His Capture Kit onto 8 channels of a sensor chip (provided with 16 flow cells; one channel consisting of two flow cells) which had carboxymethyl dextran immobilized on a gold film. The immobilization was performed by feeding an activation solution (NHS and EDC mixed in equal amounts) at a flow rate of 10 µl/min for 7 minutes, an immobilization solution at a flow rate of 5 µl/min for 7 minutes, and 1 M ethanolamine hydrochloride-NaOH (pH 8.5) at a flow rate of 10 µl/min for 7 minutes. The buffer used at the time of immobilization was 1x HBS-EP+ Buffer. After the immobilization and before applying a sample, Fc receptor adjusted to 1 µg/ml with 1x HBS-EP+ Buffer was fed to one of the two flow cells of each channel in an amount of 20 µl at a flow rate of 10 µl/min to thereby capture Fc receptor on the sensor chip. As regards the other flow cell, 1x HBS-EP+ Buffer was fed in the same manner to prepare a control flow cell. Each antibody was adjusted to 1 mg/ml with 1x HBS-EP+ Buffer, and then subjected to 2-fold 7-step serial dilution using 1x HBS-EP Buffer (when measuring FcγRIIIA(V158), 4-fold 7-step serial dilution was performed). The resultant sample solution (15 µl) was fed to each flow cell at a flow rate of 30 µl/min and the response was measured. After subtracting the response of control flow cell from the response of the flow cell which was allowed to capture the ligand, K_{D} value was calculated by analysis of equilibrium constants using Biacore8 K Evaluation Software.

### Activity Evaluation on Cell Lines

The activity of anti-human PD-1 antibody was evaluated as an effect on cytokine production caused by interaction between human PD-1-expressing T cells and antigen-presenting cells. Prepared as T cells were: a cell line obtained by treating DO11.10 T cell hybridoma cell line (kindly provided by Department of Immunology and Genomic Medicine, Graduate School of Medicine, Kyoto University) with Cas9 (Invitrogen) to knock-out mouse PD-1; and the knock-out cell line which was rendered to express human PD-1. Prepared as antigen-presenting cells were: a cell line obtained from IIA1.6 B cell line (kindly provided by Department of Immunology and Genomic Medicine, Graduate School of Medicine, Kyoto University) by knocking out mouse PD-L1; and the knock-out cell line which was rendered to express human PD-L1 or mouse FcyRIIB. For evaluating human PD-1 agonist activity, mouse or human FcγRIIB-expressing IIA1.6 cells were used; and for evaluating antagonist activity, human PD-L1-expressing IIA1.6 cells were used. Further, for evaluating dependency on each Fc receptor, IIA1.6 cells expressing various human Fc receptors were used. Human PD-1-expressing DO11.10 T cell hybridomas and respective IIA1.6 cells were suspended in a medium (10% fetal bovine serum-containing RPMI1640 medium). DO11.10 T cell hybridomas and IIA1.6 cells were seeded in round bottom 96-well plates at 5 × 10⁴ and 1 × 10⁴ cells/well/50 µl, respectively. Anti-human PD-1 antibody was added to the plates to give a final concentration of 5, 0.5, 0.05 or 0.005 µg/ml at 50 µl/well. Subsequently, OVA₃₂₃₋₃₃₉ peptide (Eurofins) was added as antigen to give a final concentration of 2 µg/ml at 50 µl/well. Eighteen hours later, IL-2 concentration in the culture supernatant was measured using mouse IL-2 DuoSet ELISA (R&D Systems).

In the evaluation of human PD-1 agonist activity, the cytokine suppression obtained by using human PD-L1-expressing IIA1.6 cells was taken as positive control; and the result obtained by using DO11.10 T cell hybridomas not rendered to express human PD-1 was taken as negative control. In the evaluation of human PD-1 antagonist activity, anti-human PD-1 antibody (clone: EH12.2H7) with known antagonist activity was used.

### Measurement of Affinity to Human Fc Receptor-Expressing Cell Line with Flow Cytometer

Various antibodies (5 µg/ml, 50 µl each) were added to IIA1.6 cells which had been rendered to express human FcyRIIB or human FcyRIIIA. The cells were incubated at 4°C for 15 minutes. Then, cells were washed, and antibodies bound to cell surfaces were stained by incubating with APC-labeled anti-human IgG Fab antibody at 4°C for 15 minutes. After washing cells again, antibodies bound to cell surfaces were detected by FACS analysis and GMFI was calculated as affinity to FcyRIIB or FcyRIIIA.

### Analysis of the Binding Characteristics of Anti-Human PD-1 Antibody

The gene of human PD-1 (wild-type) or substituted human PD-1 in which regions 1 to 8 as shown in Fig. 2A were replaced with mouse sequences and a gene of fluorescent protein such as GFP were co-expressed in a cell line (such as DO11.10 T cell hybridoma or HEK293T) by means of a vector ligating the genes with IRES sequences. The expression level of PD-1 on this cell line is correlated with the expression level of fluorescent protein such as GFP. Anti-PD-1 antibody was bound to the PD-1/GFP expressing cells, and the bound antibody was detected with a secondary antibody labeled with a different fluorescent dye. Briefly, PD-1-expressing cells were seeded in V-shaped bottom 96-well plates at 1 × 10⁵ cells/well. To each cell pellet, 50 µl of anti-human PD-1 antibody adjusted to 5 µg/ml was added and mixed. The mixture was incubated at 4°C for 15 minutes. After washing, BV421-labeled anti-mouse IgG antibody or BV421-labeled anti-human IgG antibody, each adjusted to 5 µg/ml, was added in 50 µl. The mixture was incubated at 4°C for 15 minutes. After washing, cells were suspended in 200 µl of buffer, and passed through a 70 µm filter. The fluorescence intensity of the filtrate was measured with a flow cytometer. The thus obtained fluorescence intensity can be rephrased as the binding capacity of anti-human PD-1 antibody to human PD-1 (wild-type or substituted). When the binding capacity to substituted human PD-1 which had a specific region replaced with the corresponding sequence of mouse PD-1 decreased compared to the binding capacity to wild-type human PD-1, that specific region was defined as a region binding the antibody. Binding characteristics to R143A point mutant or substituted Mouse PD-1 (hu38-48) were also analyzed in the same manner.

### Preparation of Human PD-1 Knock-In Mouse using Genome Editing

gRNA targeting 5'-GCCAGGGGCTCTGGGCATGT-3' and a donor vector containing human PD-1 gene was microinjected into C57BL/6N mouse-derived pronuclear stage fertilized egg together with Cas9 protein (Invitrogen). The resultant egg was transplanted into the oviduct of foster mouse. With respect to the resultant mice (F0), indel mice were selected and mated with wild-type mice to obtain F1 mice. Those F1 mice confirmed for gene transfer were further mated to thereby obtain homozygous mice. These homozygous mice were used in experiments as human PD-1 knock-in mice.

### Evaluation of PD-1 Agonist using House Dust Mite Extract (HDM)-Induced Allergy Model

In experiments under preventive regimen, HDM (D. Pteronyssinus; Greer) (total protein: 400 ng; 10 ng in terms of Derp1) was administered intraperitoneally to human PD-1 knock-in mouse. Simultaneously, 500 µg of anti-human PD-1 antibody was administered intraperitoneally (Day 0). Further, the same dose of anti-human PD-1 antibody was administered intraperitoneally after 3 days, 7 days and 10 days. In experiments under therapeutic regimen, anti-human PD-1 antibody was administered only once after 10 days. From 7 days after intraperitoneal administration of HDM, HDM (total protein 25 µg/25 µl) was administered intranasally under anesthesia. This intranasal administration was performed for 8 consecutive days. Four hours after final intranasal administration, mice were euthanized after collecting blood samples, and bronchoalveolar lavage fluid and the lung were collected. Mononuclear cells infiltrating into the alveoli were isolated from the bronchoalveolar lavage fluid. The lung was subjected to enzyme treatment and density gradient centrifugation to isolate mononuclear cells and the numbers of CD4⁺ T cells, eosinophils (CD11c⁻ SiglecF⁺) and the like were determined by FACS analysis. Intracellular cytokines in bronchoalveolar lavage fluid-derived mononuclear cells and lung-derived mononuclear cells were analyzed by FACS. Blood concentration of HDM specific IgE was measured by ELISA (mouse serum anti-HDM IgE antibody assay kit; Chondrex).

### Evaluation of PD-1 Agonist using Acute Graft-Versus-Host Disease (GVHD) Model

Cyclophosphamide (Fuji Film Wako Pure Chemical) dissolved in PBS was intraperitoneally administered to BDF1 mice (recipient, H-2K^{b+}H-2K^{d+}) at a dose of 100 mg/kg (Day -1). One day later, splenocytes were collected from human PD-1 knock-in mice (donor, H-2K^{b+}H-2K^{d-}), washed with PBS, and suspended in PBS at 5 × 10⁷ cells /200 µl. This cell suspension was transferred into BDF1 mice in 200 µl via the tail vein (Day 0). After Day 0, anti-human PD-1 agonist antibody was administered intraperitoneally once every 3 to 4 days at a dose of 500 µg/mouse. For evaluating GVHD symptoms, body weights of mice were measured after 4, 7, 11 and 14 days and the percent weight loss from Day 0 was calculated. Further, blood samples were taken after 7 and 14 days. Plasma was collected by centrifugation, and then PBMC was isolated by density gradient centrifugation. After 14 days, mice were euthanized. The spleen and the liver were collected and cell suspensions were prepared. These suspensions were analyzed with a flow cytometer and cell counting was performed. From the results obtained, rates of PBMC and donor cells (H-2K^{b+}H-2K^{d-}) in the spleen or the liver and respective cell counts were calculated. Aspartate aminotransferase (AST) and alanine aminotransferase (ALT) in plasma were measured with Transaminase CII-Test Wako (Fuji Film), and IFN-γ concentration in plasma with Mouse IFN-γ ELISA MAX Deluxe (BioLegend).

### Evaluation of PD-1 Agonist using EAE Model

Wild-type mice were immunized at two subcutaneous sites in the back with 100 µg each of MOG₃₅₋₅₅ peptide (Eurofins) emulsified by mixing with Freund's complete adjuvant. Pertussis toxin (Fuji Film Wako Pure Chemical, 400 ng) was administered to the mice via the tail vein. After 1 day, pertussis toxin (400 ng) was administered again. After 13 days, cells were isolated from the spleen and the axillary lymph node. CD8⁺ cells and Gr-1⁺ cells were removed with AutoMACS. The remaining cells were seeded in flat bottom 12-well plates at 1.05 × 10⁷ cells/well and cultured in the presence of 20 µg/ml MOG peptide and 10 ng/ml IL-23 for 5 days. On the first and second days of culture, cells were rendered to express human PD-1 using retrovirus. After 5 days of culture, cells were harvested and their concentration was adjusted with PBS so that the number of activated CD4⁺ T cells in lymphocytes would be 1 × 10⁶ in 200 µl. The resultant cell sample was transferred into wild-type mice via the tail vein at 200 µl/mouse (Day 0). After Day 0 of cell transfer, anti-human PD-1 agonist antibody was administered intraperitoneally at 500 µg/mouse once every 3 days. Measurement of body weight and scoring were performed at the timing indicated in Fig. 8. Scores were recorded as follows. 0: no symptom, 0.5: paralysis at the tip of the tail, 1.0: paralysis in the tail, 1.5: light paralysis in one lower limb, 2.0: light paralysis in both lower limbs, 2.5: paralysis in one lower limb, 3.0: paralysis in both lower limbs, 3.5: paralysis in four limbs, 4.0: moribundity, 5.0: death.

### Evaluation of PD-1 Agonist using Colitis Model

Cells were prepared from the spleen and the lymph nodes of human PD-1 knock-in mice, and CD25⁻CD4⁺ cells were isolated with a cell sorter. These cells were suspended in PBS at 5 × 10⁵ cells/200 µl and transferred into RAG2 KO mice in 200 µl via the tail vein (Day 0). From Day 0, anti-PD-1 agonist antibody was administered to the mice intraperitoneally once every 3 to 4 days at 500 µg/mouse. Body weight was measured twice a week and the percent weight loss from Day 0 was calculated. At week 8, mice were euthanized, the colon was collected and its length was measured. Further, CD4⁺ T cells infiltrating inherently into the lamina propria of the colon were collected and analyzed with a flow cytometer after staining intracellular cytokines.

### Evaluation of PD-1 Agonist using Chronic GVHD/Lupus-Like Model

Cyclophosphamide (Fuji Film Wako Pure Chemical) dissolved in PBS was intraperitoneally administered to BDF1 mice (recipient, H-2K^{b+}H-2K^{d+}) at a dose of 100 mg/kg (Day -1). After 1 day, splenocytes were collected from human PD-1 knock-in mice (donor, H-2K^{b+}H-2K^{d-}) and freed of CD8⁺ cells using AutoMACS. Subsequently, the resultant cells were washed with PBS, and suspended in PBS at 2 × 10⁷ cells /200 µl. This cell suspension was transferred into BDF1 mice in 200 µl via the tail vein (Day 0). From Day 0, anti-human PD-1 agonist antibody was administered intraperitoneally once every 3 to 4 days at a dose of 500 µg/mouse. For evaluating lupus-like symptoms, blood samples were weekly taken for 11 weeks, and plasma concentration of IL-21 or anti-dsDNA antibody was measured by ELISA. After 7 or 14 days, mice were euthanized and the spleen was collected to prepare cell suspensions. These donor-derived Tfh cells (CXCR5⁺ICOS⁺H-2K^{d-}CD4⁺) or recipient-derived germinal center B cells (CD95⁺GL7⁺H-2K^{d+}B220⁺), class-switched B cells (IgD⁻IgM⁻H-2K^{d+}B220⁺), and plasma cells (CD138⁺H-2K^{d+}CD19⁺) were analyzed with a flow cytometer.

### Evaluation of PD-1 Agonist in Human T Cell / B Cell Mixed Lymphocyte Reaction (MLR)

In mixed lymphocyte reaction of human CD4⁺ T cells (LONZA) and human CD19⁺ B cells (Precision for Medicine) both isolated from PBMC of healthy donors by negative selection, the activity of anti-human PD-1 agonist antibody against T cell's reaction to alloantigen was evaluated. Specifically, human T cells (2 × 10⁵ cells) stained with CFSE were mixed with B cells (1 × 10⁵ cells), and the mixture was seeded in round bottom 96-well plates at 200 µl/well. After culturing for 6 to 7 days, cells were harvested. The harvested cells were freed of B cells by negative selection with CD19 antibody. Then, B cells from the same donor were newly added to re-stimulate the T cells. At the time of this re-stimulation, anti-human PD-1 antibodies or CTLA-4-Ig were added simultaneously to give a final concentration of 5, 0.5, 0.05 or 0.005 µg/ml. After 12 hours, the culture supernatant was collected, and IFN-y concentration was measured with Human IFN-y ELISA MAX Deluxe (BioLegend).

### Evaluation of ADCC Activity

As a target cell, Raji-hPD-1 cells (InvivoGen) were suspended in a medium at 1.1 × 10⁶ cells/ml and seeded in round bottom 96-well plates at 90 µl/well. Simultaneously, anti-human PD-1 antibody was added to the plates at 20 µl/well to give a final concentration of 1000, 300, 100, 30, 10, 3, 1, 0.3, 0.1, 0.03 or 0.01 ng/ml. After one hour culture, Jurkat-Lucia NFAT-CD16 Cells (InvivoGen) were suspended as effector cells in a medium at 2.2 × 10⁶ cells/ml and added thereto at 90 µl/well. After 6-hour culture, 50 µl of QUANTI-Luc solution (InvivoGen) was added to 20 µl of the culture supernatant. Luminescence intensity was measured with a microplate reader.

### Evaluation of PD-1 Agonist in Antigen Response of Human PBMC

Frozen PBMC from healthy donor (Precision for Medicine) were thawed, suspended in a medium at 1 × 10⁷ cells/2 ml, and seeded in 12-well plates at 2 µl/well. After 24-hour preculture, cells were collected, stained with CFSE, suspended in a medium at 3 × 10⁶ cells/ml, and seeded in round bottom 96-well plates at 100 µl/well. As an antigen to this cell, tetanus toxoid (Merck) was added at 50 µl/well to give a final concentration of 1 µg/ml. Further, anti-human PD-1 antibodies or CTLA-4-Ig were added at 50 µl/well to give a final concentration of 5, 0.5, 0.05 or 0.005 µg/ml. After 4-5 days, proliferation of CD4⁺ T cells linked to CFSE as an indicator was evaluated with a flow cytometer. Further, IFN-y concentration in the culture supernatant was measured with Human IFN-y ELISA MAX Deluxe (BioLegend).

### Evaluation of PD-1 Agonist with respect to ERK Phosphorylation

Human PD-1-expressing DO11.10 T cell hybridomas and mouse FcyRIIB-expressing IIA1.6 cells were suspended in a medium (10% fetal bovine serum-containing RPMI1640 medium). DO11.10 T cell hybridomas and IIA1.6 cells were seeded in round bottom 96-well plates at 5 × 10⁴ and 1 × 10⁴ cells/well/50 µl, respectively. Control mouse antibody (MOPC-21) or anti-human PD-1 antibody HM266 was added to the plates at 50 µl/well to give a final concentration of 5 µg/ml. Subsequently, OVA₃₂₃₋₃₃₉ peptide (Eurofins) was added as antigen at 50 µl/well to give a final concentration of 2 µg/ml. Immediately after the addition of OVA (0 hour) or after incubating at a 37°C, 5% CO₂ for 2, 4 or 6 hours, cells were harvested. The resultant cells were fixed with 4% paraformaldehyde for 15 minutes, washed, and permeabilized with 0.1% Triton X-100 for 15 minutes. After washing, the cells were suspended in 50% methanol-PBS and left standing overnight at -20°C. After washing, the cells were stained with Alexa488-anti-phospho-p44/42 MAPK (Erk1/2) (Thr202/Tyr204) mAb, PE-anti-DO11.10 TCR mAb and APC-anti-B220 mAb. Stained cells were analyzed by FACS, and phosphorylated ERK positive ratio in DO11.10 (DO11.10 TCR⁺B220⁻) was calculated.

### Evaluation of PD-1 Agonist by T Cell-Dependent Antibody Response

Co-precipitate of NP-OVA (total protein: 100 µg) and alum adjuvant were administered intraperitoneally to human PD-1 knock-in mice; simultaneously, 500 µg of anti-human PD-1 antibody was administered intraperitoneally (Day 0). The same dose of anti-human PD-1 antibody was further administered intraperitoneally 3 days and 7 days after the first administration. Seven and 10 days after NP-OVA administration, blood samples were collected. Antigen-specific antibody titer and the amount of II,-21 in blood were measured by ELISA. Antibodies (anti-NP2 and anti-NP25) binding to a complex of 2 or 25 molecules of NP binding to one molecule of bovine serum albumin (BSA) were measured by ELISA, and were defined either as high affinity antigen-specific antibody or as low affinity antigen-specific antibody. Further, mice were euthanized 10 days after immunization with antigen, and the spleen was collected to prepare cell suspensions. These Tfh cells (CXCR5⁺ICOS⁺CD4⁺), germinal center B cells (CD95⁺GL7⁺B220⁺), class-switched B cells (IgD⁻IgM⁻B220⁺) and plasma cells (CD138⁺B220⁻) were analyzed with a flow cytometer.

### Evaluation of PD-1 Agonist in Co-Culture System of Activated Human T Cells or Human Tfh-Like Cells and THP-1 Cells

Activated human T cells were prepared as described below. Briefly, human CD4⁺ T cells isolated from PBMC of healthy donors by negative selection (LONZA) were adjusted to 1 × 10⁶ cells/ml, seeded in anti-CD3 antibody (3 µg/ml) immobilizing 24-well plates at 1 ml/well, and stimulated for 3 days. Human Tfh-like cells were prepared as described below. Briefly, naive T cell (CD45RA⁺CXCR5⁻CD11c⁻) fraction was sorted from human CD4⁺ T cells with a cell sorter and adjusted to 1 × 10⁶ cells/ml. 25 µl of pre-washed Dynabeads Human T-activator CD3/28 (Thermo Fisher) per 1 ml of the sorted cells was added. The resultant mixture of cells and beads was seeded at 1 ml/well in 24 well plates. On the following day, cells which had been stimulated with the beads were harvested. After removal of the beads, cells were adjusted to 5 × 10⁵ cells/ml, and seeded at 100 µl/well in anti-CD3 antibody (5 µg/ml) immobilized flat bottom 96-well plates. Subsequently, Human IL-23 (final concentration: 25 ng/ml), Human TGFβ (final concentration: 5 ng/ml), Human IL-12 (final concentration: 1 ng/ml) and anti-human CD28 (final concentration: 1 µg/ml) were added to make 200 µl/well. Then, plates were incubated at a 37°C, 5% CO₂ for 48 to 72 hours. The thus cultured cells were analyzed with FACS, and it was confirmed that more than 50% of the cells became Tfh-like cells (CXCR5⁺ICOS⁺PD-1⁺). THP-1 cell line (parental cell line or a cell line overexpressing human FcgyRIIB) was suspended in dilute solution of mitomycin C (500 µg/ml) at 1 × 10⁷ cells/ml, incubated at 37°C for 2 hours and washed 3 times with a culture medium. The thus prepared activated human T cells or human Tfh-like cells were seeded in round bottom 96-well plates at 5 × 10⁴ cells/well and the THP-1 cells at 2.5 × 10⁴ cells/well. Further, as a stimulant, CytoStim (Miltenyi Biotec) was added at 0.2 µl/well and anti-human PD-1 antibodies or CTLA-4-Ig were added to give a final concentration of 5-5000 ng/ml. Then, plates were cultured at a 37°C, 5% CO₂ for 18 hours. After the culture, the supernatant was collected, and cytokine concentrations were measured using IFN-γ ELISA MAX Deluxe (BioLegend) and Human IL-21 Duoset ELISA (R&D).

### Evaluation of PD-1 Agonist in the Autoantigen Response of PBMC from Autoimmune Disease Patients

RA or SLE patient-derived PBMC (Precision for Medicine, STEMCELL Technologies) was thawed, suspended in a medium at 1 × 10⁷ cells/2 ml and seeded in 12-well plates at 2 ml/well. After 16-24 hour preculture, cells were collected, stained with CFSE, suspended in a medium at 3 × 10⁶ cells/ml and seeded in round bottom 96-well plates at 100 µl/well. As antigens to these cells, citrullinated fibrinogen (Cayman) for RA patient-derived PBMC and Sm antigen (AROTEC DIAGNOSTICS) for SLE patient-derived PBMC were added at 50 µl/well to give final concentrations of 10 µg/ml and 5 µg/ml, respectively. Further, anti-human PD-1 antibodies or CTLA-4-Ig were added at 50 µl/well to give a final concentration of 5, 0.5, 0.05 or 0.005 µg/ml. After 6 to 7 days, proliferation of CD4⁺ T cells linked to CFSE as an indicator was evaluated with a flow cytometer. Further, IFN-y concentration in the culture supernatant was measured with Human IFN-y ELISA MAX Deluxe (BioLegend).

### Results and Discussion

There are anti-PD-1 antibodies which are claimed to suppress T cell activity by PD-1 agonist activity (Ref. 1). However, it has never been demonstrated if any anti-PD-1 antibodies can really stimulate PD-1. Preceding researches discuss T cell inhibition relying on an artificial system based on the effect of immobilized anti-PD-1 antibody on T cell stimulation with immobilized anti-CD3ε antibody. However, such an artificial condition does not faithfully reproduce T cell activation *in vivo.* It is a different question whether the administration of anti-PD-1 antibody in a free form can suppress *in vivo* T cell activation in the interaction with antigen-presenting cells.

To find out anti-human PD-1 agonist antibody that functions under physiological conditions, an ideal evaluation system will be the one reproducing antigen-specific T cell activation by the interaction between T cells and antigen-presenting cells. In addition, a test system that permits easy modification of experimental conditions is useful for the analysis of the mechanism of action. A combination of established cell lines, DO11.10 T cell hybridoma and IIA1.6 cells as antigen-presenting cells, provides a useful platform, which enables antigen-specific T cell activation and the analysis of mechanism of action in an efficient and reproducible manner (Fig. 1). DO11.10 T cell hybridoma is a T cell hybridoma cell line which was originated from CD4⁺ T cells of DO11.10 mouse expressing MHC class II (I-A^{d})-restricted T cell receptor that recognizes an ovalbumin-derived peptide (OVA₃₂₃₋₃₃₉). DO 11.10 T cell hybridoma produces IL-2 in response to the antigenic peptide presented on MHC class II (I-A^{d}) expressed on IIA1.6 cells (a B cell lymphoma cell line) (Fig. 1A). Since antigen-specific T cell activation is inducible in this combination of cells, this experimental system has a substantial advantage of reproducing physiological immune activation scheme, which is not available in experimental systems using non-specific T cell activators. Furthermore, the modification of the cells such as gene knock-out and overexpression can be easily done.

DO11.10 T cell hybridoma cells expressing human PD-1 were confirmed to clearly reduce IL-2 production upon antigen stimulation by PD-L1-expressing IIA1.6 cells in a PD-1-dependent mechanism (Fig. 1B). PD-1 and PD-L1 on the cell membrane should have a native protein conformation, which is also an advantage of this experimental system over artificial experimental systems using water-soluble fusion proteins of PD-1/PD-L1. Indeed, anti-human PD-1 antibody (EH12.2H7), an established blocking antibody, reversed the PD-L1-dependent suppression of IL-2, indicating that this experimental system can reliably detect blocking antibodies (Fig. 1C). The present inventors have concluded that this experimental system using cellular interaction is suitable combination for detecting PD-1-mediated T cell suppression and proceeded to characterize the binding domain of anti-human PD-1 antibodies with the agonist activity and to determine the specific conditions necessary for inducing agonistic activity.

To find and characterize biologically functional anti-human PD-1 antibodies, a number of monoclonal antibodies binding to various domains of human PD-1 were prepared. Hybridomas producing these antibodies were generated from human PD-1-immunized mice. The antibodies were classified by the binding specificity to the eight putative domains constituting the molecular surface of PD-1 that were selected based on the structure of human PD-1 (Fig. 2). The analysis of binding specificity confirmed that the antibody panel included a number of antibodies with various binding epitopes.

Next, the obtained antibodies were tested for the agonistic activity. The detection of immunosuppressive activity by anti-PD-1 agonist antibodies should be conducted in the absence of PD-L1-dependent immunosuppression. The screening system for agonistic antibodies is different from that for blocking antibody. This system involves a combination of human PD-1-expressing DO11.10 T cell hybridoma and IIA1.6 cells which were made to be lacking PD-L1 but expressing FcyRIIB. The present inventors evaluated the anti-human PD-1 antibody panel with a variety of binding epitopes as well as commercially available anti-human PD-1 monoclonal antibodies for biological activities using both screening systems for blocking antibodies and agonist antibodies. As a result, agonist antibodies and blocking antibodies with various degrees of activity were obtained (Figs. 3 to 5). It was confirmed that these activities are not observed in the absence of PD-1. No antibody particularly possessed both agonist activity and blocking activity. Clone HM266, which is one of the strongest agonist antibodies, could suppress ERK phosphorylation occurring downstream in the stimulated DO11.10 cells, indicating the interruption of T cell receptor signaling (Fig. 22).

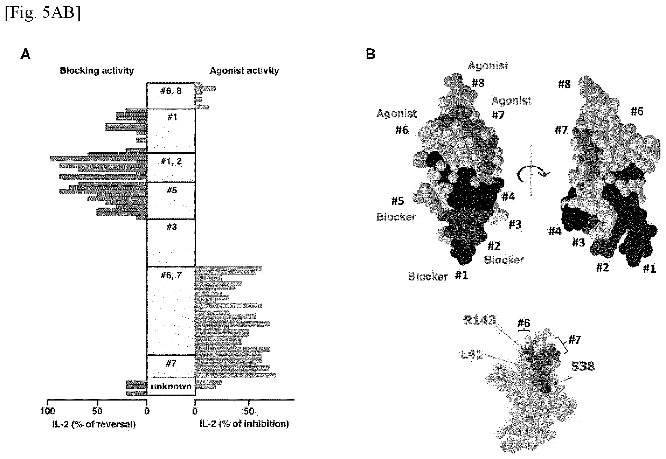

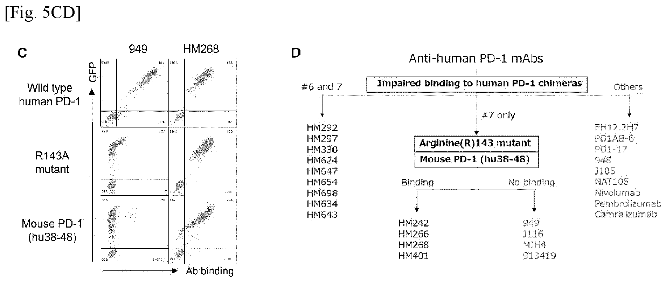

The biological activities were correlated with the binding site of antibodies, and agonist antibodies and blocking antibodies found clearly distinct binding sites (Fig. 5). Specifically, while agonist antibodies bound to domain #6 (SEQ ID NO: 8), domain #7 (SEQ ID NO: 9) or domain #8 (SEQ ID NO: 10) on the surface of human PD-1 molecule, blocking antibodies bound predominantly to domain #1 (SEQ ID NO: 3), domain #2 (SEQ ID NO: 4) or domain #5 (SEQ ID NO: 7). Agonist antibodies specifically binding to domain #7 exhibited strong activity. Included in this group are clones HM266, HM242, HM297 and HM268. The correlation between the agonistic activity and binding sites of human PD-1 molecule has never been reported in defining a group of anti-human PD-1 agonist antibodies.

Since the anti-human PD-1 agonist antibodies were selected in the experimental system involving antigen-specific T cell activation in the interaction with antigen-presenting cells, the immunosuppressive activity by the addition of free anti-human PD-1 antibody indicates its promise to function under physiological conditions. The current agonist antibodies did not affect PD-1-deficient T cells, clearly showing the PD-1-dependent immunosuppressive action. When anti-CD3 antibody was co-immobilized with anti-PD-1 as in the other studies, the immobilized amount of anti-CD3 antibody may substantially decrease due to some distraction by anti-PD-1 antibody used in combination. On such an occasion, apparent activation of T cells is suppressed, but this is caused by attenuation of stimulation from anti-CD3 antibody and what was observed is a non-specific phenomenon irrelevant to the function of anti-PD-1 antibody. Actually, the present inventors have also experienced such cases. To our experience, when this type of experimental system is to be used in PD-1 studies, it will be very important to pay attention to the immobilized amount of anti-CD3 antibody, and PD-1-dependent immunosuppression should be confirmed using PD-1 deficient T cells.

The anti-human PD-1 agonist antibodies shown by the present inventors suppress activated T cells *in vitro,* implying efficacy on a wide variety of inflammatory diseases. Activated T cells have been reported to be involved in various diseases in different organs and allergic diseases by type 1 hypersensitivity (Ref. 2). For example, it is known that the neutralization of activated T cell-derived cytokines decreases inflammatory responses in these disease (Ref. 3). Cancer patients under treatment with anti-PD-1 blocking antibody often experience symptoms similar to diseases with strong T cell involvement as adverse events (Ref. 4). In animal experiments, PD-1 induction is observed in T cell-dependent inflammation (Ref. 5), and PD-1-deficiency or PD-1 blockade result in exaggerated inflammation (Ref. 6), showing that PD-1 is very important for the regulation of activated T cells in pathological conditions. Therefore, therapeutic intervention targeting PD-1 is expected to suppress activated T cells involved in pathological conditions and has a potential to attenuate a wide spectrum of proinflammatory activities in various inflammatory diseases. Next, the present inventors examined the anti-inflammatory action of PD-1 agonist antibodies in multiple mouse inflammation models.

Anti-inflammatory property of PD-1 agonist antibody *in vivo* was tested using an animal experimental model of allergic asthma induced by inhalation of dust mite antigen. Preventive administration of anti-human PD-1 agonist antibody HM266 to human PD-1 knock-in mice reduced infiltration of eosinophils and CD4⁺ T cells into alveolar space and IL-4-, IL-5- and IL-13-producing CD4⁺ T cells (Figs. 6 and 7). Corresponding to the decrease of Th2 cells, the blood concentration of mite antigen-specific IgE also decreased greatly, indicating the suppression of Th2-type immune response (Fig. 7). Importantly, HM266 treatment in therapeutic regimen could also suppress infiltration of eosinophils and Th2-type CD4⁺ T cells (Figs. 6 and 7). These results indicate that T cell suppression by PD-1 agonists is useful as a preventive and a therapeutic strategy for allergic inflammation. Administration of J116, a weaker PD-1 agonist antibody than HM266, also significantly suppressed eosinophils infiltration into the alveolar space. J116 decreased the numbers of IL-5- and IL-13-producing cells, but it did not significantly reduce CD4⁺ T cell infiltration or IL-4-producing cells. (Figs. 24 and 25). Since HM266 has a higher agonist activity than J116 (Fig. 23), the difference in the anti-inflammatory effect might reflect the potential as agonists, and HM266 is one of the excellent clones among the group of antibodies that bind to domain #7 of human PD-1.

The anti-inflammatory efficacy of anti-human PD-1 agonist antibodies was further examined in other T cell-mediated disease models such as multiple sclerosis model (EAE model), colitis model, acute GVHD and chronic GVHD (lupus-like) model. These disease models were induced by T cell transfer, and treatment with anti-human PD-1 agonist antibody started at the same time as T cell transfer. As a result, Encephalomyelitis score in EAE model was significantly reduced by HM266 treatment (Fig. 8). In colitis model, HM266 attenuated body weight loss, shortened colon length and infiltration of cytokine-producing CD4⁺ cells in the lamina propria (Figs. 9 and 26). Additionally, in acute GVHD model, anti-human PD-1 agonist antibodies strongly suppressed acute GVHD symptoms as evidenced from the marked suppression of weight loss, donor-derived cell expansion in peripheral blood and organs and AST, ALT and IFN-y levels in plasma. These anti-inflammatory effects were consistent in anti-human PD-1 agonist antibodies: HM266, HM242, HM268 and HM297 (Figs. 10 and 27). Moreover, anti-human PD-1 agonist antibody blocked the induction of autoantibodies and increases of Tfh cells and antibody-producing B cells involved in autoantibody production in the chronic GVHD (lupus-like) model (Figs. 8-11 and 29). This result indicates the efficacy of anti-human PD-1 agonist antibodies on T cell-dependent antibody production.

The consistent anti-inflammatory action of anti-human PD-1 agonist antibodies in multiple T cell-mediated disease models suggested that these antibodies in present invention are useful as a therapeutic strategy for a wide range of inflammatory diseases. Neutralizing antibodies against T cell-derived cytokines that have already been in clinical use may require continuous treatment in order to sustain anti-inflammatory effects; however, anti-human PD-1 agonist antibody may be able to achieve long-term suppression of T cells and to cure of diseases. Specifically, the remarkable suppression of CD8⁺ T cells in acute GVHD model suggests that anti-human PD-1 agonist antibodies will be effective as therapeutics for GVHD and alopecia areata in which CD8⁺ T cells is mainly involved. Since these antibodies could suppress CD4⁺ T cells in asthma, colitis and EAE models, anti-human PD-1 agonist antibodies will be applicable as therapeutics for allergic disorders, colitis, multiple sclerosis, as well as psoriasis, in all of which CD4⁺ T cells play an important role. The blockade of autoantibodies in the lupus-like model suggests that anti-PD-1 agonist antibodies are applicable as therapeutics for diseases involving autoantibody, as exemplified by lupus nephritis and rheumatoid arthritis.

The present inventors converted the anti-human PD-1 agonist antibodies into humanized antibodies in order to enable application in humans. The seven anti-human PD-1 agonist antibodies that are originally mouse IgG were converted to chimeric antibodies by replacing the sequences of Fc regions to those of human IgG1-K322A. The agonistic activity of the resultant chimeric antibodies was evaluated using human PD-1-expressing cell lines and human FcγRIIB-expressing cell lines, and all the chimeric antibodies showed an activity comparable to that of the original mouse IgG (Fig. 12). Among these seven antibodies, three antibodies with a relatively superior stability were selected. Next, the Fv region of these antibodies were converted to human sequences. For each antibody, 12 or 15 patterns of humanized Fv were created and combined with the constant region of human IgG1-K322A. All the twelve variants derived from HM242 maintained a high PD-1 agonist activity and most of them were also excellent in stability. With respect to 15 variants derived from HM297 and 12 variants from HM268, despite being humanized same as HM242, some of them unexpectedly became inferior to their original form in terms of agonistic activity and stability. Among these humanized antibodies, four humanized antibodies were chosen as antibodies with excellent agonist activity and stability (Fig. 13, Tables 1 to 3).

These humanized antibodies were tested for the immunosuppressive activity in primary human lymphocytes. Human peripheral blood mononuclear cell-derived CD4⁺ T cells were mixed with B cells from other individual, and the extent of allogenic T cell activation was compared. While CTLA-4-Ig inhibited T cell response, none of these four anti-human PD-1 agonist antibodies showed a significant suppressive activity (Fig. 14).

To solve this problem, the present inventors sought to modify the humanized anti-human PD-1 agonist antibodies. Some agonist antibodies require antibody crosslinking to exert their agonistic activity, and Fc receptors play an important role as a means of antibody crosslinking. However, no one has addressed whether Fc receptor-dependent crosslinking is required for the activity of anti-human PD-1 agonist antibodies. In the validation by the present inventors, anti-human PD-1 agonist antibodies failed to exert agonist activity in the absence of Fc receptors. In contrast, in the presence of Fc receptors, anti-human PD-1 agonist antibodies exerted agonistic activity, and FcyRIIB was the most effective for the activity exerted by the anti-human PD-1 agonist antibody among several existing human Fc receptors (Figs. 15 and 16). Although immunosuppressive activity can also be induced by FcyRIIA(H131) or FcyRIIA(R131), the intensity was inferior compared to FcyRIIB, and FcγRIIIA(F158) or FcγRIIIA(V158) was even weaker. Unexpectedly, cytokine production rather increased when FcγRIA-expressing antigen presenting cells were used to attach anti-PD-1 antibodies.

The agonistic activity of humanized anti-human PD-1 agonist antibody increases dependent on the human FcyRIIB levels on the antigen-presenting cells (Fig. 16). A high immunosuppressive activity emerged when antigen-presenting cells express FcyRIIB at high levels, whereas only marginal immunosuppressive activity was observed when FcyRIIB levels were low. In this sense, it suggested that the agonist antibodies in the present invention might be optimized to increase their binding to human FcyRIIB to exert immunosuppression through low levels of human FcyRIIB as found in actual human lymphocytes. This problem, or solution for the problem, could not be found in the test system (antibody immobilization) employed by others in previously reported anti-PD-1 agonist antibodies. This is a finding that guarantees the inventive step of the present invention.

Then, the present inventors sought to increase the affinity of agonist antibodies to human FcγRIIB. Briefly, by introducing amino acid modifications into the Fc region and so on, the inventors have found combinations of modifications which can selectively improve the affinity to FcyRIIB. These modified Fc regions were attached to anti-human PD-1 agonist antibodies to produce chimeric antibodies. The resultant chimeric antibodies demonstrated the enhanced agonistic activities even under conditions of low expression of FcγRIIB, and they showed an improved immunosuppressive activity in primary human cells (Figs. 17, 18, 30, 35 and 37). The humanized antibodies with these Fc modifications were confirmed to have a more potent efficacy than CTLA-4-Ig in the suppression of human T cells activation (Figs. 19 and 38). These results show that Fc-modified anti-human PD-1 agonist antibodies in the present invention are capable of exerting a potent immunosuppressive action in a condition with physiological expression levels of PD-1 and FcγRIIB.

Some of the Fc variants not only promoted PD-1 agonist activity via improved FcγRIIB affinity but also increased affinity to FcγRIIIA, which is responsible for the FcγRIIIA-mediated ADCC activity (Figs. 20, 31, 36 and 39). These antibodies, compared to those having low affinity to FcγRIIIA, showed higher T cell-suppressive activity in the assay system in which human PBMC was stimulated with antigen (Figs. 21, 32 and 40). The overall immunosuppressive activity of anti-PD-1 agonist antibodies may be further improved by the addition of ADCC activity eliminating PD-1-expressing cells.

So far, for cell surface molecules such as TNF receptor superfamily, some antibodies targeting these cell surface molecules were known to exert agonistic activityin an FcγRIIB-dependent manner (Ref. 7). These agonist antibodies trigger the signal transduction through TRAFs (TNF receptor-associated factors), it is downstream of target molecules, and so on as a result of the binding to FcyRIIB and the crosslinking of target molecules (Ref. 8). Conversely, PD-1 has an immunoinhibitory domain (ITIM/ITSM) in its intracellular domain, recruits phosphatase SHP-1/2, and dephosphorylates various immune activation signaling molecules, thereby causing immunosuppression (Ref. 9). The agonistic signal transduction by the anti-human PD-1 antibody related to the present invention is completely different from signal transduction by known antibodies in terms of the molecular species and the intracellular signaling mode. Indeed, the FcyRIIB-dependency of the agonist antibodies to the molecules of the immunoglobulin superfamily, to which the anti-PD-1 agonist antibody belongs has never been reported before, and it is unpredictable that anti-human PD-1 agonist antibodies in the present invention show the same characters as known antibodies as above. Furthermore, it is also unpredictable that the agonistic activity of anti-human PD-1 antibodies emerges only when the antibody has bound to the specific domain of human PD-1.

Anti-CD40 antibody, one of the agonist antibodies to the above-described TNF receptor family, has proceeded to clinical trial; however, the therapeutic outcome was not as remarkable as the efficacy shown in mouse models (Ref. 10). In this report, they speculate that Fc modification to increase the binding of the antibodies to FcyRIIB may improve the clinical response in humans. However, with respect to the anti-PD-1 agonist antibody, the dependence on FcyRIIBhas not previously reported in the first place and the necessity of high affinity of anti-PD-1 agonist antibody to FcyRIIB has never been known at all. The novel ideas by the present inventors of enhancing the affinity of anti-human PD-1 agonist antibodies for FcyRIIB enables the antibodies in the present invention to exert adequate PD-1 agonistic activity and achieve immunosuppressive treatment of inflammatory diseases even in the human physiological condition with low FcyRIIB expression level. Further, it has not known before that the Fc modification to enhance ADCC activity further improves the immunosuppressive effect of anti-human PD-1 agonist antibodies. The enhancement of PD-1 agonist activity, together with ADCC activity, by the modification of Fc region produce potent immunosuppressive anti-human PD-1 antibodies compared to pre-existing anti-human PD-1 antibodies, thus showing the inventive step of the antibody of the present invention.

**[Table 1]**

| Activity and Property of Humanized HM242 | | | | | | | | |
|---|---|---|---|---|---|---|---|---|
| Activity and Property of Humanized HM242 | | | | | | | | |
| **VH** | **VL** | **Sample** | **Productivity (mg/l)** | **Monomer ratio(%) _Gel filtration** | **Cell-cell contact assay (ng/ml)** | **SPR(PD-1) K_{D} (M)** | **DSC Tm2 (°C)_Fab** | **Identical with human sequence(%)** |
| mouse | | HM242-IgG1-K322A | n.d. | 78.3 | 13.7 | <5.105E-11 | 78.9 | 91% |
| A | 1 | HM242-A1 | 352 | 88.5 | 13.1 | <3.561E-11 | 82.5 | 94% |
| | 2 | HM242-A2 | 97 | 93.4 | 16.7 | <4.080E-11 | 87.2 | 95% |
| | 3 | HM242-A3 | 252 | 95.1 | 18.8 | <2.172E-11 | 90.2 | 96% |
| B | 1 | HM242-B1 | 216 | 91.4 | 7.15 | <4.482E-11 | 82.2 | 95% |
| | 2 | HM242-B2 | 91 | 100.0 | 8.83 | <3.153E-11 | 85.9 | 96% |
| | 3 | HM242-B3 | 141 | 100.0 | 20.8 | <3.072E-11 | 87.1 | 97% |
| C | 1 | HM242-C1 | 485 | 94.5 | 19.0 | <1.786E-11 | 82.7 | 95% |
| | 2 | HM242-C2 | 187 | 73.9 | 18.1 | <2.107E-11 | 88.3 | 96% |
| | 3 | HM242-C3 | 409 | 96.0 | 23.7 | <1.861E-11 | 91.8 | 97% |
| D | 1 | HM242-D1 | 485 | 95.4 | 24.8 | 1.03E-10 | 82.1 | 95% |
| | 2 | HM242-D2 | 158 | 86.8 | 33.3 | <6.031E-11 | 87.7 | 96% |
| | 3 | HM242-D3 | 368 | 95.7 | 31.5 | <4.924E-11 | 92.4 | 97% |

| | | | | | | | | |
|---|---|---|---|---|---|---|---|---|
| (n.d. = not determined) | | | | | | | | |

**[Table 2]**

| Activity and Property of Humanized HM297 | | | | | | | | |
|---|---|---|---|---|---|---|---|---|
| Activity and Property of Humanized HM297 | | | | | | | | |
| **VH** | **VL** | **Sample** | **Productivity (mg/l)** | **Monomer ratio(%) _Gel filtration** | **Cell-cell contact assay (ng/ml)** | **SPR(PD-1) K_{D} (M)** | **DSC Tm2 (°C)_Fab** | **Identical with human sequence(%)** |
| mouse | | HM297-IgG1-K322A | n.d. | 95.7 | 78.3 | 4.932E-11 | 78.21 | 92% |
| E | 4 | HM297-E4 | 311 | 85.70 | 100 | 1.293E-10 | 80.18 | 94% |
| | 5 | HM297-E5 | 614 | 94.12 | 78.6 | 5.022E-08 | 83.35 | 94% |
| | 6 | HM297-E6 | 508 | 94.02 | 65.4 | 1.259E-08 | 84.47 | 95% |
| F | 4 | HM297-F4 | 736 | 87.84 | 92.3 | 2.412E-08 | 80.82 | 94% |
| | 5 | HM297-F5 | 555 | 85.39 | 98.9 | 2.349E-09 | 85.40 | 95% |
| | 6 | HM297-F6 | 447 | 84.72 | 150 | 4.825E-09 | 86.45 | 96% |
| G | 4 | HM297-G4 | 449 | 84.23 | 55.4 | 4.507E-10 | 80.39 | 94% |
| | 5 | HM297-G5 | 276 | 85.78 | 102 | 2.319E-10 | 82.62 | 95% |
| | 6 | HM297-G6 | 350 | 79.17 | 59.7 | 1.892E-09 | 82.61 | 96% |
| H | 4 | HM297-H4 | 740 | 89.90 | 185 | 2.206E-10 | 80.99 | 95% |
| | 5 | HM297-H5 | 728 | 90.72 | 96.4 | 5.630E-10 | 84.98 | 95% |
| | 6 | HM297-H6 | 757 | 86.03 | 141 | 1.860E-09 | 85.71 | 96% |
| I | 4 | HM297-I4 | 686 | 90.07 | 65.6 | 4.844E-09 | 82.61 | 95% |
| | 5 | HM297-I5 | 538 | 90.11 | 94.9 | 2.200E-09 | 86.13 | 95% |
| | 6 | HM297-I6 | 558 | 88.57 | 137 | 3.479E-09 | 86.83 | 96% |

| | | | | | | | | |
|---|---|---|---|---|---|---|---|---|
| (n.d. = not determined) | | | | | | | | |

**[Table 3]**

| Activity and Property of Humanized HM268 | | | | | | | | |
|---|---|---|---|---|---|---|---|---|
| | | | | | | | | |
| **VH** | **VL** | **Sample** | **Productivity (mg/l)** | **Monomer ratio(%) _Gel filtration** | **Cell-cell contact assay (ng/ml)** | **SPR(PD-1) K_{D} (M)** | **DSC Tm2 (°C)_Fab** | **Identical with human sequence(%)** |
| mouse | | HM268-IgG1-K322A | n.d. | 94.9 | 46.5 | <1.694E-12 | 70.4 | 91% |
| J | 7 | HM268-J7 | 332 | 100.0 | 70.9 | 4.816E-12 | 80.4 | 95% |
| | 8 | HM268-J8 | 639 | 99.1 | 63.1 | <2.837E-12 | 82.5 | 96% |
| | 9 | HM268-J9 | 201 | 97.3 | 301 | 2.524E-11 | 81.4 | 97% |
| K | 7 | HM268-K7 | 473 | 76.4 | 43.7 | 6.906E-12 | 80.6 | 95% |
| | 8 | HM268-K8 | 905 | 91.8 | 39.4 | <3.062E-12 | 86.9 | 96% |
| | 9 | HM268-K9 | 366 | 65.3 | 150 | 1.511E-10 | 87.5 | 97% |
| L | 7 | HM268-L7 | 369 | 98.0 | 103 | 2.161E-12 | 82.0 | 96% |
| | 8 | HM268-L8 | 701 | 99.1 | 98.2 | 1.291E-12 | 87.2 | 96% |
| | 9 | HM268-L9 | 612 | 87.1 | 87.4 | 1.105E-11 | 87.1 | 97% |
| M | 7 | HM268-M7 | 401 | 83.7 | 149 | 1.199E-11 | 82.5 | 96% |
| | 8 | HM268-M8 | 521 | 93.0 | 75.8 | 3.588E-12 | 87.4 | 97% |
| | 9 | HM268-M9 | 295 | 66.5 | 1.43E+03 | 6.143E-11 | 88.3 | 97% |

| | | | | | | | | |
|---|---|---|---|---|---|---|---|---|
| (n.d. = not determined) | | | | | | | | |

### References

### Ref. 1

- Japanese Patent No. 6174321
- WO 2017/058859
- WO 2018/053405
- WO 2018/183459
- Japanese Patent No. 4511943
- WO 2019/168745
- WO 2018/226580
- WO 2019/156199
- WO 2019/219709
- WO 2020/247648
- Bennett F, Luxenberg D, Ling V, Wang IM, Marquette K, Lowe D, Khan N, Veldman G, Jacobs KA, Valge-Archer VE, Collins M, Carreno BM. Program Death-1 Engagement Upon TCR Activation Has Distinct Effects on Costimulation and Cytokine-Driven Proliferation: Attenuation of ICOS, IL-4, and IL-21, But Not CD28, IL-7, and IL-15 Responses. J Immunol. 170:711-718 (2003).

### Ref. 2

- Hirahara K, Nakayama T. CD4+ T-cell subsets in inflammatory diseases: beyond the Th1/Th2 paradigm. Int Immunol. 28:163-171 (2016).
- Noda S, Krueger JG, Guttman-Yassky E. The translational revolution and use of biologics in patients with inflammatory skin diseases. J Allergy Clin Immunol. 135:324-336 (2015).

### Ref. 3

- Simpson EL, Bieber T, Guttman-Yassky E, Beck LA, Blauvelt A, Cork MJ, Silverberg JI, Deleuran M, Kataoka Y, Lacour JP, Kingo K, Worm M, Poulin Y, Wollenberg A, Soo Y, Graham NM, Pirozzi G, Akinlade B, Staudinger H, Mastey V, Eckert L, Gadkari A, Stahl N, Yancopoulos GD, Ardeleanu M; SOLO 1 and SOLO 2 Investigators. Two Phase 3 Trials of Dupilumab versus Placebo in Atopic Dermatitis. N Engl J Med. 375:2335-2348 (2016).
- Langley RG, Elewski BE, Lebwohl M, Reich K, Griffiths CE, Papp K, Puig L, Nakagawa H, Spelman L, Sigurgeirsson B, Rivas E, Tsai TF, Wasel N, Tyring S, Salko T, Hampele I, Notter M, Karpov A, Helou S, Papavassilis C; ERASURE Study Group; FIXTURE Study Group. Secukinumab in plaque psoriasis--results of two phase 3 trials. N Engl J Med. 371:326-38 (2014).

### Ref. 4

- Rosskopf S, Jahn-Schmid B, Schmetterer KG, Zlabinger GJ, Steinberger P. PD-1 has a unique capacity to inhibit allergen-specific human CD4+ T cell responses. Sci Rep. 8:13543 (2018).
- Bottlaender L, Amini-Adle M, Maucort-Boulch D, Robinson P, Thomas L, Dalle S. Cutaneous adverse events: a predictor of tumour response under anti-PD-1 therapy for metastatic melanoma, a cohort analysis of 189 patients. J Eur Acad Dermatol Venereol. 34:2096-2105 (2020).

### Ref. 5

- Liang SC, Latchman YE, Buhlmann JE, Tomczak MF, Horwitz BH, Freeman GJ, Sharpe AH. Regulation of PD-1, PD-L1, and PD-L2 expression during normal and autoimmune responses. Eur J Immunol. 33:2706-16 (2003).

### Ref. 6

- Nishimura H, Nose M, Hiai H, Minato N, Honjo T. Development of lupus-like autoimmune diseases by disruption of the PD-1 gene encoding an ITIM motif-carrying immunoreceptor. Immunity. 11:141-51 (1999).
- Wang J, Yoshida T, Nakaki F, Hiai H, Okazaki T, Honjo T. Establishment of NOD-Pdcd1/ mice as an efficient animal model of type I diabetes. Proc. Natl Acad. Sci. USA. 102:11823-8 (2005).
- Ansari MJ, Salama AD, Chitnis T, Smith RN, Yagita H, Akiba H, Yamazaki T, Azuma M, Iwai H, Khoury SJ, Auchincloss H Jr, Sayegh MH. The programmed death-1 (PD-1) pathway regulates autoimmune diabetes in nonobese diabetic (NOD) mice. J Exp Med. 198:63-9 (2003).
- Nishimura H, Okazaki T, Tanaka Y, Nakatani K, Hara M, Matsumori A, Sasayama S, Mizoguchi A, Hiai H, Minato N, Honjo T. Autoimmune dilated cardiomyopathy in PD-1 receptor-deficient mice. Science. 291:319-22 (2001).
- Okazaki T, Tanaka Y, Nishio R, Mitsuiye T, Mizoguchi A, Wang J, Ishida M, Hiai H, Matsumori A, Minato N, Honjo T. Autoantibodies against cardiac troponin I are responsible for dilated cardiomyopathy in PD-1-deficient mice. Nat Med. 9:1477-83 (2003).
- Okazaki T, Honjo T. Pathogenic roles of cardiac autoantibodies in dilated cardiomyopathy. Trends Mol Med. 11:322-6 (2005).
- Tsushima F, Iwai H, Otsuki N, Abe M, Hirose S, Yamazaki T, Akiba H, Yagita H, Takahashi Y, Omura K, Okumura K, Azuma M. Preferential contribution of B7-H1 to programmed death-1-mediated regulation of hapten-specific allergic inflammatory responses. Eur J Immunol. 33:2773-82 (2003).
- Tanaka R, Ichimura Y, Kubota N, Saito A, Nakamura Y, Ishitsuka Y, Watanabe R, Fujisawa Y, Kanzaki M, Mizuno S, Takahashi S, Fujimoto M, Okiyama N. Activation of CD8 T cells accelerates anti-PD-1 antibody-induced psoriasis-like dermatitis through IL-6. Commun Biol. 3:571 (2020).

### Ref. 7

- Li F, Ravetch JV. Inhibitory Fcγ receptor engagement drives adjuvant and anti-tumor activities of agonistic CD40 antibodies. Science. 333:1030-4 (2011).
- White AL, Chan HT, Roghanian A, French RR, Mockridge CI, Tutt AL, Dixon SV, Ajona D, Verbeek JS, Al-Shamkhani A, Cragg MS, Beers SA, Glennie MJ. Interaction with FcyRIIB is critical for the agonistic activity of anti-CD40 monoclonal antibody. J Immunol. 187:1754-63 (2011).
- Wilson NS, Yang B, Yang A, Loeser S, Marsters S, Lawrence D, Li Y, Pitti R, Totpal K, Yee S, Ross S, Vernes JM, Lu Y, Adams C, Offringa R, Kelley B, Hymowitz S, Daniel D, Meng G, Ashkenazi A. An Fcγ receptor-dependent mechanism drives antibody-mediated target-receptor signaling in cancer cells. Cancer Cell. 19:101-13 (2011).
- Li F, Ravetch JV. Apoptotic and antitumor activity of death receptor antibodies require inhibitory Fcγ receptor engagement. Proc Natl Acad Sci U S A. 109:10966-71 (2012).

### Ref. 8

- Elgueta R, Benson MJ, de Vries VC, Wasiuk A, Guo Y, Noelle RJ. Molecular mechanism and function of CD40/CD40L engagement in the immune system. Immunol Rev. 229:152-72 (2009).

### Ref. 9

- Okazaki T, Maeda A, Nishimura H, Kurosaki T, Honjo T. PD-1 immunoreceptor inhibits B cell receptor-mediated signaling by recruiting src homology 2-domain-containing tyrosine phosphatase 2 to phosphotyrosine. Proc Natl Acad Sci U S A. 98:13866-13871(2001).
- Sheppard KA, Fitz LJ, Lee JM, Benander C, George JA, Wooters J, Qiu Y, Jussif JM, Carter LL, Wood CR, Chaudhary D. PD-1 inhibits T-cell receptor induced phosphorylation of the ZAP70/CD3zeta signalosome and downstream signaling to PKCtheta. FEBS Lett. 574:37-41 (2004).
- Yokosuka T, Takamatsu M, Kobayashi-Imanishi W, Hashimoto-Tane A, Azuma M, Saito T. Programmed cell death 1 forms negative costimulatory microclusters that directly inhibit T cell receptor signaling by recruiting phosphatase SHP2. J Exp Med. 209:1201-1217 (2012).

### Ref. 10

- Dahan R, Barnhart BC, Li F, Yamniuk AP, Korman AJ, Ravetch JV. Therapeutic activity of agonistic, human anti-CD40 monoclonal Abs requires selective FcyR-engagement. Cancer Cell. 29(6): 820-831 (2016).

All publications, patents and patent applications cited herein are incorporated herein by reference in their entirety.

### INDUSTRIAL APPLICABILITY

The present invention is applicable to treatment and/or prevention of inflammatory diseases.

### SEQUENCE LISTING FREE TEXT

| **Seq. ID** | **Seq. name** |
|---|---|
| **1** | humam PD-1 Amino Acid Sequence |
| **2** | mouse PD-1 Amino Acid Sequence |
| **3** | epitope / binding Region #1 of humam PD-1 |
| **4** | epitope / binding Region #2 of humam PD-1 |
| **5** | epitope / binding Region #3 of humam PD-1 |
| **6** | epitope / binding Region #4 of humam PD-1 |
| **7** | epitope / binding Region #5 of humam PD-1 |
| **8** | epitope / binding Region #6 of humam PD-1 |
| **9** | epitope / binding Region #7 of humam PD-1 |
| **10** | epitope / binding Region #8 of humam PD-1 |
| **11** | human FcgRIA Amino Acid Sequence |
| **12** | human FcgRIIA(H131) Amino Acid Sequence |
| **13** | human FcgRIIA(R131) Amino Acid Sequence |
| **14** | human FcgRIIB Amino Acid Sequence |
| **15** | human FcgRIIIA(F158) Amino Acid Sequence |
| **16** | human FcgRIIIA(V158) Amino Acid Sequence |
| **17** | mouse HM242 VH Region Amino Acid Sequence |
| **18** | humanized VH verA (HM242) Amino Acid Sequence |
| **19** | humanized VH ver.B (HM242) Amino Acid Sequence |
| **20** | humanized VH ver.C (HM242) Amino Acid Sequence |
| **21** | humanized VH ver.D (HM242) Amino Acid Sequence |
| **22** | mouse HM297 VH Region Amino Acid Sequence |
| **23** | humanized VH ver.E (HM297) Amino Acid Sequence |
| **24** | humanized VH ver.F (HM297) Amino Acid Sequence |
| **25** | humanized VH ver.G (HM297) Amino Acid Sequence |
| **26** | humanized VH ver.H (HM297) Amino Acid Sequence |
| **27** | humanized VH ver.I (HM297) Amino Acid Sequence |
| **28** | mouse HM268 VH Region Amino Acid Sequence |
| **29** | humanized VH ver.J (HM268) Amino Acid Sequence |
| **30** | humanized VH ver.K (HM268) Amino Acid Sequence |
| **31** | humanized VH ver.L (HM268) Amino Acid Sequence |
| **32** | humanized VH ver.M (HM268) Amino Acid Sequence |
| **33** | mouse HM266 VH Region Amino Acid Sequence |
| **34** | mouse HM242 VL Region Amino Acid Sequence |
| **35** | humanized VL ver.1 (HM242) Amino Acid Sequence |
| **36** | humanized VL ver.2 (HM242) Amino Acid Sequence |
| **37** | humanized VL ver.3 (HM242) Amino Acid Sequence |
| **38** | mouse HM297 VL Region Amino Acid Sequence |
| **39** | humanized VL ver.4 (HM297) Amino Acid Sequence |
| **40** | humanized VL ver.5 (HM297) Amino Acid Sequence |
| **41** | humanized VL ver.6 (HM297) Amino Acid Sequence |
| **42** | mouse HM268 VL Region Amino Acid Sequence |
| **43** | humanized VL ver.7 (HM268) Amino Acid Sequence |
| **44** | humanized VL ver.8 (HM268) Amino Acid Sequence |
| **45** | humanized VL ver.9 (HM268) Amino Acid Sequence |
| **46** | mouse HM266 VL Region Amino Acid Sequence |
| **47** | human IgG1 WT Fc Region Amino Acid Sequence |
| **48** | human IgG1 Fc_K322A Amino Acid Sequence |
| **49** | human IgG1 Fc_G236D/H268D(X2) Amino Acid Sequence |
| **50** | human IgG1 Fc_G236D/H268D/K322A(X2-K) Amino Acid Sequence |
| **51** | human IgG1 Fc_G236D/H268D/E293A/K322A(X2-KE) Amino Acid Sequence |
| **52** | human IgG1 Fc_S236D/S267G/H268D/K322A(X2-KS) Amino Acid Sequence |
| **53** | human IgG1 Fc_S239D/H268D(X3) Amino Acid Sequence |
| **54** | human IgG1 Fc_S239D/H268D/K322A(X3-K) Amino Acid Sequence |
| **55** | human IgG1 Fc_S239D/H268D/E293A/K322A(X3-KE) Amino Acid Sequence |
| **56** | human IgG1 Fc_S239D/H268D/K322A/I332E(X3-KI) Amino Acid Sequence |
| **57** | human IgG1 Fc_S239D/H268D/L328Y/K322A(X3-KL) Amino Acid Sequence |
| **58** | human IgG1 Fc_S239D/S267G/H268D/K322A(X3-KS) Amino Acid Sequence |
| **59** | human IgG1 Fc_S239D/S267G/H268D/K322A/I332E(X3-KSI) Amino Acid Sequence |
| **60** | human IgG1 Fc_S239D/S267G/H268D/L328Y/K322A(X3-KSL) Amino Acid Sequence |
| **61** | human IgG1 Fc_S239D/H268D/L328Y/I332E(X4) Amino Acid Sequence |
| **62** | human IgG1 Fc_S239D/H268D/K322A/L328Y/I332E(X4-K) Amino Acid Sequence |
| **63** | human IgG1 Fc_S239D/S267G/H268D/K322A/L328Y/I332E(X4-KS) Amino Acid Sequence |
| **64** | human IgG4 Fc Amino Acid Sequence |
| **65** | human IgG4 Fc_S228P Amino Acid Sequence |
| **66** | human IgG4 Fc_S228P/G236D/Q268D(X2-P) Amino Acid Sequence |
| **67** | human IgG4 Fc_S228P/S239D/Q268D(X3-P) Amino Acid Sequence |
| **68** | human IgG4 Fc_S228P/S239D/Q268D/L328Y/I332E(X4-P) Amino Acid Sequence |
| **69** | mouse HM242 Heavy Chain Amino Acid Sequence |
| **70** | mouse HM242 Heavy Chain DNA Sequence |
| **71** | mouse HM242 Light Chain Amino Acid Sequence |
| **72** | mouse HM242 Light Chain DNA Sequence |
| **73** | mouse HM266 Heavy Chain Amino Acid Sequence |
| **74** | mouse HM266 Heavy Chain DNA Sequence |
| **75** | mouse HM266 Light Chain Amino Acid Sequence |
| **76** | mouse HM266 Light Chain DNA Sequence |
| **77** | mouse HM268 Heavy Chain Amino Acid Sequence |
| **78** | mouse HM268 Heavy Chain DNA Sequence |
| **79** | mouse HM268 Light Chain Amino Acid Sequence |
| **80** | mouse HM268 Light Chain DNA Sequence |
| **81** | mouse HM297 Heavy Chain Amino Acid Sequence |
| **82** | mouse HM297 Heavy Chain DNA Sequence |
| **83** | mouse HM297 Light Chain Amino Acid Sequence |
| **84** | mouse HM297 Light Chain DNA Sequence |
| **85** | chimeric HM242-IgG1-K322A Heavy Chain Amino Acid Sequence |
| **86** | chimeric HM242-IgG1-K322A Heavy Chain DNA Sequence |
| **87** | chimeric HM242-IgG1-K322A Light Chain Amino Acid Sequence |
| **88** | chimeric HM242-IgG1-K322A Light Chain DNA Sequence |
| **89** | chimeric HM266-IgG1-K322A Heavy Chain Amino Acid Sequence |
| **90** | chimeric HM266-IgG1-K322A Heavy Chain DNA Sequence |
| **91** | chimeric HM266-IgG1-K322A Light Chain Amino Acid Sequence |
| **92** | chimeric HM266-IgG1-K322A Light Chain DNA Sequence |
| **93** | chimeric HM268-IgG1-K322A Heavy Chain Amino Acid Sequence |
| **94** | chimeric HM268-IgG1-K322A Heavy Chain DNA Sequence |
| **95** | chimeric HM268-IgG1-K322A Light Chain Amino Acid Sequence |
| **96** | chimeric HM2fi8-IgG1-K322A Light Chain DNA Sequence |
| **97** | chimeric HM297-IgG1-K322A Heavy Chain Amino Acid Sequence |
| **98** | chimeric HM297-IgG1-K322A Heavy Chain DNA Sequence |
| **99** | chimeric HM297-IgG1-K322A Light Chain Amino Acid Sequence |
| **100** | chimeric HM297-IgG1-K322A Light Chain DNA Sequence |
| **101** | chimeric HM624-IgG1-K322A Heavy Chain Amino Acid Sequence |
| **102** | chimeric HM624-IgG1-K322A Heavy Chain DNA Sequence |
| **103** | chimeric HM624-IgG1-K322A Light Chain Amino Acid Sequence |
| **104** | chimeric HM624-IgG1-K322A Light Chain DNA Sequence |
| **105** | chimeric HM647-IgG1-K322A Heavy Chain Amino Acid Sequence |
| **106** | chimeric HM647-IgG1-K322A Heavy Chain DNA Sequence |
| **107** | chimeric HM647-IgG1-K322A Light Chain Amino Acid Sequence |
| **108** | chimeric HM647-IgG1-K322A Light Chain DNA Sequence |
| **109** | chimeric HM698-IgG1-K322A Heavy Chain Amino Acid Sequence |
| **110** | chimeric HM698-IgG1-K322A Heavy Chain DNA Sequence |
| **111** | chimeric HM698-IgG1-K322A Light Chain Amino Acid Sequence |
| **112** | chimeric HM698-IgG1-K322A Light Chain DNA Sequence |
| **113** | chimeric HM266-X2 Heavy Chain Amino Acid Sequence |
| **114** | chimeric HM266-X2 Heavy Chain DNA Sequence |
| **115** | chimeric HM266-X2 Light Chain Amino Acid Sequence |
| **116** | chimeric HM266-X2 Light Chain DNA Sequence |
| **117** | chimeric HM266-X2-K Heavy Chain Amino Acid Sequence |
| **118** | chimeric HM266-X2-K Heavy Chain DNA Sequence |
| **119** | chimeric HM266-X2-K Light Chain Amino Acid Sequence |
| **120** | chimeric HM266-X2-K Light Chain DNA Sequence |
| **121** | chimeric HM266-X2-KE Heavy Chain Amino Acid Sequence |
| **122** | chimeric HM266-X2-KE Heavy Chain DNA Sequence |
| **123** | chimeric HM266-X2-KE Light Chain Amino Acid Sequence |
| **124** | chimeric HM266-X2-KE Light Chain DNA Sequence |
| **125** | chimeric HM266-X2-KS Heavy Chain Amino Acid Sequence |
| **126** | chimeric HM266-X2-KS Heavy Chain DNA Sequence |
| **127** | chimeric HM266-X2-KS Light Chain Amino Acid Sequence |
| **128** | chimeric HM266-X2-KS Light Chain DNA Sequence |
| **129** | chimeric HM266-X3 Heavy Chain Amino Acid Sequence |
| **130** | chimeric HM266-X3 Heavy Chain DNA Sequence |
| **131** | chimeric HM266-X3 Light Chain Amino Acid Sequence |
| **132** | chimeric HM266-X3 Light Chain DNA Sequence |
| **133** | chimeric HM266-X3-K Heavy Chain Amino Acid Sequence |
| **134** | chimeric HM266-X3-K Heavy Chain DNA Sequence |
| **135** | chimeric HM266-X3-K Light Chain Amino Acid Sequence |
| **136** | chimeric HM266-X3-K Light Chain DNA Sequence |
| **137** | chimeric HM266-X3-KE Heavy Chain Amino Acid Sequence |
| **138** | chimeric HM266-X3-KE Heavy Chain DNA Sequence |
| **139** | chimeric HM266-X3-KE Light Chain Amino Acid Sequence |
| **140** | chimeric HM266-X3-KE Light Chain DNA Sequence |
| **141** | chimeric HM266-X3-KS Heavy Chain Amino Acid Sequence |
| **142** | chimeric HM266-X3-KS Heavy Chain DNA Sequence |
| **143** | chimeric HM266-X3-KS Light Chain Amino Acid Sequence |
| **144** | chimeric HM266-X3-KS Light Chain DNA Sequence |
| **145** | chimeric HM266-X4 Heavy Chain Amino Acid Sequence |
| **146** | chimeric HM266-X4 Heavy Chain DNA Sequence |
| **147** | chimeric HM266-X4 Light Chain Amino Acid Sequence |
| **148** | chimeric HM266-X4 Light Chain DNA Sequence |
| **149** | chimeric HM266-X4-K Heavy Chain Amino Acid Sequence |
| **150** | chimeric HM266-X4-K Heavy Chain DNA Sequence |
| **151** | chimeric HM266-X4-K Light Chain Amino Acid Sequence |
| **152** | chimeric HM266-X4-K Light Chain DNA Sequence |
| **153** | chimeric HM266-IgG4-S228P Heavy Chain Amino Acid Sequence |
| **154** | chimeric HM266-IgG4-S228P Heavy Chain DNA Sequence |
| **155** | chimeric HM266-IgG4-S228P Light Chain Amino Acid Sequence |
| **156** | chimeric HM266-IgG4-S228P Light Chain DNA Sequence |
| **157** | chimeric HM266-X2-P Heavy Chain Amino Acid Sequence |
| **158** | chimeric HM266-X2-P Heavy Chain DNA Sequence |
| **159** | chimeric HM266-X2-P Light Chain Amino Acid Sequence |
| **160** | chimeric HM266-X2-P Light Chain DNA Sequence |
| **161** | chimeric HM266-X3-P Heavy Chain Amino Acid Sequence |
| **162** | chimeric HM266-X3-P Heavy Chain DNA Sequence |
| **163** | chimeric HM266-X3-P Light Chain Amino Acid Sequence |
| **164** | chimeric HM266-X3-P Light Chain DNA Sequence |
| **165** | chimeric HM266-X4-P Heavy Chain Amino Acid Sequence |
| **166** | chimeric HM266-X4-P Heavy Chain DNA Sequence |
| **167** | chimeric HM266-X4-P Light Chain Amino Acid Sequence |
| **168** | chimeric HM266-X4-P Light Chain DNA Sequence |
| **169** | humanized HM242-C3-IgG1-K322A Heavy Chain Amino Acid Sequence |
| **170** | humanized HM242-C3-IgG1-K322A Heavy Chain DNA Sequence |
| **171** | humanized HM242-C3-IgG1-K322A Light Chain Amino Acid Sequence |
| **172** | humanized HM242-C3-IgG1-K322A Light Chain DNA Sequence |
| **173** | humanized HM242-D3-IgG1-K322A Heavy Chain Amino Acid Sequence |
| **174** | humanized HM242-D3-IgG1-K322A Heavy Chain DNA Sequence |
| **175** | humanized HM242-D3-IgG1-K322A Light Chain Amino Acid Sequence |
| **176** | humanized HM242-D3-IgG1-K322A Light Chain DNA Sequence |
| **177** | humanized HM268-K8-IgG1-K322A Heavy Chain Amino Acid Sequence |
| **178** | humanized HM268-K8-IgG1-K322A Heavy Chain DNA Sequence |
| **179** | humanized HM268-K8-IgG1-K322A Light Chain Amino Acid Sequence |
| **180** | humanized HM268-K8-IgG1-K322A Light Chain DNA Sequence |
| **181** | humanized HM297-H5-IgG1-K322A Heavy Chain Amino Acid Sequence |
| **182** | humanized HM297-H5-IgG1-K322A Heavy Chain DNA Sequence |
| **183** | humanized HM297-H5-IgG1-K322A Light Chain Amino Acid Sequence |
| **184** | humanized HM297-H5-IgG1-K322A Light Chain DNA Sequence |
| **185** | humanized HM242-C3-X2-K Heavy Chain Amino Acid Sequence |
| **186** | humanized HM242-C3-X2-K Heavy Chain DNA Sequence |
| **187** | humanized HM242-C3-X2-K Light Chain Amino Acid Sequence |
| **188** | humanized HM242-C3-X2-K Light Chain DNA Sequence |
| **189** | humanized HM242-C3-X3-K Heavy Chain Amino Acid Sequence |
| **190** | humanized HM242-C3-X3-K Heavy Chain DNA Sequence |
| **191** | humanized HM242-C3-X3-K Light Chain Amino Acid Sequence |
| **192** | humanized HM242-C3-X3-K Light Chain DNA Sequence |
| **193** | humanized HM242-C3-X3-KE Heavy Chain Amino Acid Sequence |
| **194** | humanized HM242-C3-X3-KE Heavy Chain DNA Sequence |
| **195** | humanized HM242-C3-X3-KE Light Chain Amino Acid Sequence |
| **196** | humanized HM242-C3-X3-KE Light Chain DNA Sequence |
| **197** | humanized HM242-C3-X3-KI Heavy Chain Amino Acid Sequence |
| **198** | humanized HM242-C3-X3-KI Heavy Chain DNA Sequence |
| **199** | humanized HM242-C3-X3-KI Light Chain Amino Acid Sequence |
| **200** | humanized HM242-C3-X3-KI Light Chain DNA Sequence |
| **201** | humanized HM242-C3-X3-KL Heavy Chain Amino Acid Sequence |
| **202** | humanized HM242-C3-X3-KL Heavy Chain DNA Sequence |
| **203** | humanized HM242-C3-X3-KL Light Chain Amino Acid Sequence |
| **204** | humanized HM242-C3-X3-KL Light Chain DNA Sequence |
| **205** | humanized HM242-C3-X3-KS Heavy Chain Amino Acid Sequence |
| **206** | humanized HM242-C3-X3-KS Heavy Chain DNA Sequence |
| **207** | humanized HM242-C3-X3-KS Light Chain Amino Acid Sequence |
| **208** | humanized HM242-C3-X3-KS Light Chain DNA Sequence |
| **209** | humanized HM242-C3-X3-KSI Heavy Chain Amino Acid Sequence |
| **210** | humanized HM242-C3-X3-KSI Heavy Chain DNA Sequence |
| **211** | humanized HM242-C3-X3-KSI Light Chain Amino Acid Sequence |
| **212** | humanized HM242-C3-X3-KSI Light Chain DNA Sequence |
| **213** | humanized HM242-C3-X3-KSL Heavy Chain Amino Acid Sequence |
| **214** | humanized HM242-C3-X3-KSL Heavy Chain DNA Sequence |
| **215** | humanized HM242-C3-X3-KSL Light Chain Amino Acid Sequence |
| **216** | humanized HM242-C3-X3-KSL Light Chain DNA Sequence |
| **217** | humanized HM242-C3-X4-K Heavy Chain Amino Acid Sequence |
| **218** | humanized HM242-C3-X4-K Heavy Chain DNA Sequence |
| **219** | humanized HM242-C3-X4-K Light Chain Amino Acid Sequence |
| **220** | humanized HM242-C3-X4-K Light Chain DNA Sequence |
| **221** | humanized HM242-C3-X4-KS Heavy Chain Amino Acid Sequence |
| **222** | humanized HM242-C3-X4-KS Heavy Chain DNA Sequence |
| **223** | humanized HM242-C3-X4-KS Light Chain Amino Acid Sequence |
| **224** | humanized HM242-C3-X4-KS Light Chain DNA Sequence |
| **225** | humanized HM242-D3-X2-K Heavy Chain Amino Acid Sequence |
| **226** | humanized HM242-D3-X2-K Heavy Chain DNA Sequence |
| **227** | humanized HM242-D3-X2-K Light Chain Amino Acid Sequence |
| **228** | humanized HM242-D3-X2-K Light Chain DNA Sequence |
| **229** | humanized HM242-D3-X3-K Heavy Chain Amino Acid Sequence |
| **230** | humanized HM242-D3-X3-K Heavy Chain DNA Sequence |
| **231** | humanized HM242-D3-X3-K Light Chain Amino Acid Sequence |
| **232** | humanized HM242-D3-X3-K Light Chain DNA Sequence |
| **233** | humanized HM242-D3-X3-KE Heavy Chain Amino Acid Sequence |
| **234** | humanized HM242-D3-X3-KE Heavy Chain DNA Sequence |
| **235** | humanized HM242-D3-X3-KE Light Chain Amino Acid Sequence |
| **236** | humanized HM242-D3-X3-KE Light Chain DNA Sequence |
| **237** | humanized HM242-D3-X3-KI Heavy Chain Amino Acid Sequence |
| **238** | humanized HM242-D3-X3-KI Heavy Chain DNA Sequence |
| **239** | humanized HM242-D3-X3-KI Light Chain Amino Acid Sequence |
| **240** | humanized HM242-D3-X3-KI Light Chain DNA Sequence |
| **241** | humanized HM242-D3-X3-KL Heavy Chain Amino Acid Sequence |
| **242** | humanized HM242-D3-X3-KL Heavy Chain DNA Sequence |
| **243** | humanized HM242-D3-X3-KL Light Chain Amino Acid Sequence |
| **244** | humanized HM242-D3-X3-KL Light Chain DNA Sequence |
| **245** | humanized HM242-D3-X3-KS Heavy Chain Amino Acid Sequence |
| **246** | humanized HM242-D3-X3-KS Heavy Chain DNA Sequence |
| **247** | humanized HM242-D3-X3-KS Light Chain Amino Acid Sequence |
| **248** | humanized HM242-D3-X3-KS Light Chain DNA Sequence |
| **249** | humanized HM242-D3-X3-KSI Heavy Chain Amino Acid Sequence |
| **250** | humanized HM242-D3-X3-KSI Heavy Chain DNA Sequence |
| **251** | humanized HM242-D3-X3-KSI Light Chain Amino Acid Sequence |
| **252** | humanized HM242-D3-X3-KSI Light Chain DNA Sequence |
| **253** | humanized HM242-D3-X3-KSL Heavy Chain Amino Acid Sequence |
| **254** | humanized HM242-D3-X3-KSL Heavy Chain DNA Sequence |
| **255** | humanized HM242-D3-X3-KSL Light Chain Amino Acid Sequence |
| **256** | humanized HM242-D3-X3-KSL Light Chain DNA Sequence |
| **257** | humanized HM242-D3-X4-K Heavy Chain Amino Acid Sequence |
| **258** | humanized HM242-D3-X4-K Heavy Chain DNA Sequence |
| **259** | humanized HM242-D3-X4-K Light Chain Amino Acid Sequence |
| **260** | humanized HM242-D3-X4-K Light Chain DNA Sequence |
| **261** | humanized HM242-D3-X4-KS Heavy Chain Amino Acid Sequence |
| **262** | humanized HM242-D3-X4-KS Heavy Chain DNA Sequence |
| **263** | humanized HM242-D3-X4-KS Light Chain Amino Acid Sequence |
| **264** | humanized HM242-D3-X4-KS Light Chain DNA Sequence |
| **265** | humanized HM268-K8-X2-K Heavy Chain Amino Acid Sequence |
| **266** | humanized HM268-K8-X2-K Heavy Chain DNA Sequence |
| **267** | humanized HM268-K8-X2-K Light Chain Amino Acid Sequence |
| **268** | humanized HM268-K8-X2-K Light Chain DNA Sequence |
| **269** | humanized HM268-K8-X3-K Heavy Chain Amino Acid Sequence |
| **270** | humanized HM268-K8-X3-K Heavy Chain DNA Sequence |
| **271** | humanized HM268-K8-X3-K Light Chain Amino Acid Sequence |
| **272** | humanized HM268-K8-X3-K Light Chain DNA Sequence |
| **273** | humanized HM268-K8-X3-KE Heavy Chain Amino Acid Sequence |
| **274** | humanized HM268-K8-X3-KE Heavy Chain DNA Sequence |
| **275** | humanized HM268-K8-X3-KE Light Chain Amino Acid Sequence |
| **276** | humanized HM268-K8-X3-KE Light Chain DNA Sequence |
| **277** | humanized HM268-K8-X3-KI Heavy Chain Amino Acid Sequence |
| **278** | humanized HM268-K8-X3-KI Heavy Chain DNA Sequence |
| **279** | humanized HM268-K8-X3-KI Light Chain Amino Acid Sequence |
| **280** | humanized HM268-K8-X3-KI Light Chain DNA Sequence |
| **281** | humanized HM268-K8-X3-KL Heavy Chain Amino Acid Sequence |
| **282** | humanized HM268-K8-X3-KL Heavy Chain DNA Sequence |
| **283** | humanized HM268-K8-X3-KL Light Chain Amino Acid Sequence |
| **284** | humanized HM268-K8-X3-KL Light Chain DNA Sequence |
| **285** | humanized HM268-K8-X3-KS Heavy Chain Amino Acid Sequence |
| **286** | humanized HM268-K8-X3-KS Heavy Chain DNA Sequence |
| **287** | humanized HM268-K8-X3-KS Light Chain Amino Acid Sequence |
| **288** | humanized HM268-K8-X3-KS Light Chain DNA Sequence |
| **289** | humanized HM268-K8-X3-KSI Heavy Chain Amino Acid Sequence |
| **290** | humanized HM268-K8-X3-KSI Heavy Chain DNA Sequence |
| **291** | humanized HM268-K8-X3-KSI Light Chain Amino Acid Sequence |
| **292** | humanized HM268-K8-X3-KSI Light Chain DNA Sequence |
| **293** | humanized HM268-K8-X3-KSL Heavy Chain Amino Acid Sequence |
| **294** | humanized HM268-K8-X3-KSL Heavy Chain DNA Sequence |
| **295** | humanized HM268-K8-X3-KSL Light Chain Amino Acid Sequence |
| **296** | humanized HM268-K8-X3-KSL Light Chain DNA Sequence |
| **297** | humanized HM268-K8-X4-K Heavy Chain Amino Acid Sequence |
| **298** | humanized HM268-K8-X4-K Heavy Chain DNA Sequence |
| **299** | humanized HM268-K8-X4-K Light Chain Amino Acid Sequence |
| **300** | humanized HM268-K8-X4-K Light Chain DNA Sequence |
| **301** | humanized HM268-K8-X4-KS Heavy Chain Amino Acid Sequence |
| **302** | humanized HM268-K8-X4-KS Heavy Chain DNA Sequence |
| **303** | humanized HM268-K8-X4-KS Light Chain Amino Acid Sequence |
| **304** | humanized HM268-K8-X4-KS Light Chain DNA Sequence |
| **305** | humanized HM297-H5-X2-K Heavy Chain Amino Acid Sequence |
| **306** | humanized HM297-H5-X2-K Heavy Chain DNA Sequence |
| **307** | humanized HM297-H5-X2-K Light Chain Amino Acid Sequence |
| **308** | humanized HM297-H5-X2-K Light Chain DNA Sequence |
| **309** | humanized HM297-H5-X3-KS Heavy Chain Amino Acid Sequence |
| **310** | humanized HM297-H5-X3-KS Heavy Chain DNA Sequence |
| **311** | humanized HM297-H5-X3-KS Light Chain Amino Acid Sequence |
| **312** | humanized HM297-H5-X3-KS Light Chain DNA Sequence |
| **313** | humanized HM297-H5-X4-K Heavy Chain Amino Acid Sequence |
| **314** | humanized HM297-H5-X4-K Heavy Chain DNA Sequence |
| **315** | humanized HM297-H5-X4-K Light Chain Amino Acid Sequence |
| **316** | humanized HM297-H5-X4-K Light Chain DNA Sequence |
| **317** | humanized HM297-H5-X3-K Heavy Chain Amino Acid Sequence |
| **318** | humanized HM297-H5-X3-K Heavy Chain DNA Sequence |
| **319** | humanized HM297-H5-X3-K Light Chain Amino Acid Sequence |
| **320** | humanized HM297-H5-X3-K Light Chain DNA Sequence |
| **321** | humanized HM297-H5-X3-KSL Heavy Chain Amino Acid Sequence |
| **322** | humanized HM297-H5-X3-KSL Heavy Chain DNA Sequence |
| **323** | humanized HM297-H5-X3-KSL Light Chain Amino Acid Sequence |
| **324** | humanized HM297-H5-X3-KSL Light Chain DNA Sequence |
| **325** | humanized HM297-H5-X3-KSI Heavy Chain Amino Acid Sequence |
| **326** | humanized HM297-H5-X3-KSI Heavy Chain DNA Sequence |
| **327** | humanized HM297-H5-X3-KSI Light Chain Amino Acid Sequence |
| **328** | humanized HM297-H5-X3-KSI Light Chain DNA Sequence |
| **329** | humanized HM297-H5-X3-KL Heavy Chain Amino Acid Sequence |
| **330** | humanized HM297-H5-X3-KL Heavy Chain DNA Sequence |
| **331** | humanized HM297-H5-X3-KL Light Chain Amino Acid Sequence |
| **332** | humanized HM297-H5-X3-KL Light Chain DNA Sequence |
| **333** | humanized HM297-H5-X3-KI Heavy Chain Amino Acid Sequence |
| **334** | humanized HM297-H5-X3-KI Heavy Chain DNA Sequence |
| **335** | humanized HM297-H5-X3-KI Light Chain Amino Acid Sequence |
| **336** | humanized HM297-H5-X3-KI Light Chain DNA Sequence |
| **337** | humanized HM297-H5-X4-KS Heavy Chain Amino Acid Sequence |
| **338** | humanized HM297-H5-X4-KS Heavy Chain DNA Sequence |
| **339** | humanized HM297-H5-X4-KS Light Chain Amino Acid Sequence |
| **340** | humanized HM297-H5-X4-KS Light Chain DNA Sequence |
| **341** | humanized HM297-H5-X3-KE Heavy Chain Amino Acid Sequence |
| **342** | humanized HM297-H5-X3-KE Heavy Chain DNA Sequence |
| **343** | humanized HM297-H5-X3-KE Light Chain Amino Acid Sequence |
| **344** | humanized HM297-H5-X3-KE Light Chain DNA Sequence |
| **345** | HM242-CDR-H1 Amino Acid Sequence (Kabat) |
| **346** | HM242-CDR-H2 Amino Acid Sequence (Kabat) |
| **347** | HM242-CDR-H3 Amino Acid Sequence (Kabat) |
| **348** | HM242-CDR-L1 Amino Acid Sequence (Kabat) |
| **349** | HM242-CDR-L2 Amino Acid Sequence (Kabat) |
| **350** | HM242-CDR-L3 Amino Acid Sequence (Kabat) |
| **351** | HM266-CDR-H1 Amino Acid Sequence (Kabat) |
| **352** | HM266-CDR-H2 Amino Acid Sequence (Kabat) |
| **353** | HM266-CDR-H3 Amino Acid Sequence (Kabat) |
| **354** | HM266-CDR-L1 Amino Acid Sequence (Kabat) |
| **355** | HM266-CDR-L2 Amino Acid Sequence (Kabat) |
| **356** | HM266-CDR-L3 Amino Acid Sequence (Kabat) |
| **357** | HM268-CDR-H1 Amino Acid Sequence (Kabat) |
| **358** | HM268-CDR-H2 Amino Acid Sequence (Kabat) |
| **359** | HM268-CDR-H3 Amino Acid Sequence (Kabat) |
| **360** | HM268-CDR-L1 Amino Acid Sequence (Kabat) |
| **361** | HM268-CDR-L2 Amino Acid Sequence (Kabat) |
| **362** | HM268-CDR-L3 Amino Acid Sequence (Kabat) |
| **363** | HM297-CDR-H1 Amino Acid Sequence (Kabat) |
| **364** | HM297-CDR-H2 Amino Acid Sequence (Kabat) |
| **365** | HM297-CDR-H3 Amino Acid Sequence (Kabat) |
| **366** | HM297-CDR-L1 Amino Acid Sequence (Kabat) |
| **367** | HM297-CDR-L2 Amino Acid Sequence (Kabat) |
| **368** | HM297-CDR-L3 Amino Acid Sequence (Kabat) |
| **369** | HM242-CDR-H1 Amino Acid Sequence (Chothia) |
| **370** | HM242-CDR-H2 Amino Acid Sequence (Chothia) |
| **371** | HM242-CDR-H3 Amino Acid Sequence (Chothia) |
| **372** | HM242-CDR-L1 Amino Acid Sequence (Chothia) |
| **373** | HM242-CDR-L2 Amino Acid Sequence (Chothia) |
| **374** | HM242-CDR-L3 Amino Acid Sequence (Chothia) |
| **375** | HM266-CDR-H1 Amino Acid Sequence (Chothia) |
| **376** | HM266-CDR-H2 Amino Acid Sequence (Chothia) |
| **377** | HM266-CDR-H3 Amino Acid Sequence (Chothia) |
| **378** | HM266-CDR-L1 Amino Acid Sequence (Chothia) |
| **379** | HM266-CDR-L2 Amino Acid Sequence (Chothia) |
| **380** | HM266-CDR-L3 Amino Acid Sequence (Chothia) |
| **381** | HM268-CDR-H1 Amino Acid Sequence (Chothia) |
| **382** | HM268-CDR-H2 Amino Acid Sequence (Chothia) |
| **383** | HM268-CDR-H3 Amino Acid Sequence (Chothia) |
| **384** | HM268-CDR-L1 Amino Acid Sequence (Chothia) |
| **385** | HM268-CDR-L2 Amino Acid Sequence (Chothia) |
| **386** | HM268-CDR-L3 Amino Acid Sequence (Chothia) |
| **387** | HM297-CDR-H1 Amino Acid Sequence (Chothia) |
| **388** | HM297-CDR-H2 Amino Acid Sequence (Chothia) |
| **389** | HM297-CDR-H3 Amino Acid Sequence (Chothia) |
| **390** | HM297-CDR-L1 Amino Acid Sequence (Chothia) |
| **391** | HM297-CDR-L2 Amino Acid Sequence (Chothia) |
| **392** | HM297-CDR-L3 Amino Acid Sequence (Chothia) |
| **393** | HM242-CDR-H1 Amino Acid Sequence (IMGT) |
| **394** | HM242-CDR-H2 Amino Acid Sequence (IMGT) |
| **395** | HM242-CDR-H3 Amino Acid Sequence (IMGT) |
| **396** | HM242-CDR-L1 Amino Acid Sequence (IMGT) |
| **397** | HM242-CDR-L2 Amino Acid Sequence (IMGT) |
| **398** | HM242-CDR-L3 Amino Acid Sequence (IMGT) |
| **399** | HM266-CDR-H1 Amino Acid Sequence (IMGT) |
| **400** | HM266-CDR-H2 Amino Acid Sequence (IMGT) |
| **401** | HM266-CDR-H3 Amino Acid Sequence (IMGT) |
| **402** | HM266-CDR-L1 Amino Acid Sequence (IMGT) |
| **403** | HM266-CDR-L2 Amino Acid Sequence (IMGT) |
| **404** | HM266-CDR-L3 Amino Acid Sequence (IMGT) |
| **405** | HM268-CDR-H1 Amino Acid Sequence (IMGT) |
| **406** | HM268-CDR-H2 Amino Acid Sequence (IMGT) |
| **407** | HM268-CDR-H3 Amino Acid Sequence (IMGT) |
| **408** | HM268-CDR-L1 Amino Acid Sequence (IMGT) |
| **409** | HM268-CDR-L2 Amino Acid Sequence (IMGT) |
| **410** | HM268-CDR-L3 Amino Acid Sequence (iMGT) |
| **411** | HM297-CDR-H1 Amino Acid Sequence (IMGT) |
| **412** | HM297-CDR-H2 Amino Acid Sequence (IMGT) |
| **413** | HM297-CDR-H3 Amino Acid Sequence (IMGT) |
| **414** | HM297-CDR-L1 Amino Acid Sequence (IMGT) |
| **415** | HM297-CDR-L2 Amino Acid Sequence (IMGT) |
| **416** | HM297-CDR-L3 Amino Acid Sequence (IMGT) |
| **417** | HM242-CDR-H1 Amino Acid Sequence (Paratome) |
| **418** | HM242-CDR-H2 Amino Acid Sequence (Paratome) |
| **419** | HM242-CDR-H3 Amino Acid Sequence (Paratome) |
| **420** | HM242-CDR-L1 Amino Acid Sequence (Paratome) |
| **421** | HM242-CDR-L2 Amino Acid Sequence (Paratome) |
| **422** | HM242-CDR-L3 Amino Acid Sequence (Paratome) |
| **423** | HM266-CDR-H1 Amino Acid Sequence (Paratome) |
| **424** | HM266-CDR-H2 Amino Acid Sequence (Paratome) |
| **425** | HM266-CDR-H3 Amino Acid Sequence (Paratome) |
| **426** | HM266-CDR-L1 Amino Acid Sequence (Paratome) |
| **427** | HM266-CDR-L2 Amino Acid Sequence (Paratome) |
| **428** | HM266-CDR-L3 Amino Acid Sequence (Paratome) |
| **429** | HM268-CDR-H1 Amino Acid Sequence (Paratome) |
| **430** | HM268-CDR-H2 Amino Acid Sequence (Paratome) |
| **431** | HM268-CDR-H3 Amino Acid Sequence (Paratome) |
| **432** | HM268-CDR-L1 Amino Acid Sequence (Paratome) |
| **433** | HM268-CDR-L2 Amino Acid Sequence (Paratome) |
| **434** | HM268-CDR-L3 Amino Acid Sequence (Paratome) |
| **435** | HM297-CDR-H1 Amino Acid Sequence (Paratome) |
| **436** | HM297-CDR-H2 Amino Acid Sequence (Paratome) |
| **437** | HM297-CDR-H3 Amino Acid Sequence (Paratome) |
| **438** | HM297-CDR-L1 Amino Acid Sequence (Paratome) |
| **439** | HM297-CDR-L2 Amino Acid Sequence (Paratome) |
| **440** | HM297-CDR-L3 Amino Acid Sequence (Paratome) |

## Claims

1. An agonist antibody to human PD-1 or a functional fragment thereof, wherein the antibody or a functional fragment thereof binds to domain #7 as shown in SEQ ID NO: 9, of human PD-1.

2. The antibody or a functional fragment thereof of claim 1, wherein the antibody or a functional fragment thereof has any one of (A) to (D) below:
(A) an antibody heavy chain variable region sequence comprising CDR1, CDR2 and CDR3 in the amino acid sequence as shown in SEQ ID NO: 17 and an antibody light chain variable region sequence comprising CDR1, CDR2 and CDR3 in the amino acid sequence as shown in SEQ ID NO: 34;
(B) an antibody heavy chain variable region sequence comprising CDR1, CDR2 and CDR3 in the amino acid sequence as shown in SEQ ID NO: 22 and an antibody light chain variable region sequence comprising CDR1, CDR2 and CDR3 in the amino acid sequence as shown in SEQ ID NO: 38;
(C) an antibody heavy chain variable region sequence comprising CDR1, CDR2 and CDR3 in the amino acid sequence as shown in SEQ ID NO: 28 and an antibody light chain variable region sequence comprising CDR1, CDR2 and CDR3 in the amino acid sequence as shown in SEQ ID NO: 42; or
(D) an antibody heavy chain variable region sequence comprising CDR1, CDR2 and CDR3 in the amino acid sequence as shown in SEQ ID NO: 33 and an antibody light chain variable region sequence comprising CDR1, CDR2 and CDR3 in the amino acid sequence as shown in SEQ ID NO: 46.

3. The antibody or a functional fragment thereof of claim 1, wherein the antibody or a functional fragment thereof has either the following (A) to (D) below:
(A) an antibody heavy chain variable region comprising complementarity determining region (CDR) 1 of SEQ ID NO: 345, CDR2 of SEQ ID NO: 346 and CDR3 of SEQ ID NO: 347, as well as an antibody light chain variable region comprising CDR1 of SEQ ID NO: 348, CDR2 of SEQ ID NO: 349 and CDR3 of SEQ ID NO: 350;
(B) an antibody heavy chain variable region comprising CDR1 of SEQ ID NO: 351, CDR2 of SEQ ID NO: 352 and CDR3 of SEQ ID NO: 353, as well as an antibody light chain variable region comprising CDR1 of SEQ ID NO: 354, CDR2 of SEQ ID NO: 355 and CDR3 of SEQ ID NO: 356;
(C) an antibody heavy chain variable region comprising CDR1 of SEQ ID NO: 357, CDR2 of SEQ ID NO: 358 and CDR3 of SEQ ID NO: 359, as well as an antibody light chain variable region comprising CDR1 of SEQ ID NO: 360, CDR2 of SEQ ID NO: 361 and CDR3 of SEQ ID NO: 362; or
(D) an antibody heavy chain variable region comprising CDR1 of SEQ ID NO: 363, CDR2 of SEQ ID NO: 364 and CDR3 of SEQ ID NO: 365, as well as an antibody light chain variable region comprising CDR1 of SEQ ID NO: 366, CDR2 of SEQ ID NO: 367 and CDR3 of SEQ ID NO: 368.

4. The antibody or a functional fragment thereof of any one of claims 1 to 3, wherein the antibody variable region comprises the framework of human antibody or the framework of humanized antibody.

5. The antibody or a functional fragment thereof of claim 1, wherein the antibody or a functional fragment thereof has either the following (A) to (D) below:
(A) an antibody heavy chain variable region having the amino acid sequence as shown in SEQ ID NO: 20 and a light chain variable region having the amino acid sequence as shown in SEQ ID NO: 37;
(B) an antibody heavy chain variable region having the amino acid sequence as shown in SEQ ID NO: 21 and a light chain variable region having the amino acid sequence as shown in SEQ ID NO: 37;
(C) an antibody heavy chain variable region having the amino acid sequence as shown in SEQ ID NO: 26 and a light chain variable region having the amino acid sequence as shown in SEQ ID NO: 40; or
(D) an antibody heavy chain variable region having the amino acid sequence as shown in SEQ ID NO: 30 and a light chain variable region having the amino acid sequence as shown in SEQ ID NO: 44.

6. The antibody or a functional fragment thereof of any one of claims 1 to 5, wherein the antibody heavy chain constant region and the antibody light chain constant region comprise an amino acid sequence derived from human antibody.

7. The antibody or a functional fragment thereof of any one of claims 1 to 5, wherein the antibody heavy chain constant region is the antibody heavy chain constant region of human IgG.

8. The antibody or a functional fragment thereof of claim 7, wherein the antibody heavy chain constant region of said human IgG is the antibody heavy chain constant region of human IgG1.

9. The antibody or a functional fragment thereof of any one of claims 1 to 8, wherein the antibody light chain constant region is the antibody light chain constant region of human Igκ.

10. The antibody or a functional fragment thereof of any one of claims 1 to 9, wherein the antibody or a functional fragment thereof has an improved affinity to any one or more of the following receptors: human FcyRIIA, human FcyRIIB or human FcyRIIA.

11. The antibody or a functional fragment thereof of any one of claims 1 to 9, wherein the antibody or a functional fragment thereof has an improved affinity to human FcyRIIB.

12. The antibody or a functional fragment thereof of any one of claims 1 to 9 or claim 11, wherein the antibody or a functional fragment thereof has an improved affinity to human FcγRIIIA.

13. The antibody or a functional fragment thereof of claim 11 or 12, wherein the improvement of affinity to human FcyRIIB is, compared to a reference antibody having the Fc region of human IgG1-K322A, by 1.5 folds or more, preferably by 2.0 folds or more, and still more preferably by 2.5 folds or more, in a test measuring Fc receptor binding affinity using the surface plasmon resonance technique.

14. The antibody or a functional fragment thereof of any one of claims 11 to 13, wherein the improvement of affinity to human FcyRIIB is, compared to a reference antibody having the Fc region of human IgG1-K322A, by 2 folds or more, preferably by 5 folds or more, and still more preferably by 20 folds or more, in a test of measuring the affinity to a human Fc receptor-expressing cell line with a flow cytometer.

15. The antibody or a functional fragment thereof of claim 12 or 13, wherein the improvement of affinity to human FcγRIIIA is, compared to a reference antibody having the Fc region of human IgG1-K322A, by 1.5 folds or more, preferably by 2.0 folds or more, and still more preferably by 2.5 folds or more, in a test measuring Fc receptor binding affinity using the surface plasmon resonance technique.

16. The antibody or a functional fragment thereof of any one of claims 12 to 15, wherein the improvement of affinity to human FcγRIIIA is, compared to a reference antibody having the Fc region of human IgG1-K322A, by 1.5 folds or more, preferably by 2.0 folds or more, still more preferably by 4.0 folds or more, and most preferably by 5.0 folds or more, in a test of measuring the affinity to a human Fc receptor-expressing cell line with a flow cytometer.

17. The antibody or a functional fragment thereof of any one of claims 10 to 16, wherein the antibody or a functional fragment has a modified Fc region of human IgG, and the affinity of the antibody or a functional fragment thereof to said receptor has been improved by modification of the Fc region of human IgG.

18. The antibody or a functional fragment thereof of any one of claims 10 to 16, wherein the antibody or a functional fragment has a modified Fc region of human IgG1 or human IgG4, and the affinity of the antibody or a functional fragment thereof to said receptor has been improved by modification of the Fc region of human IgG1 or human IgG4.

19. The antibody or a functional fragment thereof of any one of claims 10 to 18, wherein any of the amino acids at positions 233, 234, 236, 237, 238, 239, 267, 268, 271, 296, 323, 326, 328, 330 and 332 (according to EU numbering) in the amino acid sequence of the Fc region of human IgG1 is modified.

20. The antibody or a functional fragment thereof of claim 19, wherein the modification of amino acids is any one of the combinations G236D/H268D, S239D/H268D, S239D/H268D/L328Y/I332E, G236D/H268D/K322A, S239D/H268D/K322A, S239D/S267G/H268D/K322A, G236D/H268D/E293A/K322A, S239D/H268D/K322A/L328Y/I332E, S239D/H268D/E293A/K322A, S239D/S267G/H268D/K322A/L328Y, S239D/S267G/H268D/K322A/I332E, S239D/H268D/K322A/L328Y, S239D/H268D/K322A/I332E, S239D/S267G/H268D/K322A/L328Y/I332E,E233D/G237D/P238D/H268D/P271G/A330R and S267E/L328F.

21. The antibody or a functional fragment thereof of any one of claims 10 to 18, wherein any of the amino acids at positions 236, 239, 268, 328 and 332 (according to EU numbering) in the amino acid sequence of the Fc region of human IgG4 is modified.

22. The antibody or a functional fragment thereof of claim 21, wherein the modification of amino acids is any one of the combinations S228P/G236D/Q268D, S228P/S239D/Q268D, and S228P/S239D/Q268D/L328Y/I332E.

23. The antibody or a functional fragment thereof of claim 10, wherein the antibody comprises either the following (A) to (D) below:
(A) an antibody heavy chain sequence having the amino acid sequence as shown in SEQ ID NO: 245 and an antibody light chain sequence having the amino acid sequence as shown in SEQ ID NO:247;
(B) an antibody heavy chain sequence having the amino acid sequence as shown in SEQ ID NO: 277 and an antibody light chain sequence having the amino acid sequence as shown in SEQ ID NO: 279;
(C) an antibody heavy chain sequence having the amino acid sequence as shown in SEQ ID NO: 285 and an antibody light chain sequence having the amino acid sequence as shown in SEQ ID NO: 287; or
(D) an antibody heavy chain sequence having the amino acid sequence as shown in SEQ ID NO: 289 and an antibody light chain sequence having the amino acid sequence as shown in SEQ ID NO: 291.

24. The antibody or a functional fragment thereof of any one of claims 12 to 23, wherein the antibody or a functional fragment thereof is defucosylated.

25. An isolated nucleic acid molecule encoding the antibody or a functional fragment thereof of any one of claims 1 to 24.

26. A vector comprising the nucleic acid molecule of claim 25.

27. A host cell comprising the nucleic acid molecule of claim 25.

28. A host cell comprising the vector of claim 26.

29. A method of producing an antibody or a functional fragment thereof, comprising a step of culturing the host cell of claim 27 or 28.

30. A pharmaceutical composition comprising the antibody or a functional fragment thereof of any one of claims 1 to 24 and a pharmaceutically acceptable carrier.
